# EUROPÄISCHE PATENTSCHRIFT

(11) **EP 3 055 303 B1**
(45) Veröffentlichungstag und Bekanntmachung des Hinweises auf die Patenterteilung: **02.12.2020**
(21) Anmeldenummer: 14766666.3
(22) Anmeldetag: 16.09.2014
(51) Int. Cl.: C07D 405/14, C07D 403/08, C07D 403/10, C07D 403/14, C07D 409/14, C07D 491/048, C07F 5/02, C09K 11/06, H01L 51/00, C07F 15/00, C07D 495/14, C07F 7/08, C09K 11/02, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 08.10.2013 EP 13004837
(43) Veröffentlichungstag der Anmeldung: 17.08.2016
(73) Patentinhaber: Merck Patent GmbH, 64293 Darmstadt (DE)
(72) Erfinder: PARHAM, Amir Hossain, 60486 Frankfurt am Main (DE); MARTYNOVA, Irina, 64347 Gruesheim (DE); JATSCH, Anja, 60489 Frankfurt am Main (DE); EBERLE, Thomas, 76829 Landau (DE); KROEBER, Jonas Valentin, 60311 Frankfurt am Main (DE); PFLUMM, Christof, 64291 Darmstadt (DE)
(86) Internationale Anmeldenummer: PCT/EP2014/002496
(87) Internationale Veröffentlichungsnummer: WO 2015/051869

(56) Entgegenhaltungen:
- WO-A1-2014/067614
- WO-A1-2015/014434
- WO-A2-2011/057706
- JP-A- 2014 103 103
- JP-A- 2014 116 454
- US-A1- 2012 071 668

## Beschreibung

Die Vorliegende Erfindung betrifft cyclische Verbindungen mit einer spezifischen Anordung von elektronenleitenden und lochleitenden Gruppen, deren Verwendung in elektronischen Vorrichtungen, deren Herstellung sowie elektronische Vorrichtungen.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es sowohl bei OLEDs, die Singulettemission zeigen, wie auch bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Weitere Matrixmaterialien gemäß dem Stand der Technik repräsentieren Triazine (bspw. WO 2008/056746, EP 0906947, EP 0908787, EP 0906948).

Für fluoreszierende OLEDs werden gemäß dem Stand der Technik vor allem kondensierte Aromaten, insbesondere Anthracenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis-(1-naphthyl)anthracen-Derivate für die Verwendung in OLEDs offenbart. Weitere Anthracenderivate sind in WO 01/076323, in WO 01/021729, in WO 2004/013073, in WO 2004/018588, in WO 2003/087023 oder in WO 2004/018587 offenbart. Host-Materialien, basierend auf Arylsubstituierten Pyrenen und Chrysenen, werden in WO 2004/016575 offenbart. Host-Materialien basierend auf Benzanthracenderivaten werden in WO 2008/145239 offenbart. Es ist für hochwertige Anwendungen wünschenswert, verbesserte Host-Materialien zur Verfügung zu haben.

Im Stand der Technik ist die Verwendung von Verbindungen enthaltend eine oder mehrere Carbazolgruppen in elektronischen Vorrichtungen, beispielsweise aus WO 2005/039246, US 2005/0069729, JP 2004/288381, EP 1205527 oder WO 2008/086851 bekannt

Weiterhin im Stand der Technik bekannt ist die Verwendung von Verbindungen enthaltend eine oder mehrere Indenocarbazolgruppen in elektronischen Vorrichtungen, beispielsweise aus WO 2010/136109 und WO 2011/000455.

Weiterhin im Stand der Technik bekannt ist die Verwendung von Verbindungen enthaltend eine oder mehrere elektronenarme heteroaromatische Sechsringe in elektronischen Vorrichtungen, beispielsweise aus WO 2010/015306, WO 2007/063754 und WO 2008/056746.

WO 2009/069442 offenbart Tricyclen, wie Carbazol, Dibenzofuran oder Dibenzothiophen, die hochgradig mit elektronenarmen Heteroaromaten (z.B. Pyridin, Pyrimidin oder Triazin) substituiert sind. Mit lochleitenden Gruppen, d.h. elektronenreichen Gruppen, sind die Tricyclen nicht substituiert.

JP 2009-21336 offenbart substituierte Carbazole als Matrixmaterialien, wobei die Carbazole mit einer elektronenleitenden und mit einer lochleitenden Gruppe substituiert sind. Die Verbindungen weisen allerdings keine face-to-face Substitution auf.

WO 2011/057706 offenbart substituierte Carbazole als Matrixmaterialien, wobei die Carbazole mit einer elektronenleitenden und mit einer lochleitenden Gruppe substituiert sind. Allerdings weisen die meisten der offenbarten Carbazole keine face-to-face Substitution auf. Bei den vereinzelt offenbarten face-to-face Anordnungen ist die loch- bzw. elektronenleitende Gruppe jedoch direkt an den Tricyclus gebunden.

WO 2011/057706 A2 offenbart Carbazole, Dibenzofurane und Dibenzothiophene, die zweifach mit einer lochteitenden und einer elektronenleitenden Gruppe substituiert sind, sowie deren Verwendung in organischen Elektrolumineszenzvorrichtungen. Dibenzofurane, die in den Positionen 4 und 6 subtituiert sind, weisen Carbazol und Pyrimidin als Substituenten auf.

US 2012/071668 A1 offenbart Carbazole, die mit Dibenzothiophenen oder Dibenzofuranen substituiert sind, sowie deren Verwendung in organischen lichtemittierenden Elementen.

JP 2014 116454 A offenbart offenbart Carbazole, die mit Dibenzofuranen substituiert sind, sowie deren Verwendung in organischen Elektrolumineszenzvorrichtungen.

JP 2014 103103 A offenbart Aza-Dibenzofurane, die mit Pyridylgruppen substituiert sein können, sowie deren Verwendung in organischen Elektrolumineszenzvorrichtungen.

WO 2014/067614 A1 offenbart Dibenzofurane und Dibenzothiophene, die in den Positionen 4 und 6 mit lochleitenden Gruppen substituiert sind, sowie deren Verwendung in organischen Elektrolumineszenzvorrichtungen.

Der nichtvorveröffentliche Stand der Technik WO 2015/014434 A1 offenbart Dibenzofurane, Dibenzothiophene, Biphenylene und Xanthene, die sowohl mit elektronentransportierenden als auch mit lochkeitnden Gruppen substituiert sind, sowie deren Verwendung in organischen Elektrolumineszenzvorrichtungen. Einige der darin offenbarten Strukturen wurden vom Schutzumfang der Ansprüche ausgeschlossen.

Allerdings besteht bei Verwendung dieser Materialien ebenso wie bei anderen Materialien noch Verbesserungsbedarf, insbesondere in Bezug auf die Effizienz und die Lebensdauer der Vorrichtung.

Aufgabe der vorliegenden Erfindung ist daher die Bereitstellung von Verbindungen, welche sich für den Einsatz in einer fluoreszierenden oder phosphoreszierenden OLED eignen, beispielsweise als Host- und/oder Matrixmaterial oder als Lochtransport-/Elektronenblockiermaterial bzw. Exzitonenblockiermaterial oder als Elektronentransport- bzw. Lochblockiermaterial, und welche bei Verwendung in einer OLED zu guten Device-Eigenschaften führen, sowie die Bereitstellung der entsprechenden elektronischen Vorrichtung.

Überraschend wurde gefunden, dass bestimmte, unten näher beschriebene Verbindungen diese Aufgaben lösen und zu guten Eigenschaften der organischen Elektrolumineszenzvorrichtung führen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Verbindungen sind daher der Gegenstand der vorliegenden Erfindung. Die überraschenden Effekte werden durch eine spezielle Anordnung ("face-to-face", d.h. einander gegenüberliegende Anordnung von Gruppen) elektronenleitender und lochleitender Gruppen in Verbindungen der unten aufgeführten Formeln erreicht. Ohne an eine Theorie gebunden zu sein, könnte der schnelle Ladungstransport an der relativ wohldefinierten (hochgeordneten) Parallelausrichtung der Moleküle (*face-to-face* Anordnung) liegen, in der eine gewisse Nahordnung der Moleküle vorliegt. Bedingt durch die geringen Abstände der Gruppen zueinander könnten zwischenmolekulare Wechselwirkungen, wie beispielsweise direkte π-π-Wechselwirkung für den schnellen Ladungstransfer mit ursächlich sein.

Die erfindungsgemäßen Verbindungen weisen auch eine hohe Glasübergangstempertur (T_{g}) auf, was vorteilhaft ist hinsichtlich der Prozessierung der Verbindungen bei der Herstellung elektronischer Vorrichtungen. Die hohe Glasübergangstemperatur der Verbindungen gestattet auch die Verwendung der Verbindungen in dünnen amorphen organischen Schichten.

Weiterhin erlauben die erfindungsgemäßen Verbindungen eine Stabilisierung der Ladungsträger im angeregten Zustand und weisen eine ausreichend hohe Triplett Energie auf, was für phosphoreszierende Vorrichtungen eine wichtige Vorraussetzung darstellt. Ferner zeigen die erfindungsgemäßen Verbindungen verbesserte Leistungsdaten in OLEDs gegenüber den Verbindungen aus dem Stand der Technik.

Gegenstand der vorliegenden Erfindung sind daher Verbindungen gemäß Anspruch 1.

Sofern zwei oder mehr benachbarte Reste miteinander kein mono- oder polycyclisches, aliphatisches oder aromatisches oder heteroaromatisches Ringsystem bilden, dürfen diese Reste nicht Teil eines Rings oder Ringsystems werden. Ist, beispielsweise, der Rest R¹ so definiert, dass zwei oder mehr benachbarte Reste R¹ miteinander kein mono- oder polycyclisches, aliphatisches oder aromatisches oder heteroaromatisches Ringsystem bilden, die Reste R¹ ihrerseits aber wieder mit Resten R² substituiert sein könnnen, wobei zwei oder mehr benachbarte Reste R² miteinander ein mono- oder polycyclisches, aliphatisches oder aromatisches oder heteroaromatisches Ringsystem bilden können, dann darf der Ringschluss der Reste R² nicht in der Art erfolgen, dass dadurch die Reste R¹ Teil eines Ringes oder Ringsystems werden.

Es ist bevorzugt, wenn die ETG, die mit einem oder meheren Resten R¹ substituiert sein kann keine elektronenreiche aromatische oder heteroaromatische Ringe oder Ringsysteme enthält.

Die Verbindung der allgemeinen Formel (2) enthält daher immer wenigstens einen Substituenten R⁴, der ungleich Wasserstoff ist.

Die Bindung der Reste R⁴ kann an alle noch nicht subtituierten Positionen 1 bis 8 des Carbazols erfolgen., wobei bevorzugt ist, dass die Reste R⁴ an die Positionen 5, 6, 7 und 8 des Carbazols binden.

Die Bindung der Carbazolrings in der Verbindung der Formel (2) an das Kohlenstoffatom des Rings A' kann über die Positionen 1, 2, 3 und 4 erfolgen.

In einer bevorzugten Ausführungsform erfolgt die Bindung des Carbazols an den Ring A' über die Position 1.

In einer anderen bevorzugten Ausführungsform erfolgt die Bindung des Carbazols an den Ring A' über die Position 2.

In einer anderen bevorzugten Ausführungsform erfolgt die Bindung des Carbazols an den Ring A' über die Position 3.

In noch einer anderen bevorzugten Ausführungsform erfolgt die Bindung des Carbazols an den Ring A' über die Position 4.

Demnach gilt, beispielsweise, für die Verbindungen der allgemeinen Formel (2), dass im Fall m = n = 1 und V = W = Einfachbindung die allgemeine Formel wie folgt lautet, wobei die folgende Formel auch eine ganz besonders bevorzugte Ausführungsform der vorliegenden Erfindung darstellt.

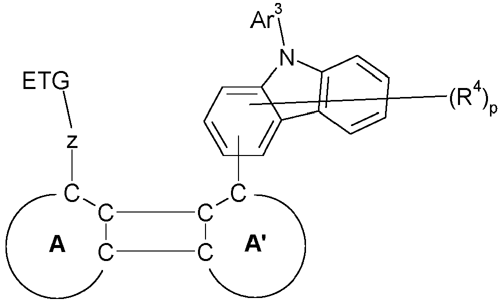

Weiterhin gilt, beispielsweise, für die Verbindungen der allgemeinen Formel (2), dass im Fall m = n = 1 und V = O und W = Einfachbindung die allgemeine Formel wie folgt lautet, wobei die folgende Formel auch eine ganz besonders bevorzugte Ausführungsform der vorliegenden Erfindung darstellt.

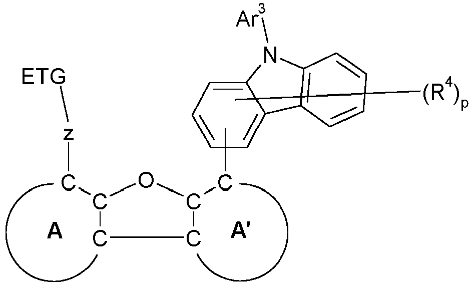

Weiterhin gilt, beispielsweise, für die Verbindungen der allgemeinen Formel (2), dass im Fall m = n = 0 die allgemeine Formel wie folgt lautetwobei die folgende Formel auch eine ganz besonders bevorzugte Ausführungsform der vorliegenden Erfindung darstellt.

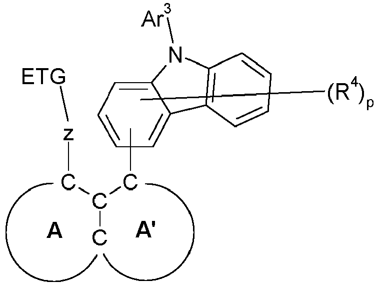

Ganz bevorzugt ist demnach eine Verbindung der allgemeinen Formeln (3) bis (11) 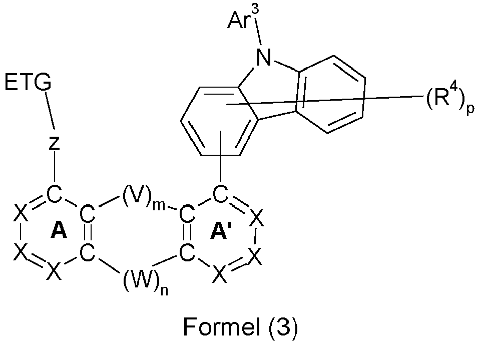 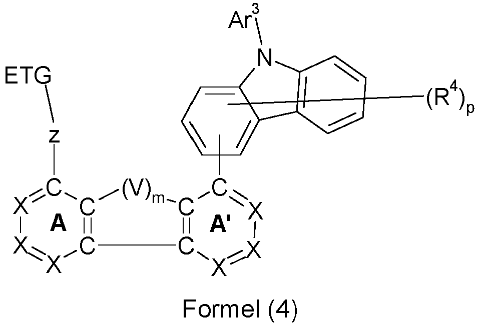 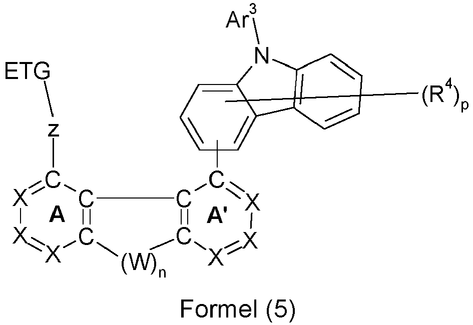 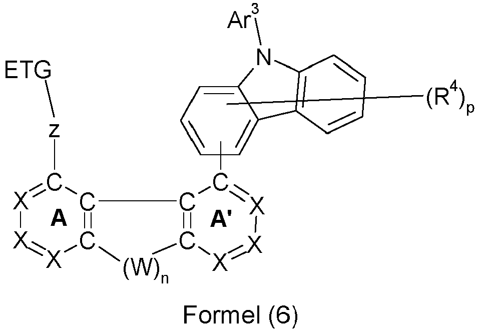 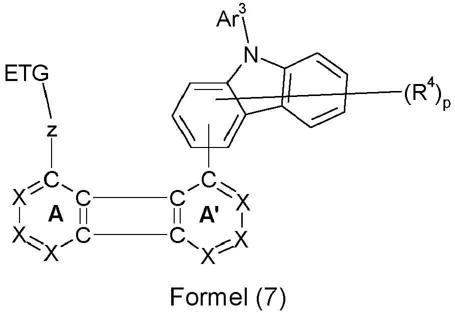 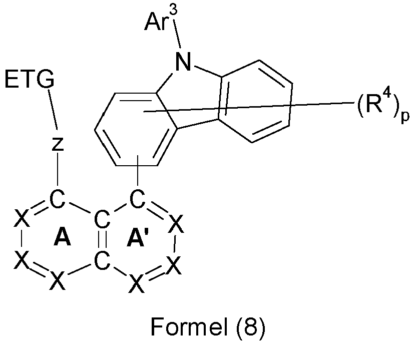 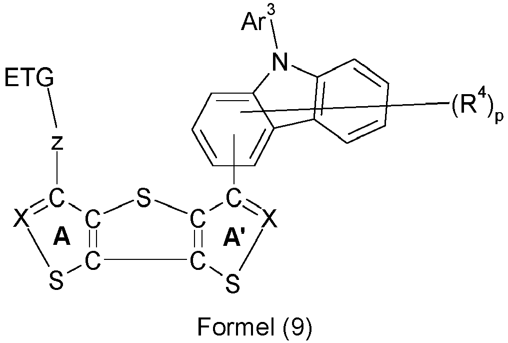 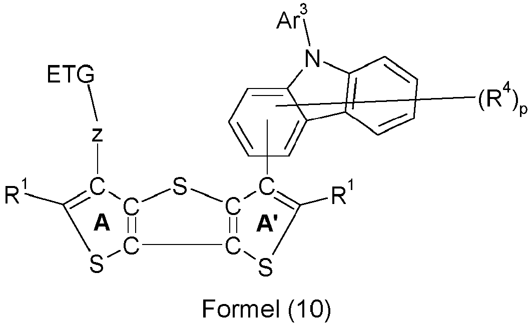 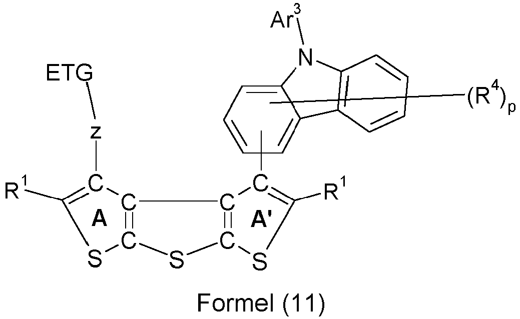 wobei eine Verbindung der allgemeinen Formeln (3) bis (8) ganz besonders bevorzugt ist und eine Verbindung der allgemeinen Formel (4) insbesondere bevorzugt ist.

Es ist weiterhin ganz besonders bevorzugt, wenn X in den Formeln (1) bis (9) gleich CR¹ ist.

In einer bevorzugten Ausführungsform betrifft die vorliegende Erfindung eine Verbindung der Formel (4), bevorzugt eine Verbindung der Formel (4) mit X gleich CR¹ und m=1, ganz bevorzugt eine Verbindung der Formel (4) mit X gleich CR¹, m=1 und V gleich O, wobei für die anderen Symbole und Indizes obige Definitionen und bevorzugte Ausführungsformen gelten.

In einer weiteren bevorzugten Ausführungsform betrifft die vorliegende Erfindung eine Verbindung der Formel (4) mit X gleich CR¹, m=1 und V gleich N-Ar³, wobei für die anderen Symbole und Indizes obige Definitionen und bevorzugte Ausführungsformen gelten.

Eine weitere bevorzugte Verbindung im Sinne der vorliegenden Erfindung ist eine der folgenden Formel (3a) 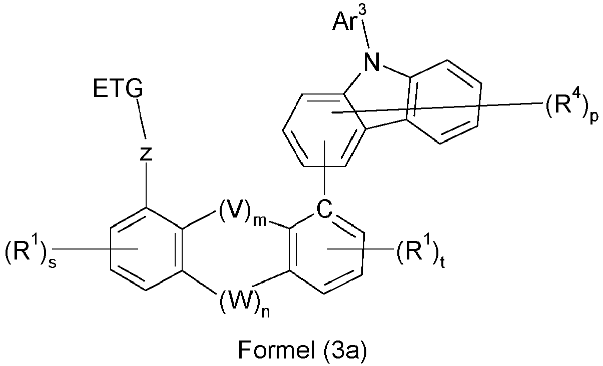 wobei s und t ganze Zahlen von 0 bis 3 sein können und wobei s+t gleich eine ganze Zahl von 0 bis 6 ist, bevorzugt ist s+t gleich 4, ganz bevorzugt ist s+t gleich 2, ganz besonders bevorzugt ist s+t gleich 1 und insbesondere bevorzugt ist s+t gleich 0.

Eine weitere bevorzugte Verbindung im Sinne der vorliegenden Erfindung ist eine der folgenden Formel (3b) 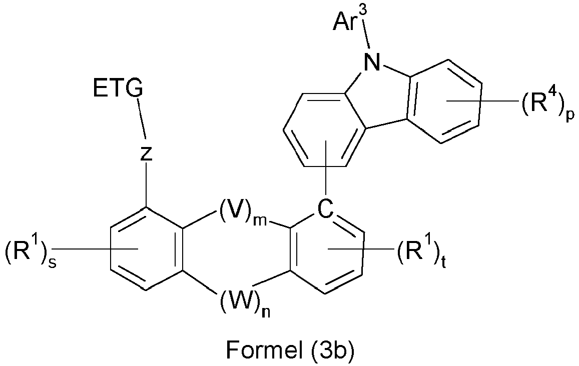 wobei p eine ganze Zahl von 1 bis 4, bevorzugt genau 2 und ganz bevorzugt genau 1 ist.

Eine weitere bevorzugte Verbindung im Sinne der vorliegenden Erfindung ist eine der folgenden Formel (3c)

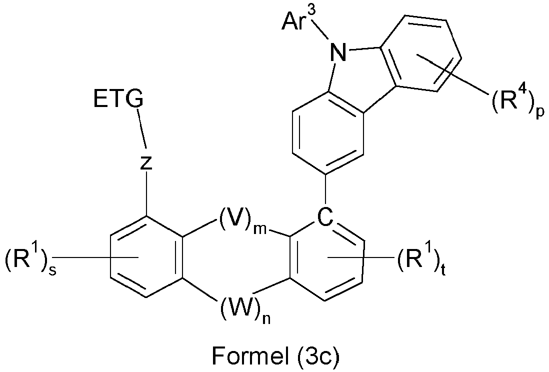

In einer weiteren bevorzugten Ausführungsform betrifft die vorliegende Erfindung eine Verbindung der allgemeinen Formel (12) 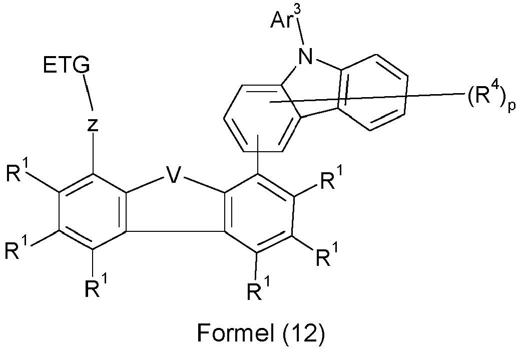 wobei V gleich O oder S ist und wobei für die verwendeten Indizes und Symbole die hierin aufgeführten Definitionen und bevorzugten Ausführungsformen gelten. Es ist ganz bevorzugt, wenn V in de Verbindung der Formel (12) gleich O ist.

In einer weiteren bevorzugten Ausführungsform betrifft die vorliegende Erfindung eine Verbindung der allgemeinen Formel (13) 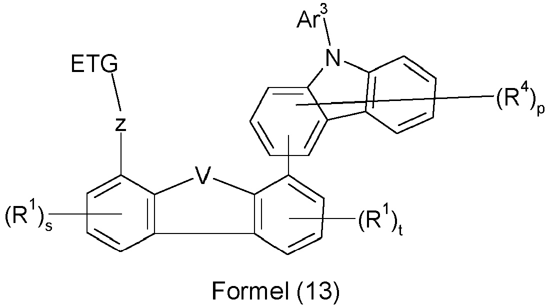 wobei V gleich O oder S ist und wobei für die verwendeten Indizes und Symbole die hierin aufgeführten Definitionen und bevorzugten Ausführungsformen gelten und wobei die aromatischen Ringe A und A' jeweils maximal einen Substituenten R¹ haben , d.h, s ist gleich 0 oder 1 und t ist gleich 0 oder 1, wobei s + t gleich 0, 1 oder 2 sein kann. Es ist ganz bevorzugt, wenn V in der Verbindung der Formel (13) gleich O ist.

In einer ganz bevorzugten Ausführungsform betrifft die vorliegende Erfindung eine Verbindung der allgemeinen Formel (14) 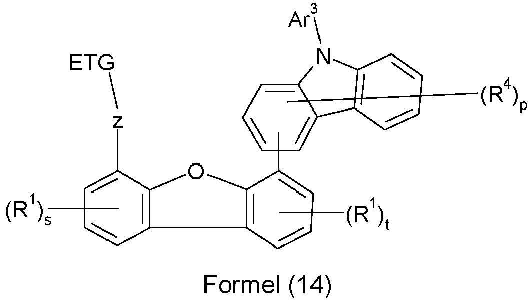 wobei für die verwendeten Indizes und Symbole die hierin aufgeführten Definitionen und bevorzugten Ausführungsformen gelten und wobei die aromatischen Ringe A und A' jeweils maximal einen Substituenten R¹ haben.

In einer ganz besonders bevorzugten Ausführungsform betrifft die vorliegende Erfindung eine Verbindung der allgemeinen Formel (15) 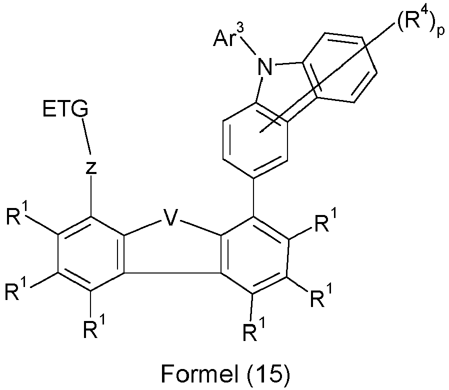 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist.

In einer ganz besonders bevorzugten Ausführungsform betrifft die vorliegende Erfindung eine Verbindung der allgemeinen Formel (16) 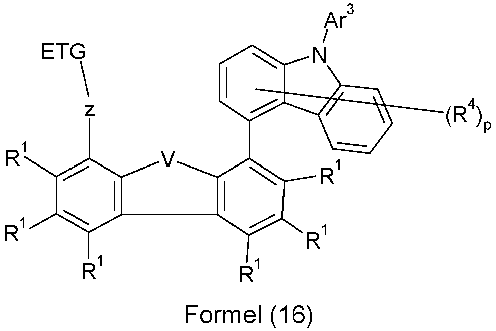 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist.

In einer ganz besonders bevorzugten Ausführungsform betrifft die vorliegende Erfindung eine Verbindung der allgemeinen Formel (17) 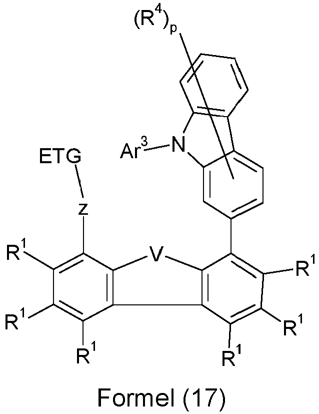 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist.

In einer ganz besonders bevorzugten Ausführungsform betrifft die vorliegende Erfindung eine Verbindung der allgemeinen Formel (18) 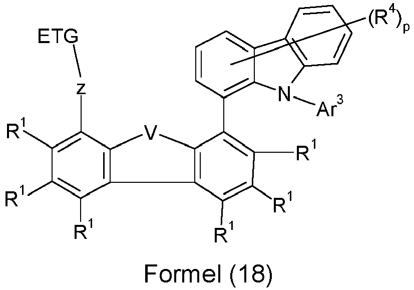 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist.

In einer weiteren ganz besonders bevorzugten Ausführungsform betrifft die vorliegende Erfindung eine Verbindung der allgemeinen Formel (19) 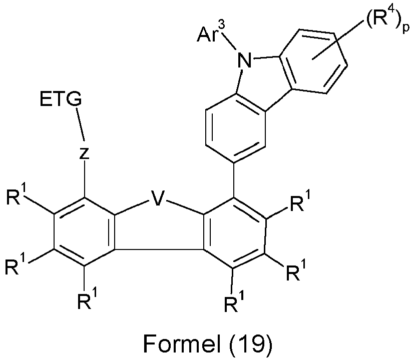 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist und wobei p eine ganze Zahl von 1 bis 4, bevorzugt von 1 oder 2, ganz bevorzugt genau 2 und insbesondere bevorzugt genau 1 ist.

In einer weiteren ganz besonders bevorzugten Ausführungsform betrifft die vorliegende Erfindung eine Verbindung der allgemeinen Formel (20) 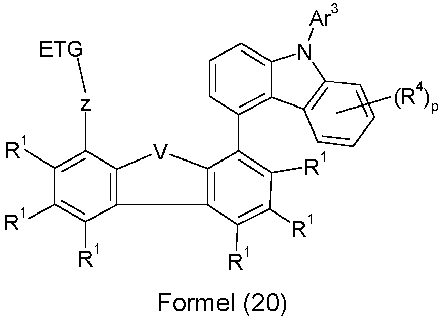 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist und wobei p eine ganze Zahl von 1 bis 4, bevorzugt von 1 oder 2, ganz bevorzugt genau 2 und insbesondere bevorzugt genau 1 ist.

In einer weiteren ganz besonders bevorzugten Ausführungsform betrifft die vorliegende Erfindung eine Verbindung der allgemeinen Formel (21) 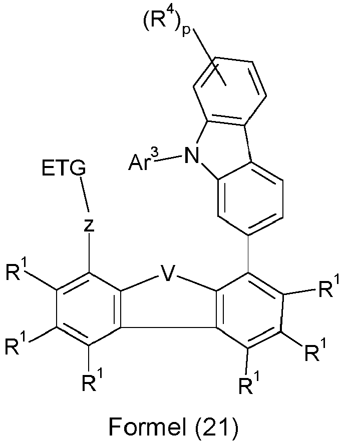 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist und wobei p eine ganze Zahl von 1 bis 4, bevorzugt von 1 oder 2, ganz bevorzugt genau 2 und insbesondere bevorzugt genau 1 ist.

In einer weiteren ganz besonders bevorzugten Ausführungsform betrifft die vorliegende Erfindung eine Verbindung der allgemeinen Formel (22) 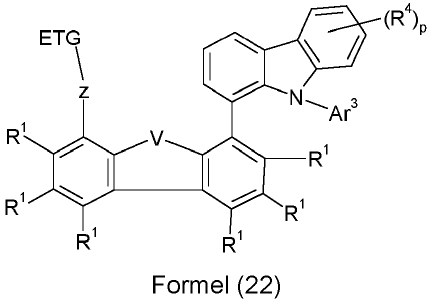 wobei für die verwendeten Symbole die hierin aufgeführten Definitionen und bevorzugten Ausführungsformen gelten und wobei die beiden mit R¹ substituierten aromatischen Ringe jeweils maximal einen Substituenten R¹ haben, wobei ganz bevorzugt ist, wenn R¹ an den beiden mit R¹ substituierten Ringen A und A' gleich H ist und wobei p eine ganze Zahl von 1 bis 4, bevorzugt von 1 oder 2, ganz bevorzugt genau 2 und insbesondere bevorzugt genau 1 ist.

Z ist bevorzugt eine Einfachbindung oder ein bivalenter aromatischer oder heteroaromatischer, bevorzugt ein aromatischer Ring oder Ringsystem mit 5 bis 60 Ringatomen, wobei bevorzugt ist, wenn der Ring oder das Ringsystem weder mit dem Ringsystem enthaltend die Ringe A und A' noch mit der ETG verbrückt ist, wobei bevorzugt ist, wenn Z eine Pyridylen-, Pyrimidylen-, Phenylen-, Biphenylen- oder Fluoren-, Spiro-, Terphenylen-, Thiophen-, Furan-, Dibenzofuran- oder Dibenzothiophengruppe ist, wobei eine Phenylen-, Biphenylen- oder Terphenylengruppe besonders und eine Phenylengruppe ganz besonders bevorzugt ist.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

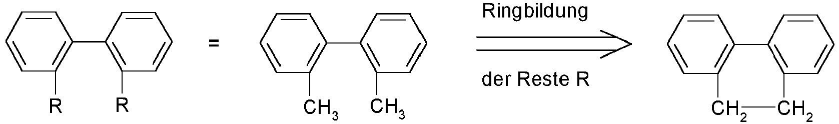

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

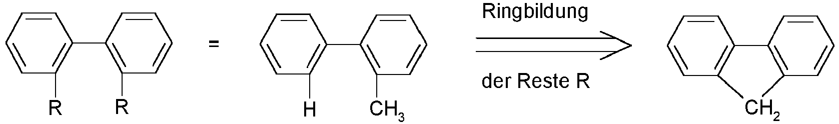

Unter einer kondensierten Arylgruppe wird dabei eine Arylgruppe verstanden, welche zwei oder mehr aromatische Ringe enthält, die miteinander kondensiert sind, d. h. eine oder mehr aromatische Bindungen miteinander teilen. Eine entsprechende Definition gilt für Heteroarylgruppen. Beispiele für kondensierte Arylgruppen ungeachtet der Zahl ihrer Ringatome sind Naphthyl, Anthracenyl, Pyrenyl, Phenanthrenyl und Perylenyl. Beispiele für kondensierte Heteroarylgruppen sind Chinolinyl, Indolyl, Carbazolyl, und Acridinyl.

Es folgen allgemeine Definitionen für chemische Gruppen im Rahmen der vorliegenden Anmeldung:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie dies beispielsweise in Benzimidazol, Chinolin oder Phenanthrolin der Fall ist.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Die Gruppe ETG ist vorzugsweise eine elektronenarme heteroaromatische Gruppe, die mit einem oder mehreren Resten R¹ substituiert sein kann. Noch stärker bevorzugt sind heteroaromatische Gruppen mit 6 aromatischen Ringatomen, von denen mindestens eines, bevorzugt 2 und ganz bevorzugt mindestens drei ein N-Atom ist, oder heteroaromatische Gruppen mit 5 aromatischen Ringatomen, von denen mindestens 2 Heteroatome sind, bevorzugt mindestens eines davon ein N-Atom, das mit R¹ substituiert sein kann, wobei an diese Gruppen jeweils auch weitere Aryl- oder Heteroarylgruppen ankondensiert sein können.

Elektronenarme heteroaromatische Gruppen sind, beispielsweise, ausgewählt aus den folgenden Gruppen. 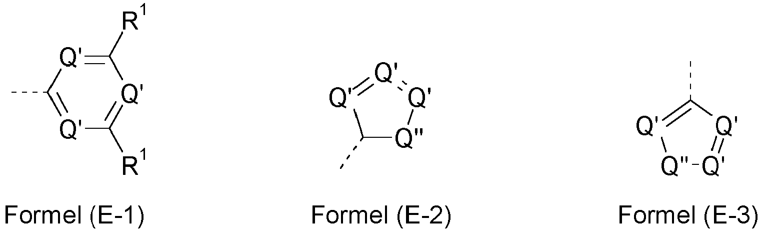 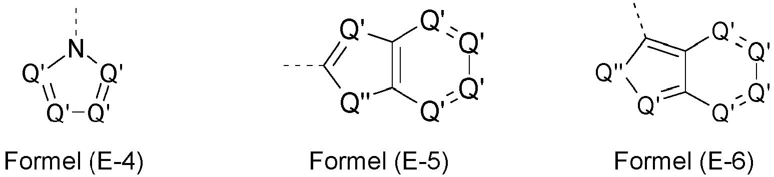 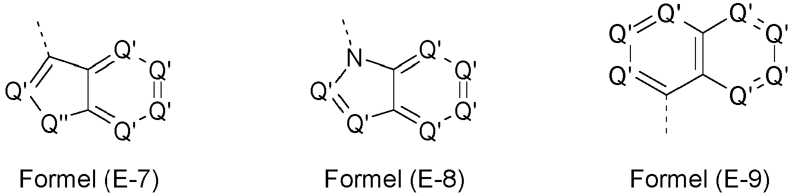 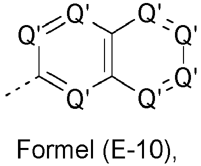 wobei die gestrichelte Bindung die Anbindungsposition markiert, R¹ wie oben definiert ist und
- Q': bei jedem Auftreten gleich oder verschieden CR¹ oder N darstellt, und
- Q": NR¹, O oder S ist;
wobei wenigstens ein Q' gleich N und/oder wenigstens ein Q" gleich NR¹ ist.

Weitere Beispiele für elektronenarme heteroaromatische Gruppen sind: Pyridine, Pyrazine, Pyrimidine, Pyridazine, 1,2,4-Triazine, 1,3,5-Triazine, Chinoline, Isochinoline, Chinoxaline, Pyrazole, Imidazole, Benzimidazole, Thiazole, Benzothiazole, Oxazole oder Benzooxazole, die jeweils mit R¹ substituiert sein können. Weitere elektronentransportierende Gruppen sind mit einem oder mehreren Resten R¹ substituiertes Pyridin, Pyrazin, Pyrimidin, Pyridazin und 1,3,5-Triazin.

Ganz bevorzugte elektronenarme heteroaromatische Gruppen sind ausgewählt aus den folgenden Gruppen

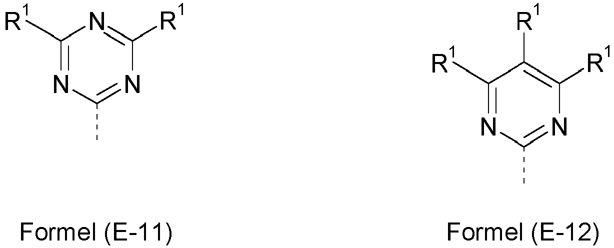

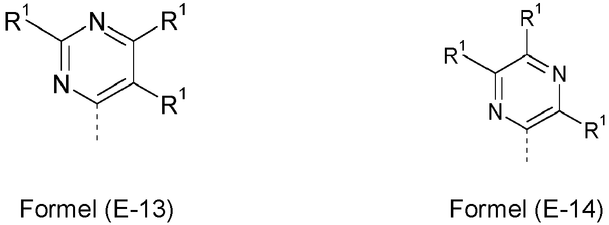

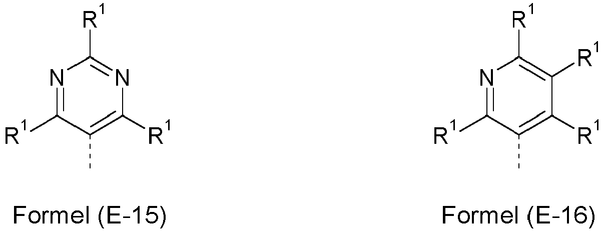

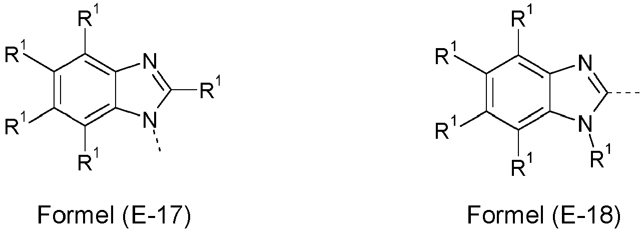

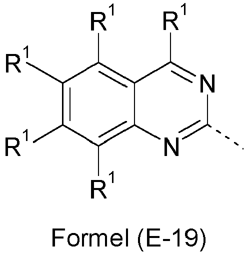

Die Substituenten R¹ in der ETG sind vorzugsweise ausgewählt aus der Gruppe bestehend aus H oder ein aromatisches oder heteroaromatisches Ringsystem mit 5 bis 60 aromatischen Ringatomen, das jeweils durch einen oder mehrere Reste R² substituiert sein kann, wobei die Gruppen der Formel (E-11), (E-17) und (E-18) noch bevorzugter sind und die Gruppe der Formel (E-11) am meisten bevorzugt ist.

Beispiele ganz besonders bevorzugter ETGs sind die folgenden Gruppen, die mit einem oder mehreren voneinander unabhängigen Resten R² substituiert sein können, wobei die gestrichelten Bindungen die Bindungspositionen zu den Gruppen Ar¹ und Ar² kennzeichnen.

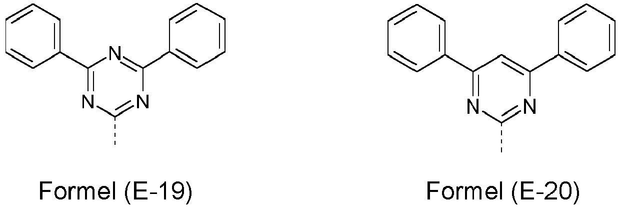

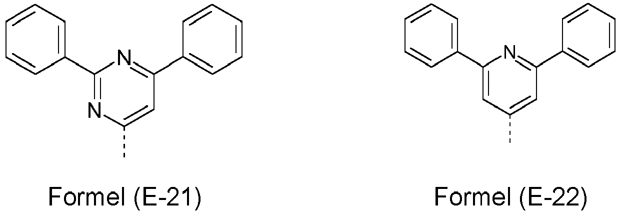

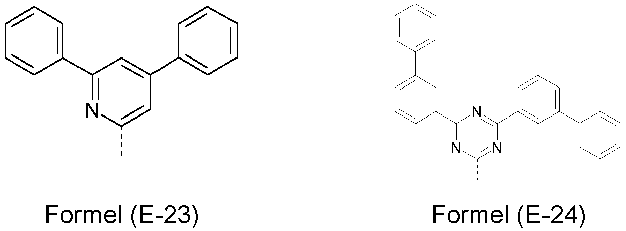

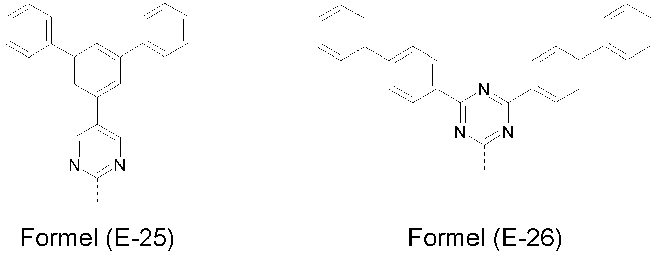

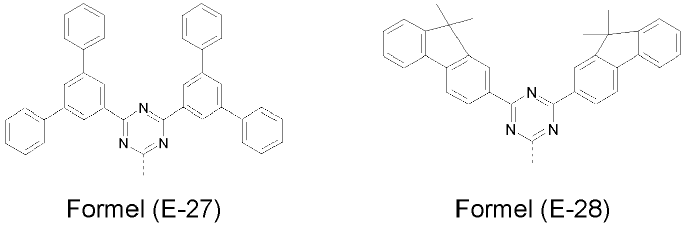

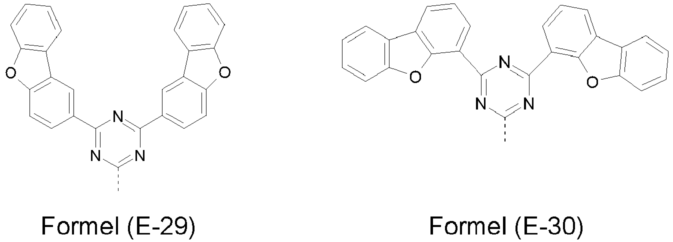

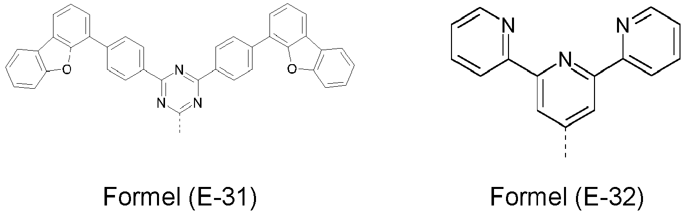

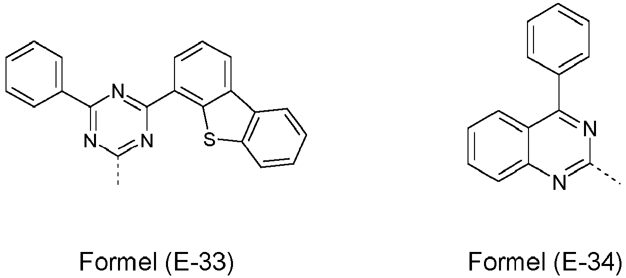

Die Elektronentransportgruppe weist bevorzugt eine LUMO (lowest unoccupied molecular orbital) Energie auf, die niedriger als -1.3 eV ist, ganz bevorzugt niedriger als -2.5 eV und ganz besonders bevorzugt niedriger als -2.7 eV. Die LUMO-Energie der Elektronentransportgruppe mit dem weiter unten beschriebenen Verfahren ermittelt. Bei den Berechnungen wird die Elektronentansportgruppe isoliert betrachtet. Bei den Gruppen der Formeln (E-1) bis (E-34) wird die getrichelte Bindung gegen eine Bindung zu einem Phenylrest ersetzt. Beispielsweise wird für die Berechnung der LUMO-Energie der Elektronentrasportgruppe der Formel (E-19) die folgende Verbindung berechnet:

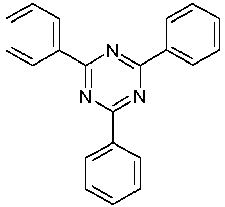

Molekülorbitale, insbesondere auch das highest occupied molecular orbital (HOMO) und das lowest unoccupied molecular orbital (LUMO), deren Energieniveaus sowie die Energie des niedrigsten Triplettzustands T₁ bzw. des niedrigsten angeregten Singulettzustands S₁ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:
HOMO(eV) = ((HEh*27.212)-0.9899)/1.1206
LUMO(eV) = ((LEh*27.212)-2.0041)/1.385

Diese Werte sind im Sinne dieser Anmeldung als HOMO- bzw. LUMO-Energieniveaus der Materialien anzusehen.

Der niedrigste Triplettzustand T₁ ist definiert als die Energie des Triplettzustands mit der niedrigsten Energie, der sich aus der beschriebenen quantenchemischen Rechnung ergibt.

Der niedrigste angeregte Singulettzustand S₁ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Weiter bevorzugt ist dieElektronentransportgruppe dadurch charakterisiert, dass die Elektronenmobilität µ- 10⁻⁶ cm²/(Vs) oder mehr beträgt, ganz bevorzugt beträgt sie 10⁻⁵ cm²/(Vs) oder mehr und ganz besonders bevorzugt beträgt sie 10⁻⁴ cm²/(Vs) oder mehr. Analog zu der Ermittlung der LUMO-Energien der ETG, wird die ETG dazu isoliert betrachtet. Weiterhin werden in den Gruppen der Formeln (E-1) bis (E-34) die getrichelten Bindung gegen eine Bindung zu einem Phenylrest ersetzt.

In den Verbindungen nach Formel (2) ist das LUMO bevorzugt auf der Elektronentransportgruppe lokalisiert sein. Ganz bevorzugt ist, wenn das LUMO mehr als 80 % auf elektrontransportierende Gruppe lokalisiert ist, noch bevorzugter, wenn das LUMO überhaupt nicht auf der Carbazolgruppe lokalisiert ist. Insbesondere bevorzugt ist, wenn die Beträge des HOMOs und des LUMOs der erfindungsgemäßen Verbindung überhaupt nicht überlappen. Der Fachmann hat keinerlei Schwierigkeiten die Überlappung der Beträge der Orbitale (Überlappungsintegral der Beträge der Wellenfunktionen) zu ermitteln. Dazu wird das hierin angegebene Berechnungsverfahren verwendet und Orbitale mit einer Aufenthaltswahrscheinlichkeit von 90% angenommen.

Ar³ ist bevorzugt ein aromatischer oder heteroaromatischer Ring oder Ringsystem mit 5 bis 30 Ring-atomen, wobei der Ring oder das jeweils durch einen oder mehrere Reste R² substi-tuiert sein kann, die durch einen oder mehrere Reste R³ substituiert sein können.

Ar³ ist ganz bevorzugt ein aromatischer Ring oder Ringsystem mit 5 bis 30 Ring-atomen, wobei der Ring oder das jeweils durch einen oder mehrere Reste R² substi-tuiert sein kann, die durch einen oder mehrere Reste R³ substituiert sein können, wobei noch bevorzugter ist, wenn Ar³ unsusbtituiert ist.

Ganz besonders bevorzugte aromatische Gruppen sind Phenyl, Biphenyl, Terphenyl und Quarterphenyl.

Ar³ ist ganz bevorzugt ein heteroaromatischer Ring oder Ringsystem mit 5 bis 30 Ring-atomen, wobei der Ring oder das jeweils durch einen oder mehrere Reste R² substi-tuiert sein kann, die durch einen oder mehrere Reste R³ substituiert sein können, wobei noch bevorzugter ist, wenn Ar³ unsusbtituiert ist.

Ganz besonders bevorzugte heteroaromatische Gruppen sind Furan, Dibenzofuran, Thiophen, Benzothiophen, Dibenzothiophen, Carbazol, Phenanthridin und Chinoxalin.

Die Reste R⁴ sind in einer bevorzugten Ausführungsform untereinander oder die Reste R⁴ sind mit dem Carbazol durch O, S, NAr³, oder C(R²)₂ verbrückt.

In einer ganz bevorzugten Ausführungsform ist p=2, und wobei ganz besonders bevorzugt ist, wenn die beiden Reste R⁴ einen Ringschluss bilden, so insbesondere bevorzugt Indenocarbazole oder Indolocarbazole entstehen, die wiederum mit einem oder meherern voneinander unabhängigen Resten R² substituiert sein können.

Die Reste R⁴ sind in noch einer bevorzugten Ausführungsform untereinander unverbrückt.

Die Reste R⁴ sind in noch einer bevorzugten Ausführungsform mit dem Carbazol unverbrückt.

In einer bevorzugten Ausführungsform der vorliegenden Efindung ist R⁴ gleich oder verschieden bei jedem Auftreten N(R²)₂, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R² substituiert sein kann, ein aromatisches oder heteroaromatisches Ringsystem mit 5 bis 60 aromatischen Ringatomen, das jeweils durch einen oder mehrere Reste R² substituiert sein kann, oder eine Aryloxy-, Arylalkoxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen; dabei können zwei oder mehrere benachbarte Reste R⁴ miteinander ein mono- oder polycyclisches, aliphatisches oder aromatisches Ringsystem bilden.

In einer ganz bevorzugten Ausführungsform der vorliegenden Efindung ist R⁴ gleich oder verschieden bei jedem Auftreten ein aromatisches oder heteroaromatisches Ringsystem mit 5 bis 60 aromatischen Ringatomen, das jeweils durch einen oder mehrere Reste R² substituiert sein kann, oder eine Aryloxy-, Arylalkoxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen; dabei können zwei oder mehrere benachbarte Reste R⁴ miteinander ein mono- oder polycyclisches, aliphatisches oder aromatisches Ringsystem bilden.

Besonders bevorzugt ist, wenn R⁴ gleich oder verschieden bei jedem Auftreten ein aromatisches oder heteroaromatisches Ringsystem mit 5 bis 60 aromatischen Ringatomen, das jeweils durch einen oder mehrere Reste R² substituiert sein kann ist; dabei können zwei oder mehrere benachbarte Reste R⁴ miteinander ein polycyclisches, aromatisches Ringsystem bilden.

Ganz besonders bevorzugte aromatische oder heteroaromatische Ringsysteme für R⁴ sind Phenyl, Biphenyl, Terphenyl, Quarterphenyl, Carbazol, Dibenzofuranyl, die mit einem oder meheren R² substituiert sein können und ganz besonders bevorzugt unsubstituiert sind.

Die erfindungsgemäßen Verbindungen können gemäß Schemata 1 und 2 dargestellt werden.

Die entsprechenden Monoboronsäuren (a) können durch Suzuki-Kupplung und anschließende Silylierung (Schema 1) hergestellt werden. Eine weitere Möglichkeit ist, ausgehend von dem den Monobromiden durch Buchwald-Kupplung und anschließende Silylierung die entsprechende Monoboronsäuren herzustellen (Schema 2). Die Reaktion von diesen Monoboronsäuren via Suzuki-Kupplung mit entsprechenden Arylbromiden oder Arylchloriden führt zu den Zielverbindungen. 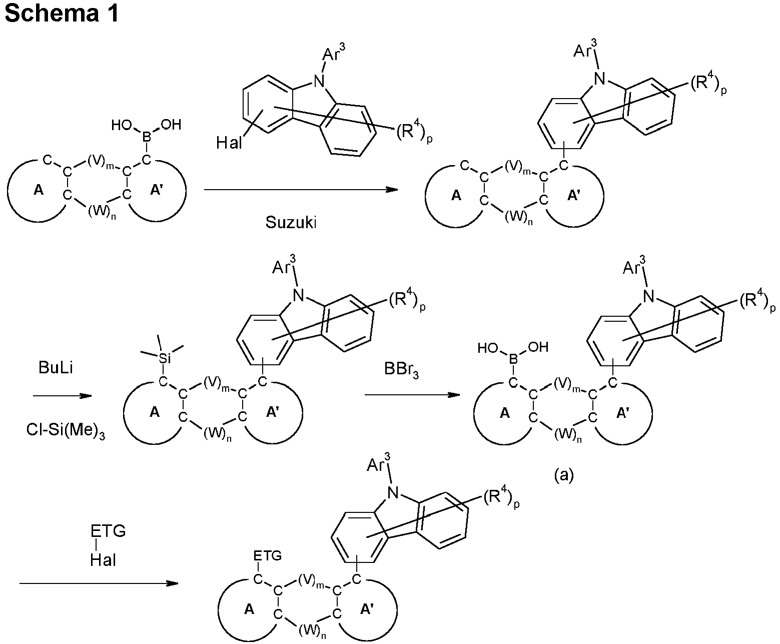 wobei für die verwendeten Indizes und Symbole die oben angegenenen Definitionen sowie deren bevorzugten Ausführungsformen gelten.

Die Suzuki Reaktion ist dem Fachmann gut bekannt und es bereitet ihm keinerlei Schwierigkeiten die Reaktionen sowie bekannte Variationen hiervon auf die erfindungsgemäßen Verbindungen anzuwenden, um sie, unter Berücksichtigung des allgemeinen Fachwissens, in der beanspruchten Breite herzustellen. Des Weiteren können sowohl bei der Suzuki- als auch bei der Buchwald-Reaktion die chemischen Funktionalitäten zwischen Substituent und der Struktur enthaltend die Ringe A und A' ausgetauscht werden. Das bedeutet, dass auch der Substituent enthaltend ETG oder Carbazol die Boronsäure enthalten kann, wohingegen die Struktur enthaltend die Ringe A und A' das Halogenid enthält. Die folgenden Schemata verdeutlichen beispielhaft anhand spezifischerer Fälle die Anwendung der genannten Verfahren, wobei obige Definitionen für die verwendeten Symbole und Indizes gelten. Hal steht für Halogenide ist bevorzugt Br oder I.

Die vorliegende Erfindung betrifft daher auch ein Verfahren zur Herstellung der erfindungsgemäßen Verbindung mittel Suzuki-Kupplung.

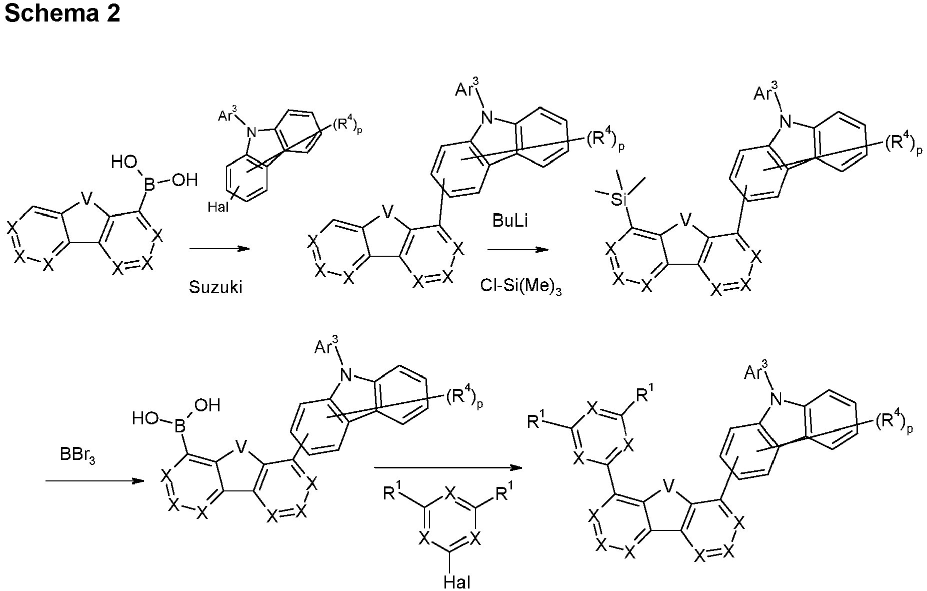

Eine weitere Möglichkeit zur Herstellung der erfindungsgemäßen Verbindungen besteht in der Umsetzung eines Dihalogenids (Hal = Br, I) mit 1 eq der ensprechenden Boronsäure und anschließebe Szuki-Kupplung zum gewünschten Produckt, wobei sich der Syntheseablauf ähnlichen Schritten wie in Schema 1 gezeigt bedient.

Eine weitere Möglichkeit ist die Umsetzung des Dihalogenids mit 2 eq der Boronsäure der ETG.

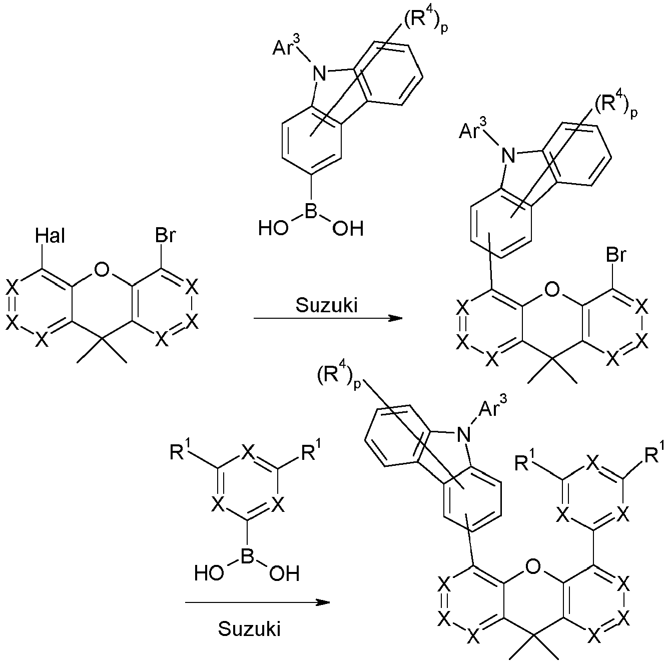

### Schema 4

Viele der Dihalogenide (b) oder Diboronsäuren (c) sind kommerziell erhältlich oder können wie in Schema 5 angegeben synthetisiert werden. Sie können anschließend über Suzuki-Kupplungen zu den gewünschten Produkten umgesetzt werden.

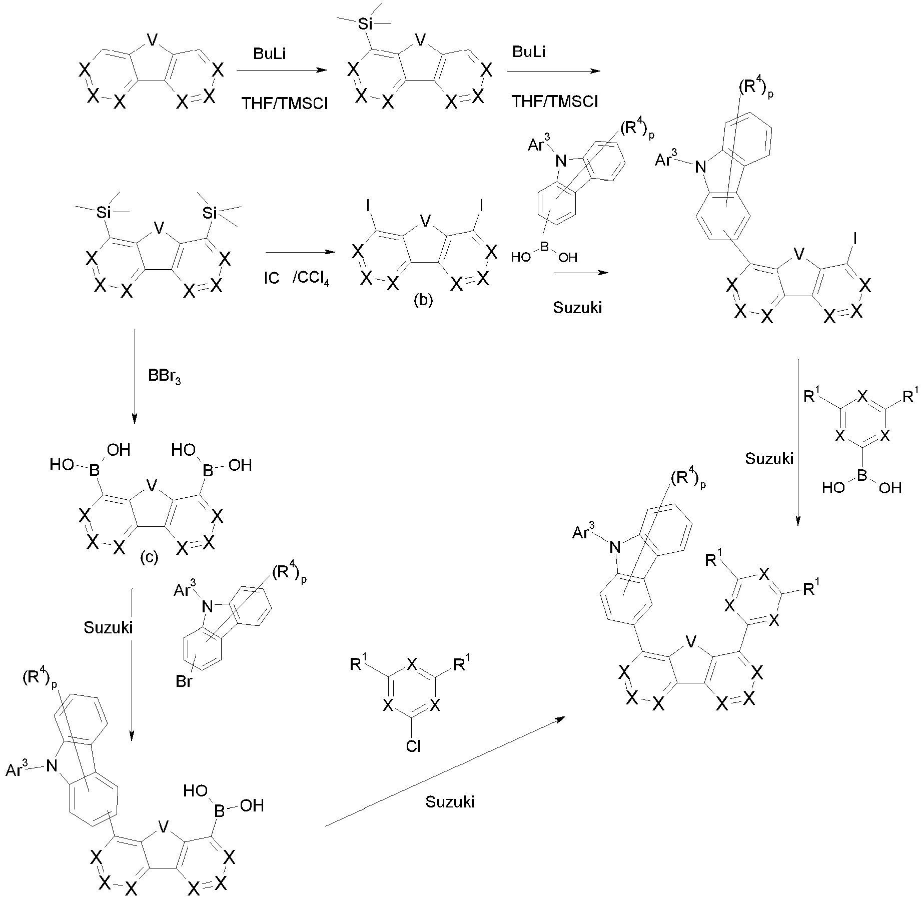

### Schema 5

Eine weitere Möglichkeit, erfindungsgemäße Verbindungen herzustellen ist die Umsetzung von Carbazol-Derivaten gefolgt von einer Ullmann- oder Buchwald-Kupplung.

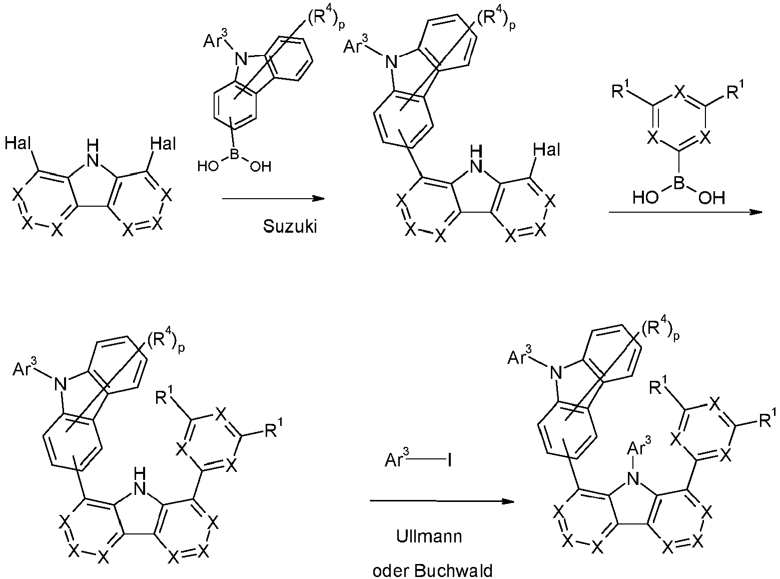

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die folgende Übersicht enthält eine beispielhafte Darstellung erfindungsgemäßer Verbindungen, die nach einem der hierin beschriebenen Verfahren hergestellt werden können.

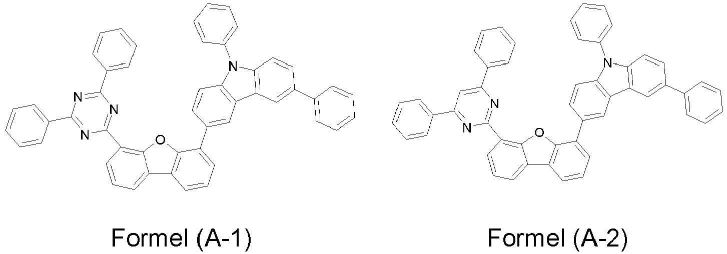

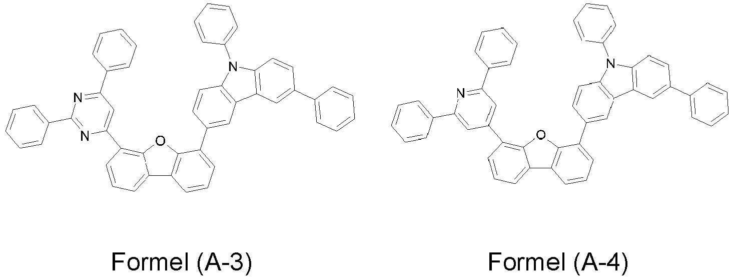

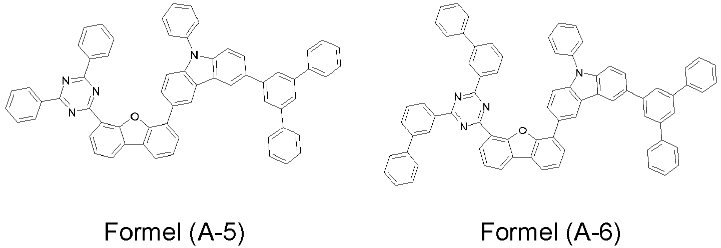

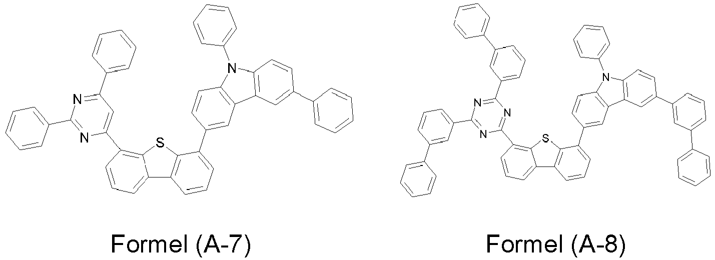

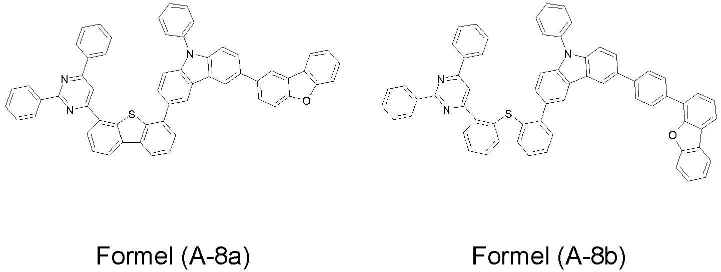

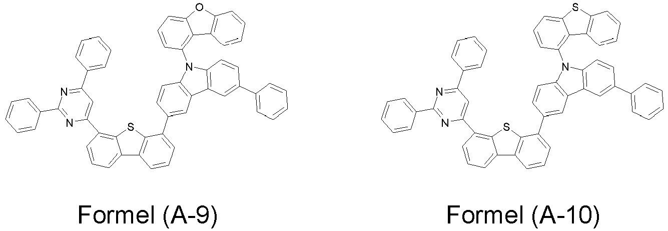

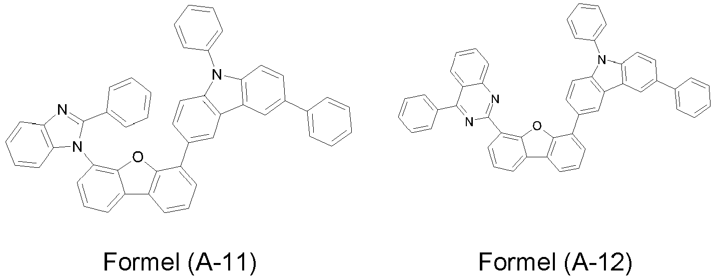

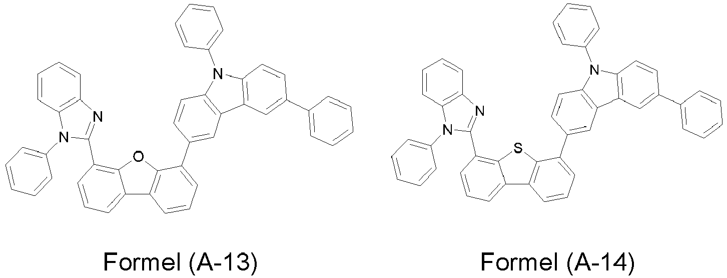

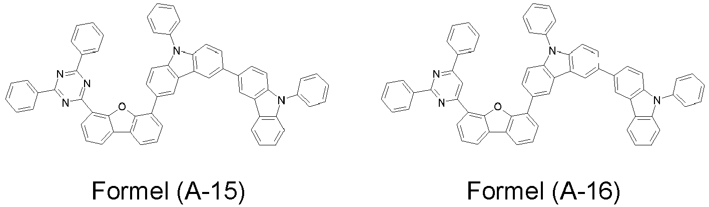

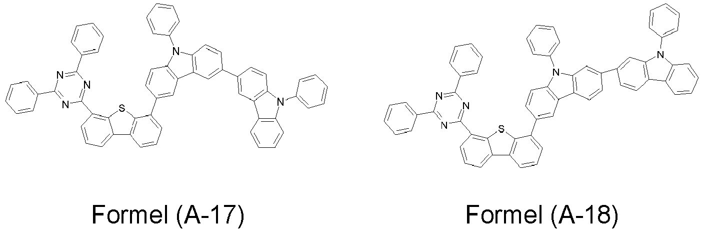

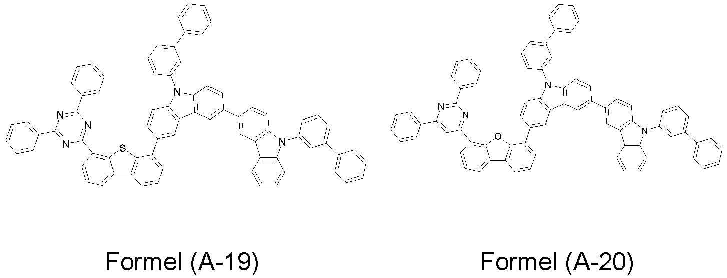

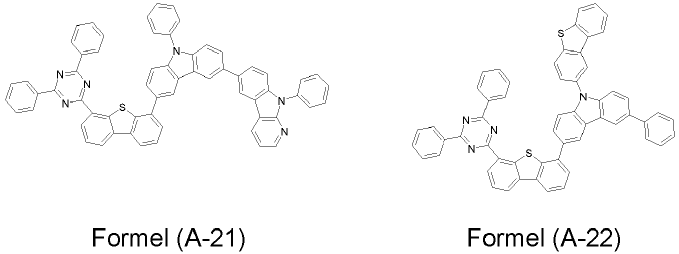

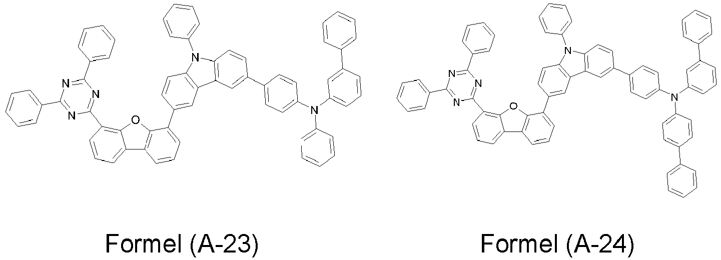

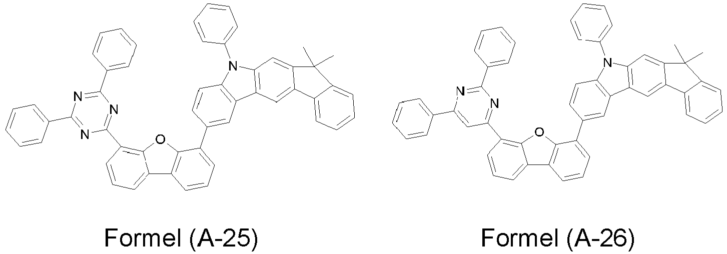

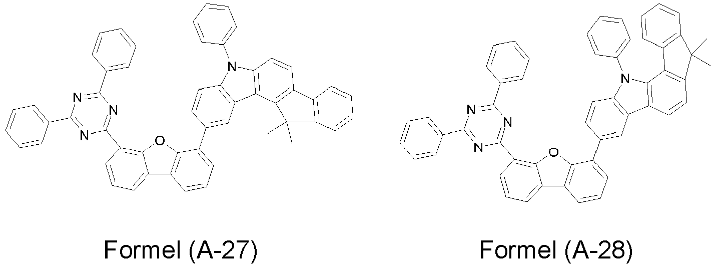

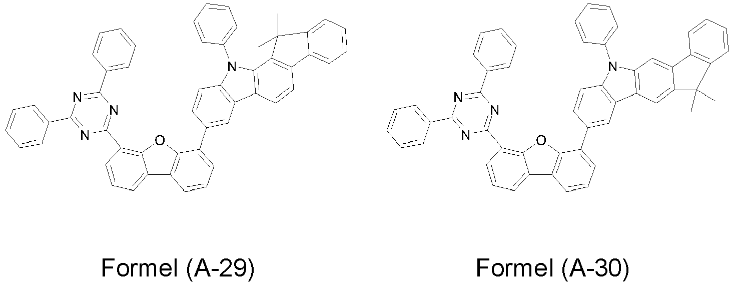

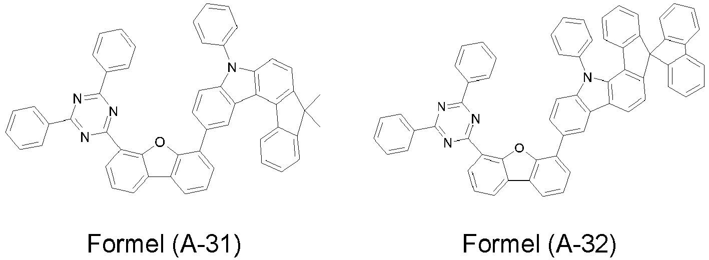

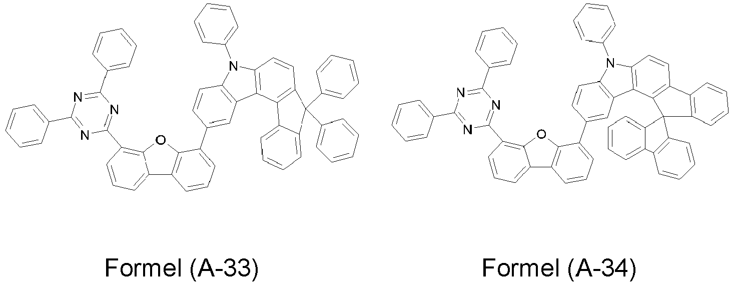

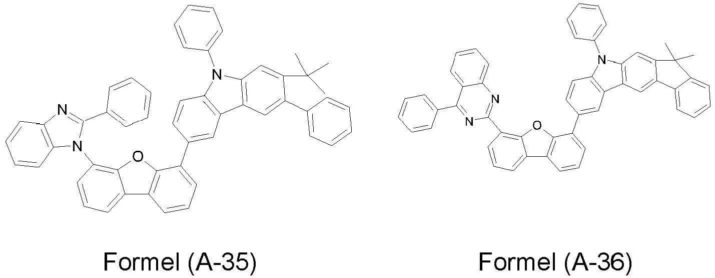

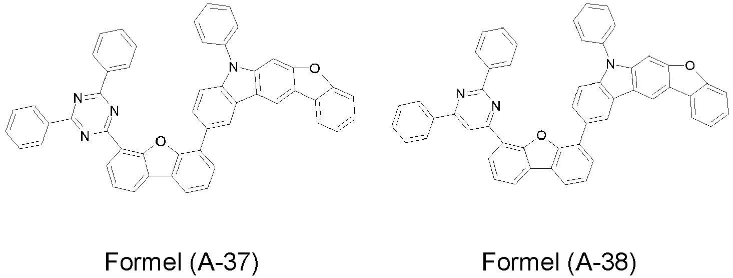

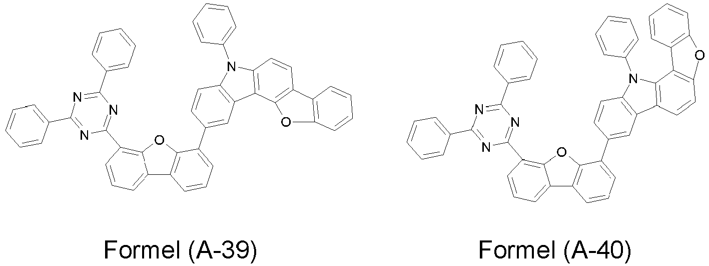

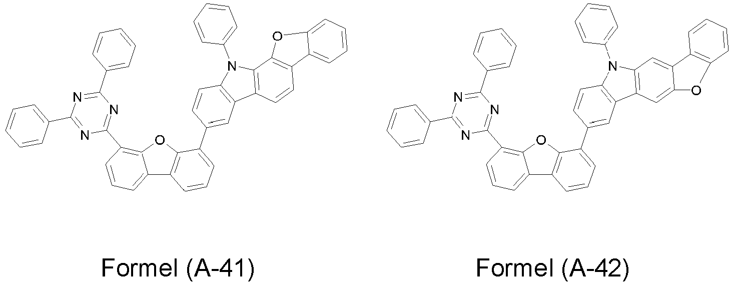

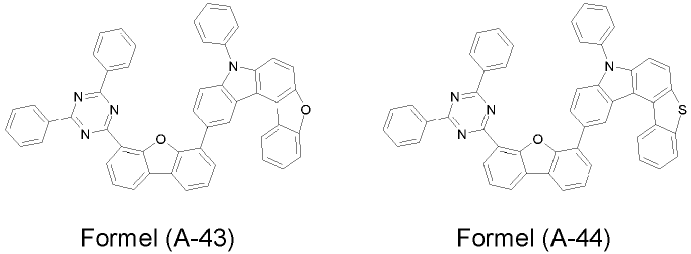

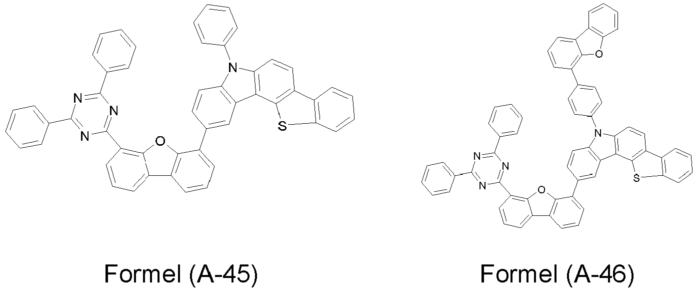

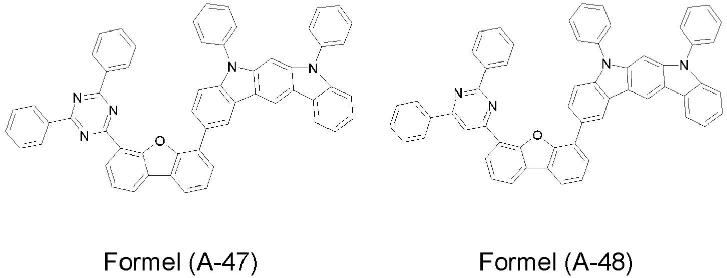

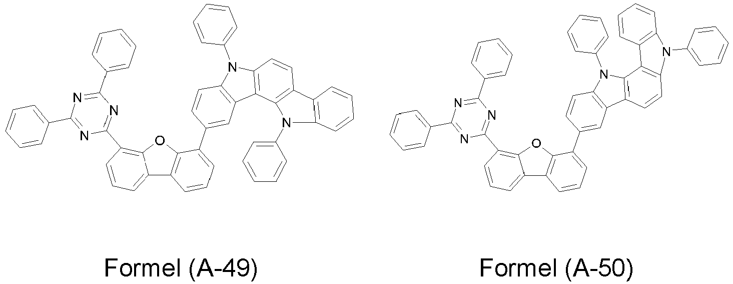

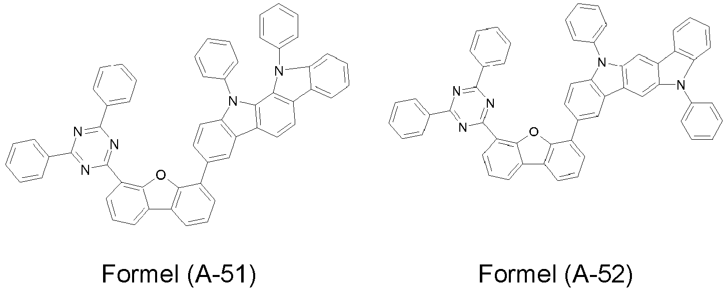

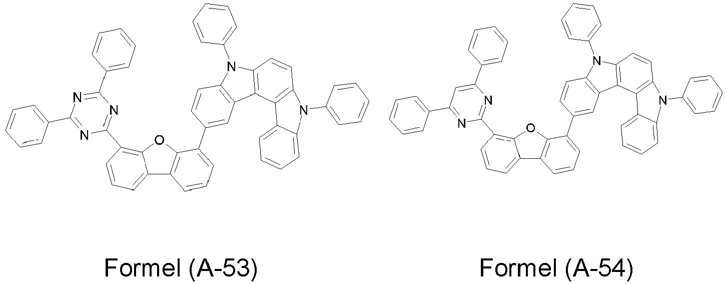

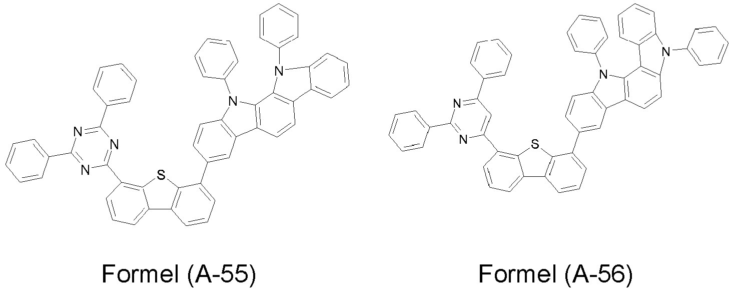

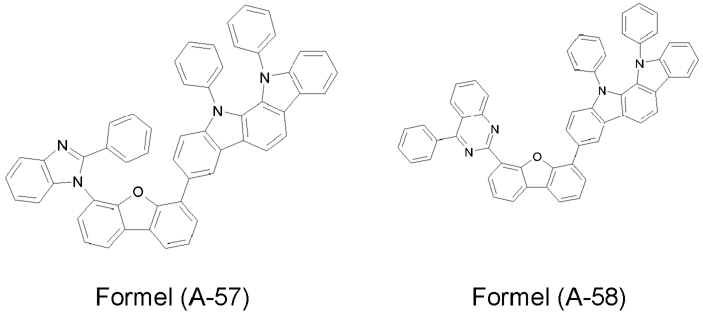

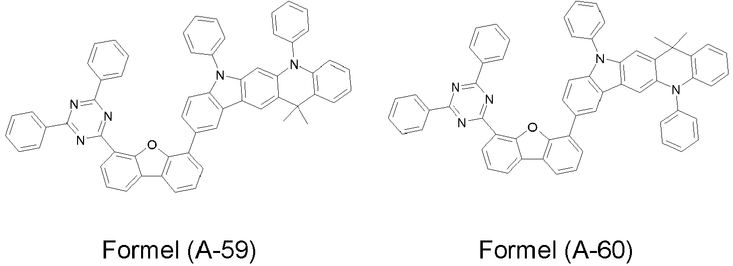

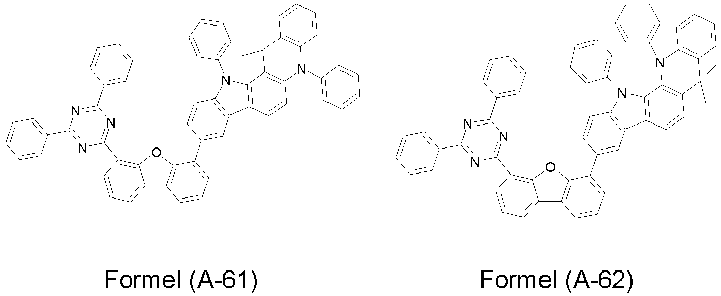

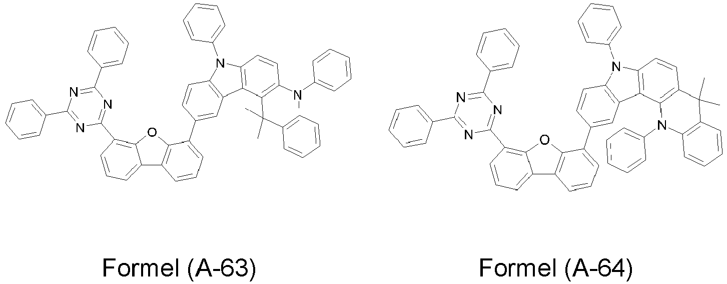

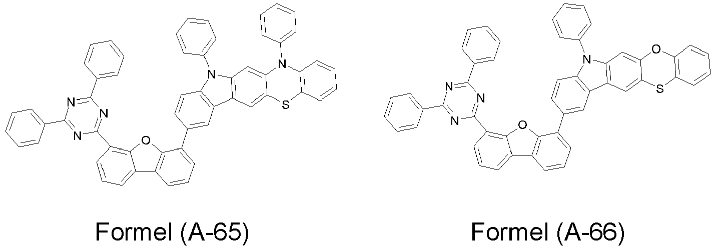

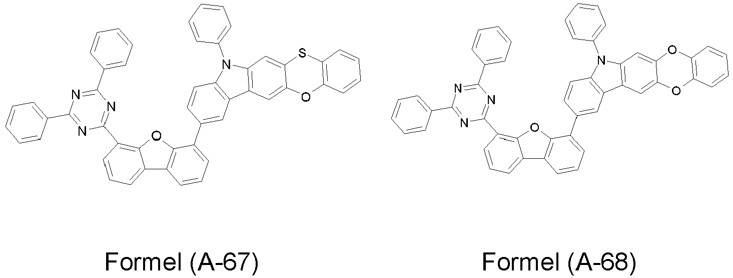

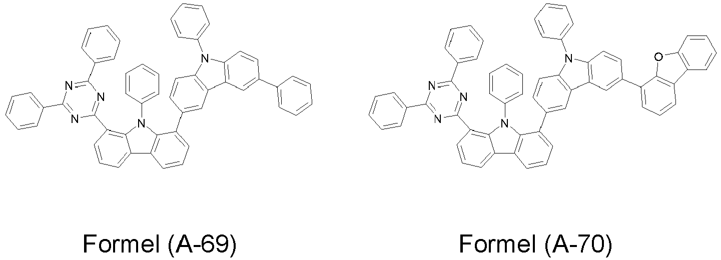

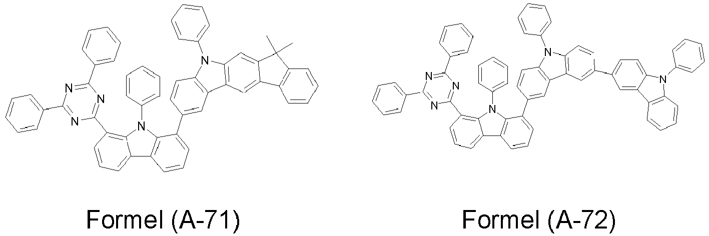

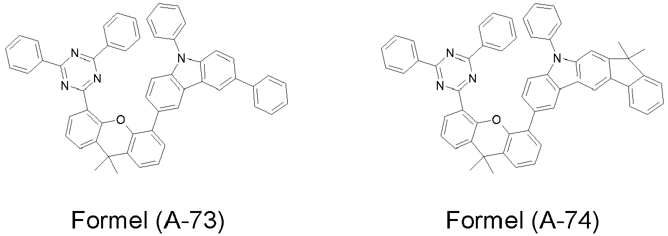

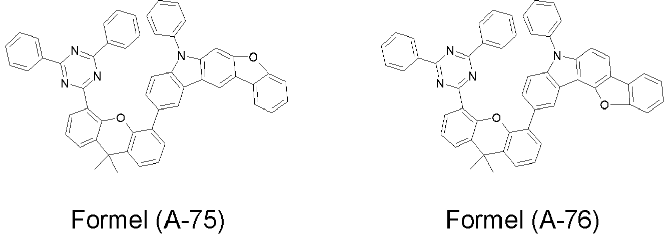

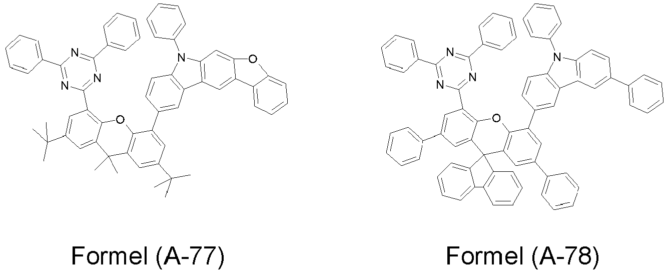

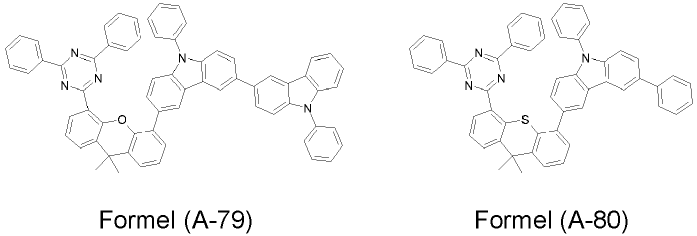

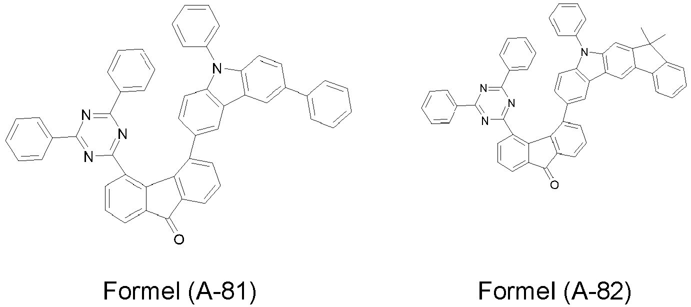

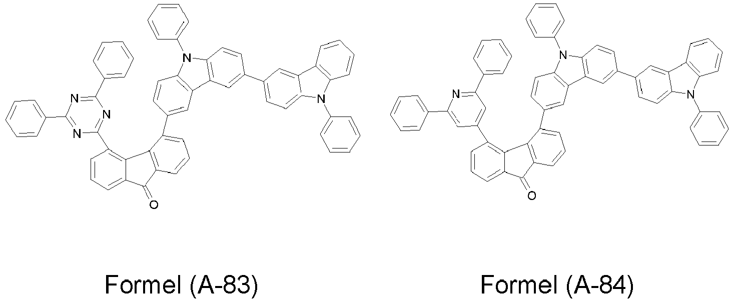

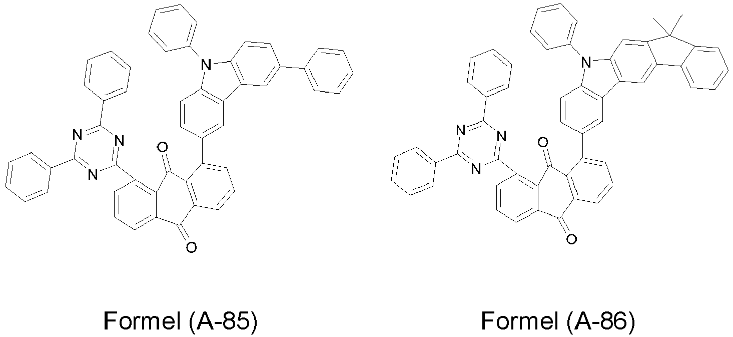

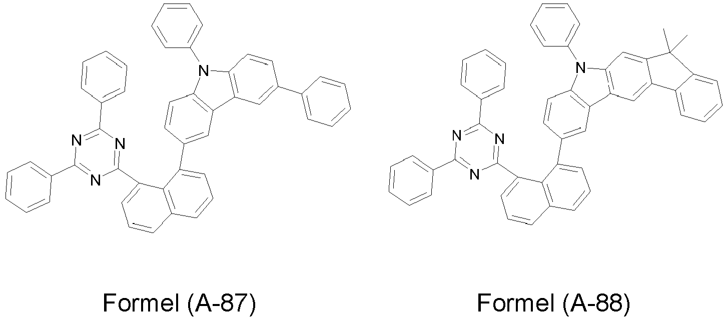

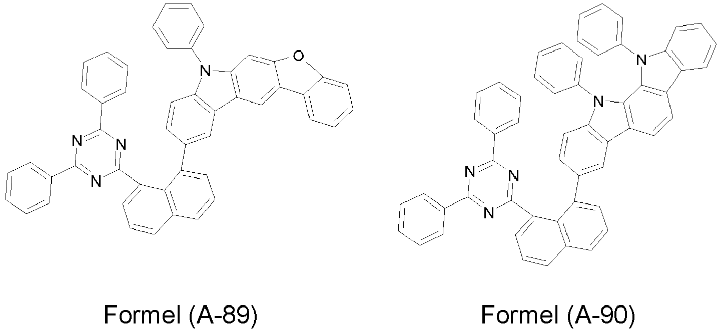

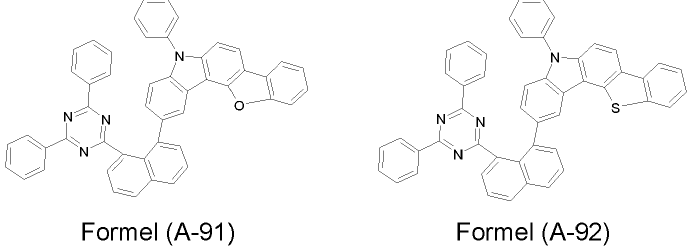

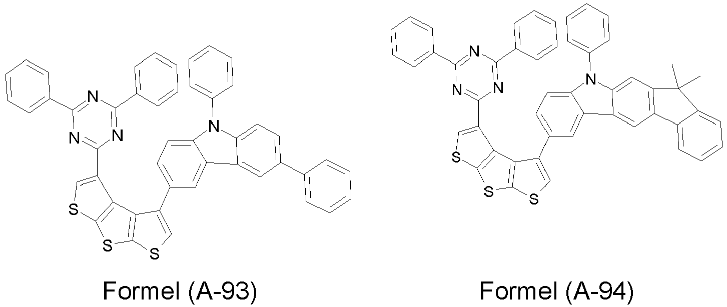

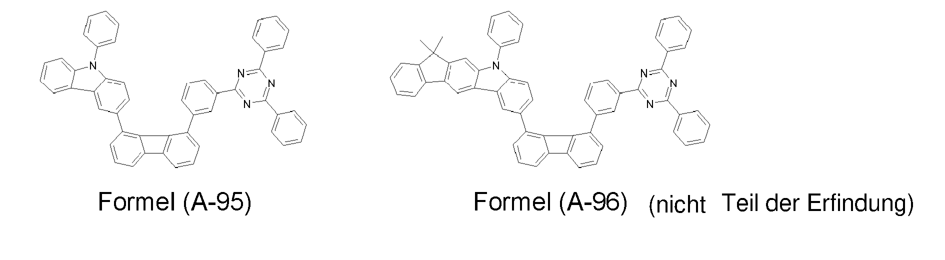

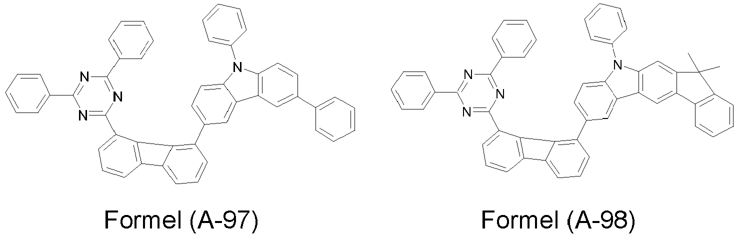

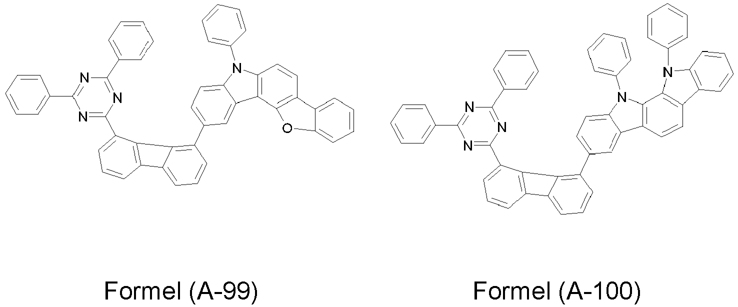

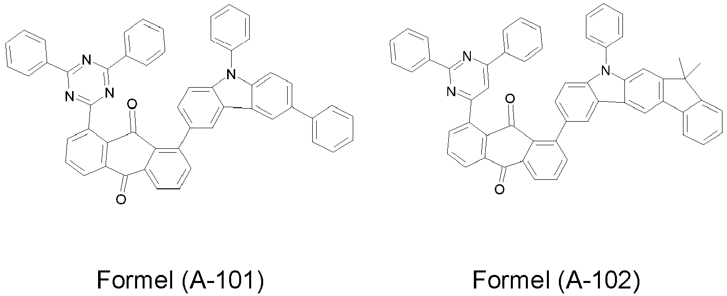

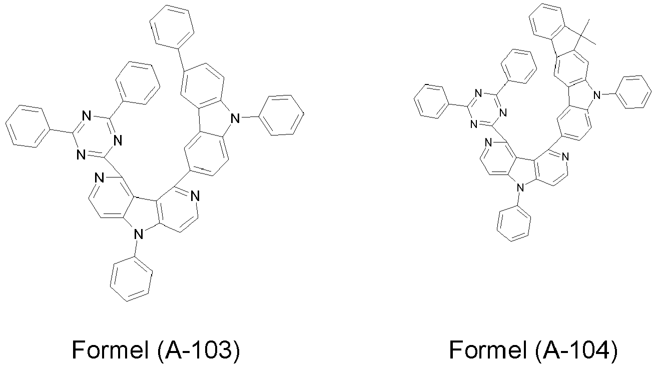

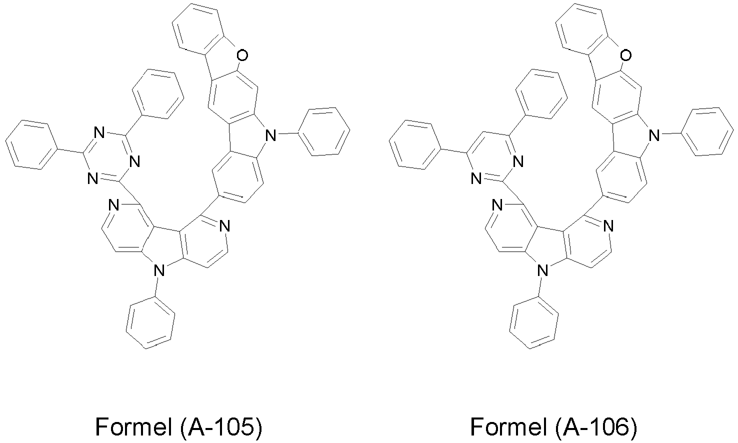

Weiterer Gegenstand der Erfindung ist die Verwendung einer Verbindung der Formel (2) in einer elektronischen Vorrichtung, bevorzugt in einer elektronentransportierenden und/oder in einer emittierenden Schicht.

Die erfindungsgemäße elektronische Vorrichtung ist bevorzugt gewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs, LECs oder LEECs), organischen Laserdioden (O-Laser) und organischen lichtemittierenden Dioden (OLEDs). Besonders bevorzugt sind die organischen Elektrolumineszenzvorrichtungen, ganz besonders bevorzugt die OLECs und OLEDs und insbesondere bevorzugt die OLEDs.

Die organische Schicht enthaltend die Verbindung der Formel (2) ist bevorzugt eine Schicht mit elektronentransportierender Funktion. Besonders bevorzugt ist sie eine Elektroneninjektionsschicht, Elektronentransportschicht, eine Lochblockierschicht oder eine emittierende Schicht.

In einer weiteren ganz besonders bevorzugten Ausführungsform wird die Verbindung der allgemeinen Formel (2) in einer emittierenden Schicht eingesetzt wird, insbesondere als Matrixmaterial.

Eine Lochtransportschicht gemäß der vorliegenden Anmeldung ist eine Schicht mit lochtransportierender Funktion, welche sich zwischen Anode und emittierender Schicht befindet.

Eine Elektronenransportschicht gemäß der vorliegenden Anmeldung ist eine Schicht mit elektronentransportierender Funktion, welche sich zwischen Kathode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wie oben bereits erwähnt, wird die Verbindung der Formel (2) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt. Dabei wird das Matrixmaterial der Formel (2) in der elektronischen Vorrichtung in Kombination mit einem oder mehreren Dotanden, vorzugsweise phosphoreszierenden Dotanden, vorhanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (2) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Weiterhin betrifft die vorliegende Erfindung eine Zusammensetzung enthaltend wenigstens eine Verbindung gemäß Formel (2) sowie wenigstens ein weiteres organisches Halbleitermaterial ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien, n-Dotanden und p-Dotanden.

Unter n-Dotanden werden hierin Reduktionsmittel, d.h. Elektronendonatoren verstanden. Bevorzugte Beispiele für n-Dotanden sind W(hpp)₄ und weitere elektronenreiche Metallkomplexe gemäß WO 2005/086251 A2, P=N-Verbindungen (z.B. WO 2012/175535 A1, WO 2012/175219 A1), Naphthylencarbodiimide (z.B. WO 2012/168358 A1), Fluorene (z.B. WO 2012/031735 A1), Radikale und Diradikale (z.B. EP 1837926 A1, WO 2007/107306 A1), Pyridine (z.B. EP 2452946 A1, EP 2463927 A1), N-heterocyclische Verbindungen (z.B. WO 2009/000237 A1) und Acridine sowie Phenazine (z.B. US 2007/145355 A1).

Unter p-Dotanden werden hierin Oxidationsmittel, d.h. Elektronenakzeptoren verstanden. Bevorzugte Beispiele für p-Dotanden sind F₄-TCNQ, F₆-TNAP, NDP-2 (Fa. Novaled), NDP-9 (Fa. Novaled), Chinone (z.B. EP 1538684 A1, WO 2006/081780 A1, WO 2009/003455 A1, WO 2010/097433 A1), Radialene (z.B. EP 1988587 A1, US 2010/102709 A1, EP 2180029 A1, WO 2011/131185 A1, WO 2011134458 A1, US 2012/223296 A1), S-haltige Übergangsmetallkomplexe (z.B. WO 2007/134873 A1, WO 2008/061517 A2, WO 2008/061518 A2, DE 102008051737 A1, WO 2009/089821 A1, US 2010/096600 A1), Bisimidazole (z.B. WO 2008/138580 A1), Phthalocyanine (z.B. WO 2008/058525 A2), Bora-Tetraazapentalene (z.B. WO 2007/115540 A1) Fullerene (z.B. DE 102010046040 A1) und Hauptgruppenhalogenide (z.B. WO 2008/128519 A2).

Die vorliegenden Erfindung betrifft auch eine Zusammensetzung enthaltend wenigstens eine Verbindung gemäß Formel (2) sowie wenigstens ein weiteres Matrixmaterial.

Die vorliegende Erfindung betrifft auch eine Zusammensetzung enthaltend wenigstens eine Verbindung gemäß Formel (2) sowie wenigstens ein wide band gap Material, wobei unter wide band gap Material ein Material im Sinne der Offenbarung von US 7,294,849 verstanden wird. Diese Systeme zeigen besondere vorteilhafte Leistungsdaten in elektrolumineszierenden Vorrichtungen.

Die vorliegende Erfindung betrifft auch eine Zusammensetzung enthaltend wenigstens eine Verbindung gemäß Formel (2) sowie wenigstens ein weiteres Matrixmaterial sowie wenigstens einen phosphoreszierenden Emitter.

Die vorliegende Erfindung betrifft auch eine Zusammensetzung enthaltend wenigstens eine Verbindung gemäß Formel (2) sowie wenigstens ein wide band gap Material sowie wenigstens einen phosphoreszierenden Emitter.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

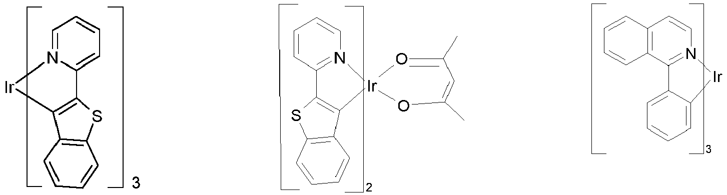

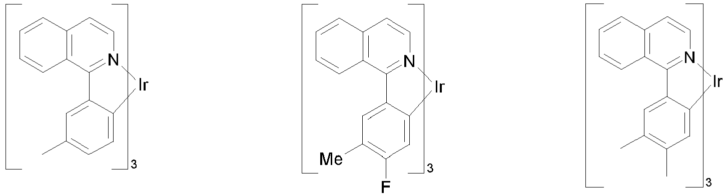

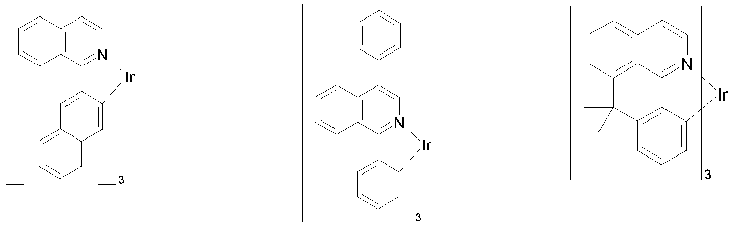

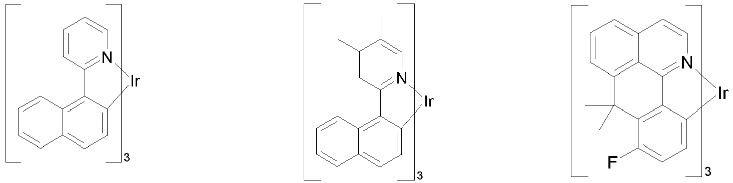

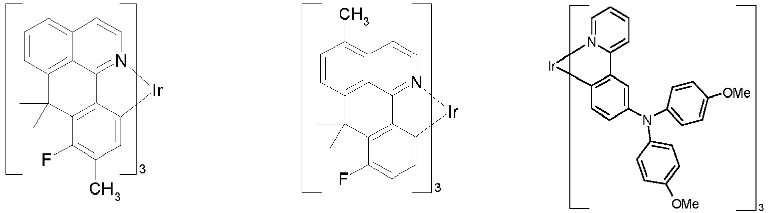

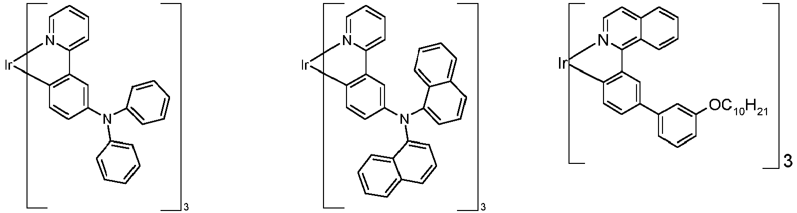

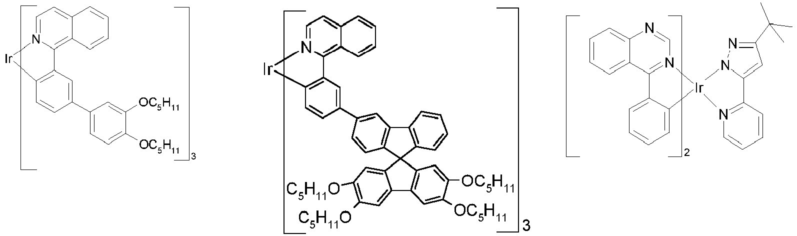

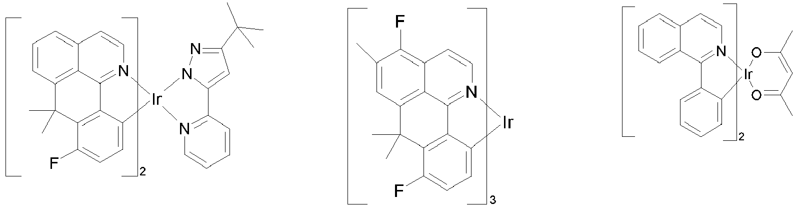

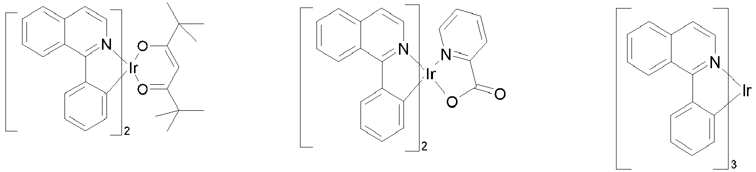

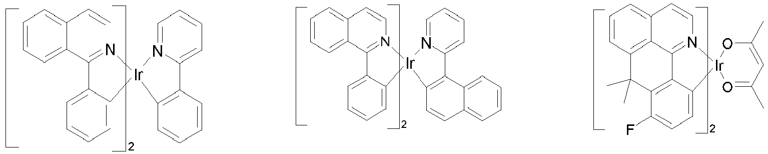

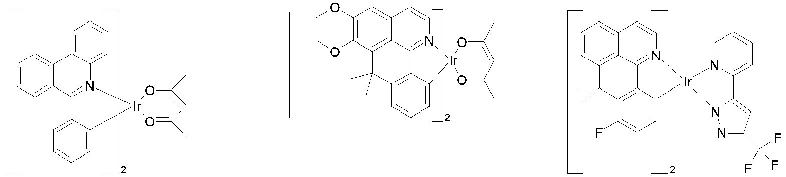

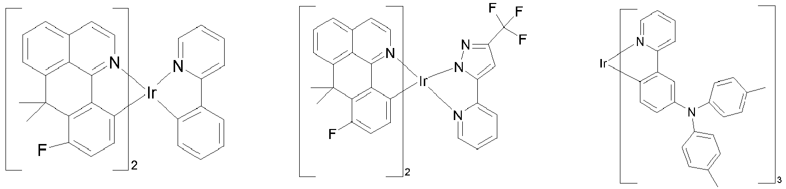

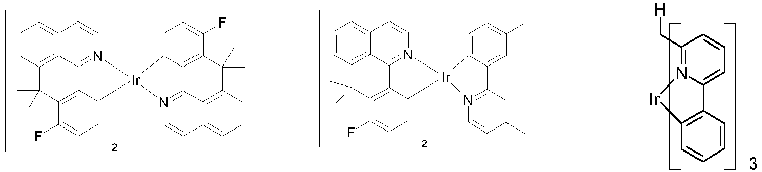

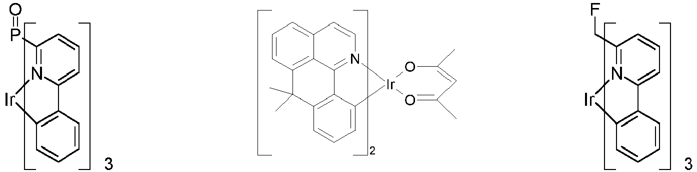

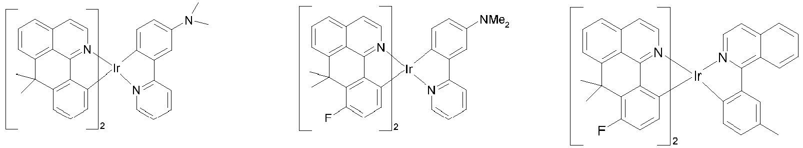

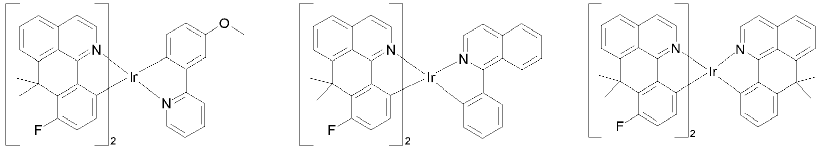

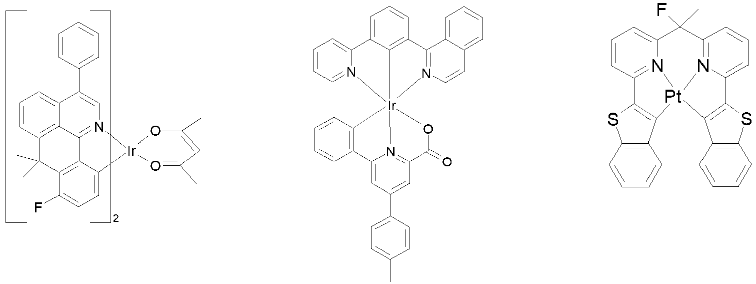

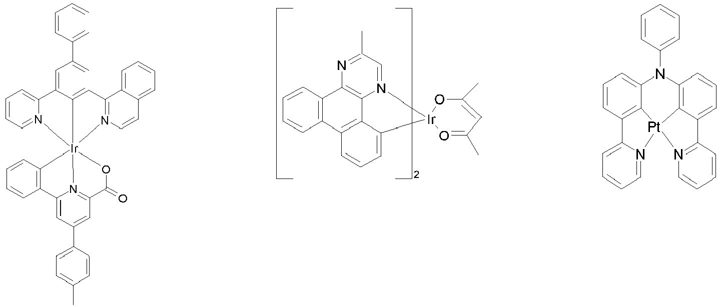

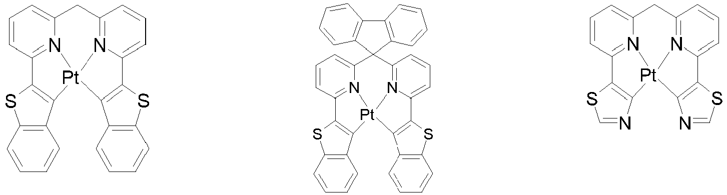

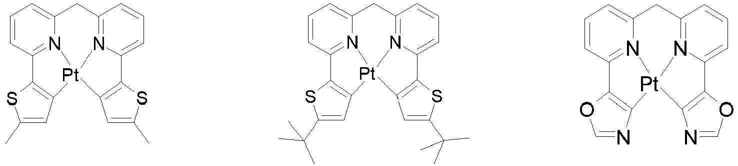

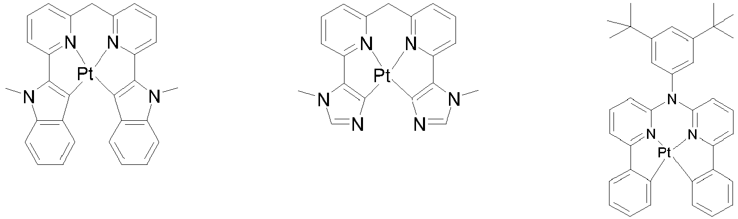

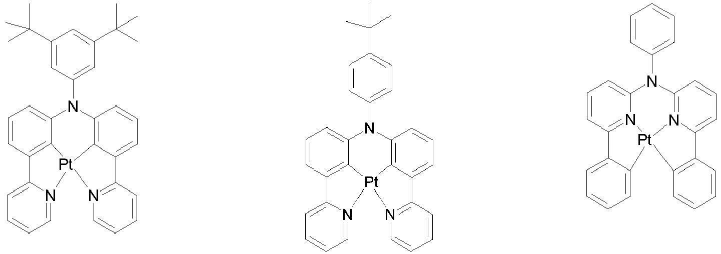

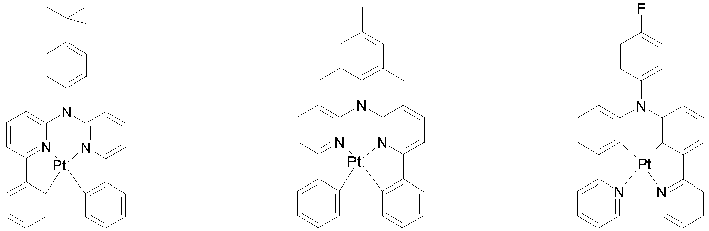

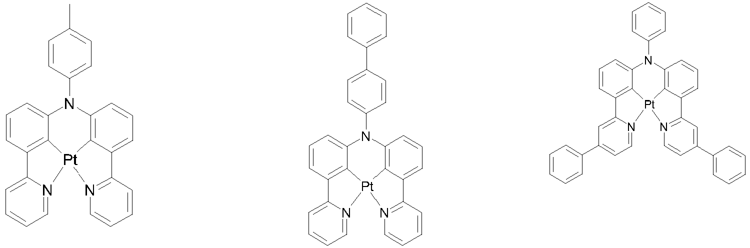

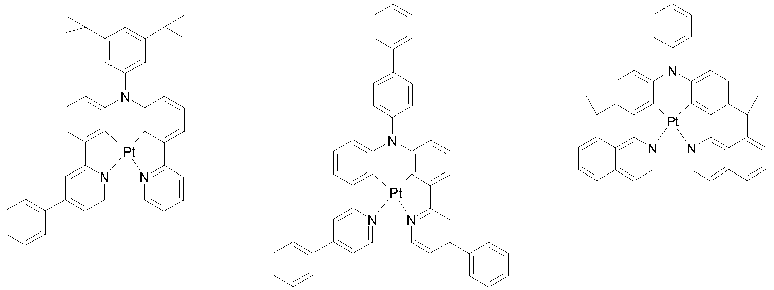

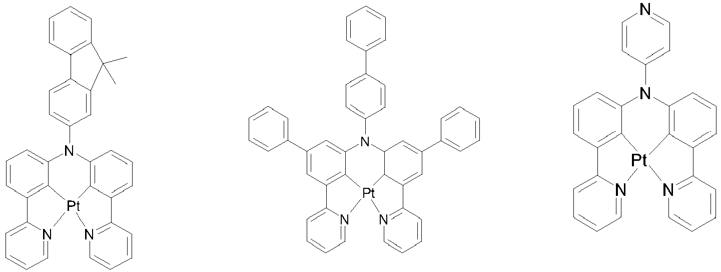

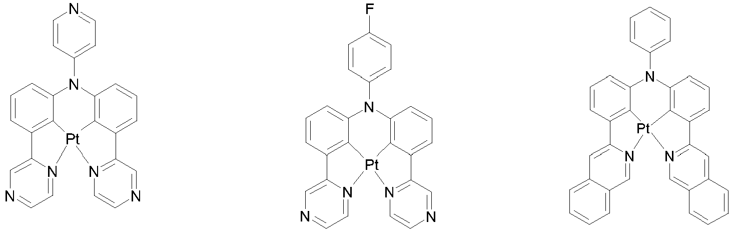

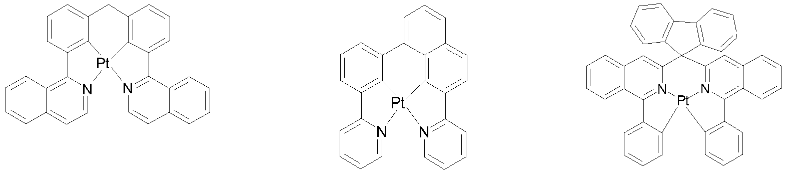

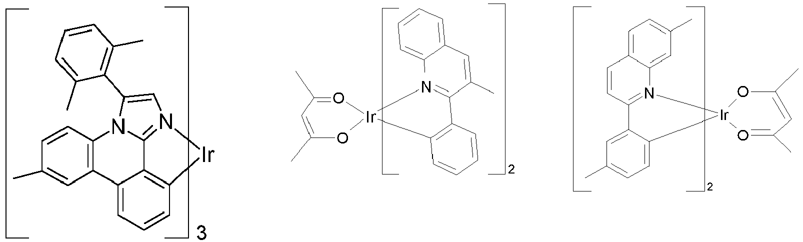

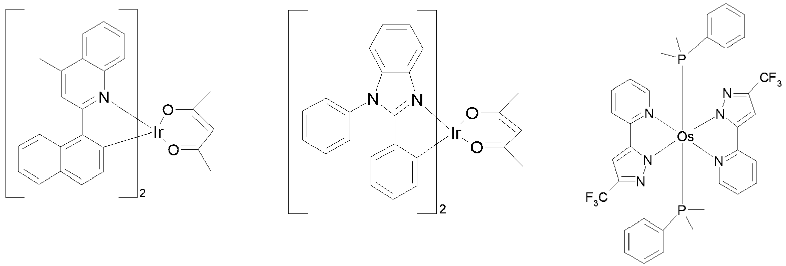

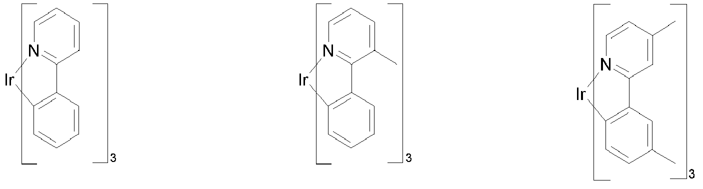

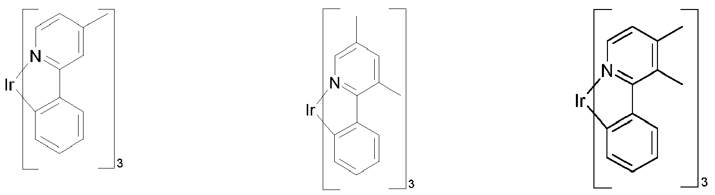

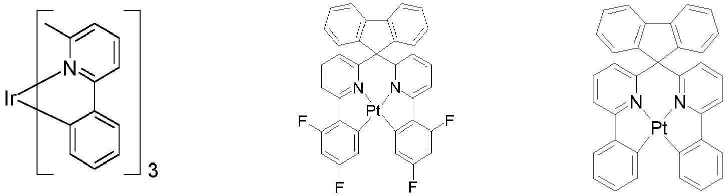

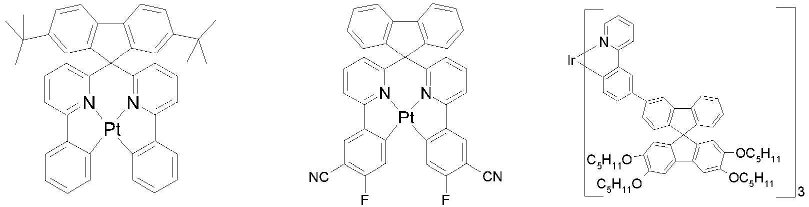

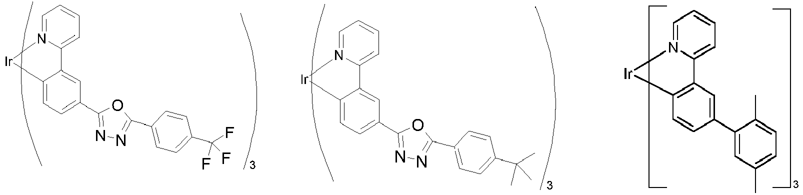

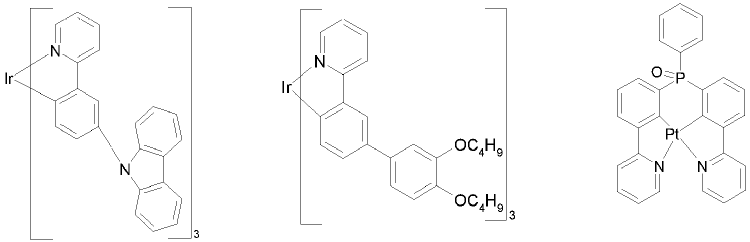

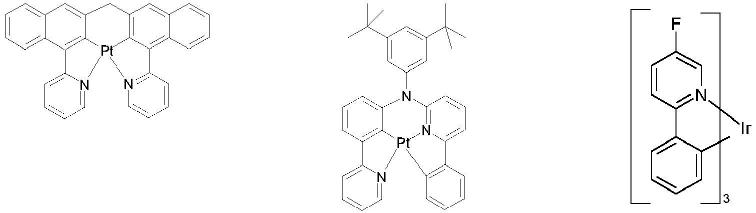

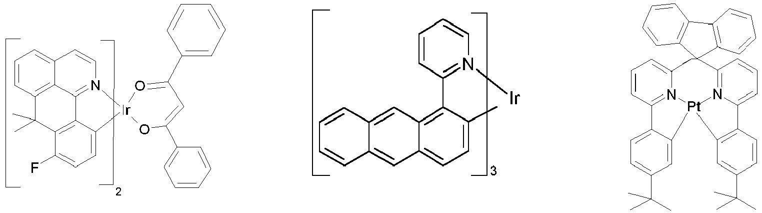

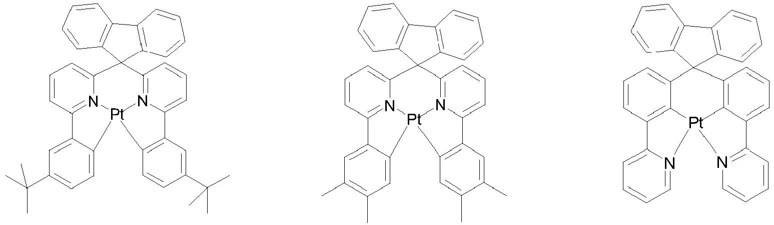

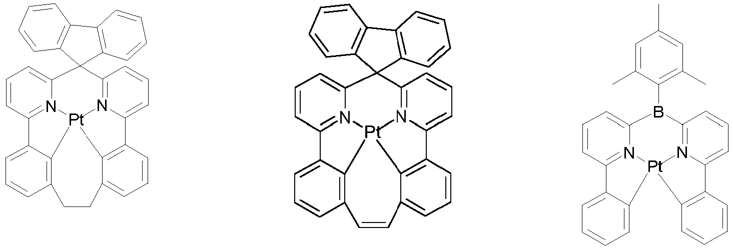

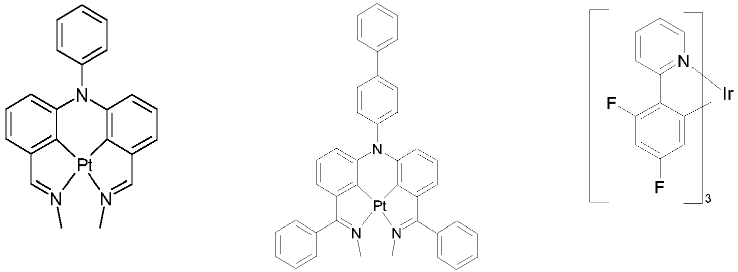

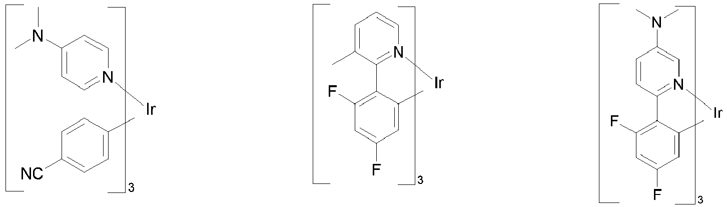

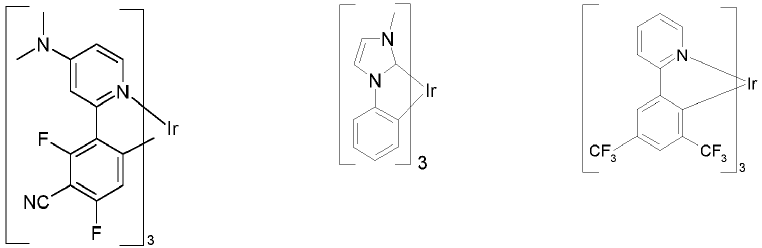

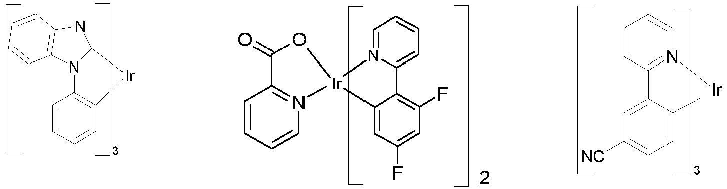

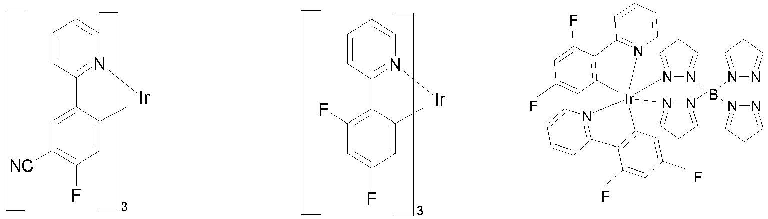

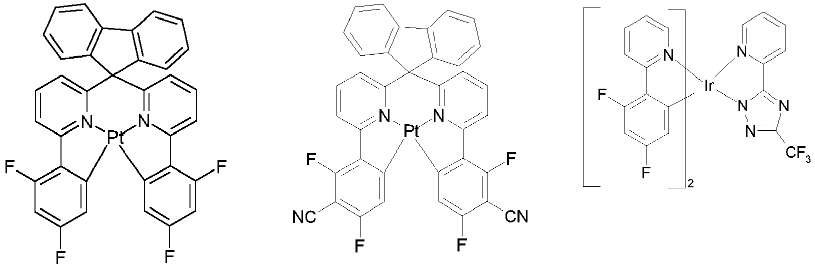

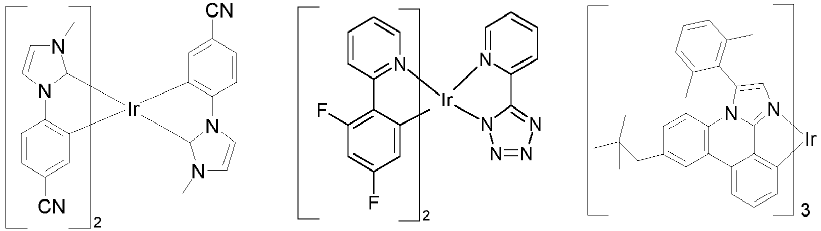

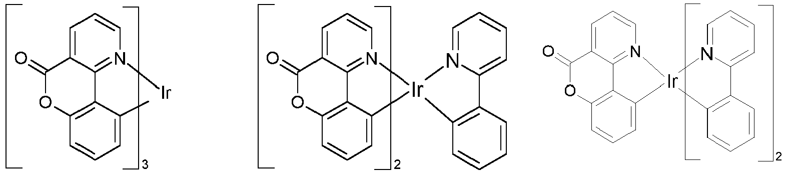

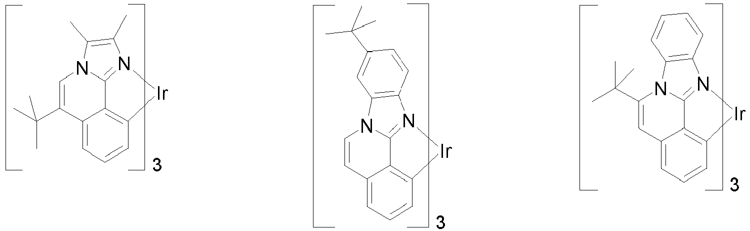

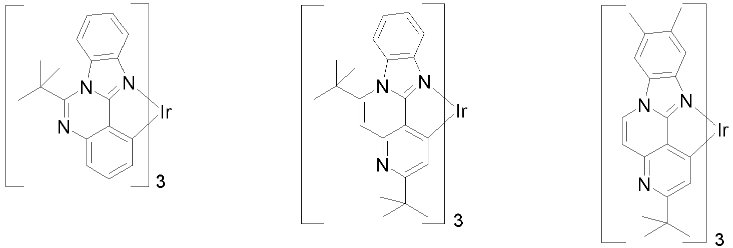

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Als Matrixmaterialien, bevorzugt für fluoreszierende Dotanden, kommen neben den Verbindungen der Formel (2) Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den Verbindungen der Formel (2) aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, DiazaphospholDerivate, z. B. gemäß WO 2010/054730 und Aluminiumkomplexe, z. B. BAIQ.

Außer Kathode, Anode und der Schicht enthaltend die Verbindung der Formel (2) kann die elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss.

Die Abfolge der Schichten der organischen Elektrolumineszenzvorrichtung ist bevorzugt die folgende:
Anode-Lochinjektionsschicht-Lochtransportschicht-emittierende Schicht-Elektronentransportschicht-Elektroneninjektionsschicht-Kathode.
Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere bevorzugt Materialien, die in einer Lochtransport-, Lochinjektions- oder Elektronenblockierschicht verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß der noch nicht offengelegten Anmeldung EP 11009127.9) und Dihydroacridin-Derivate (z. B. gemäß der noch nicht offen gelegten EP 11007067.9).

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, CS₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (2) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Gegenstand der Erfindung ist somit weiterhin ein Verfahren zur Herstellung der erfindungsggemäßen elektronischen Vorrichtung, dadurch gekennzeichnet, dass mindestens eine organische Schicht durch Gasphasenabscheidung oder aus Lösung aufgebracht wird.

Erfindungsgemäß können die elektronischen Vorrichtungen enthaltend eine oder mehrere Verbindungen gemäß Formel (2) in Displays, als Lichtquellen in Beleuchtungsanwendungen sowie als Lichtquellen in medizinischen und/oder kosmetischen Anwendungen (z.B. Lichttherapie) eingesetzt werden.

Die vorliegende Erfindung betrifft auch eine Formulierung enthaltend wenigstens eine Verbindung gemäß Formel (2) oder wenigstens eine der oben genannten Zusammensetzungen sowie wenigstens ein Lösungsmittel.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-lsopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Vorrichtungen enthaltend die Verbindungen nach Formel (2) können sehr vielseitig eingesetzt werden. So können, beispielsweise, elektrolumineszierende Vorrichtuungen enthaltend eine oder mehrere Verbindungen nach Formel (2) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach Formel (2) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Gegenstand der vorliegenden Erfindung ist daher eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (2) zur Verwendung in der Medizin zur Phototherapie.

Ein weiterer bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (2) zur Verwendung zur phototherapeutischen Behandlung von Hautkrankheiten.

Ein weiterer ganz bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (2) zur Verwendung zur phototherapeutischen Behandlung von Psoriasis, atopische Dermatitis, Entzündungserkrankungen, Vitiligo, Wundheilung und Hautkrebs.

Die vorliegende Erfindung betrifft weiterhin die Verwendung der elektronischen Vorrichtung, bevorzugt einer organischen Elektrolumineszenzvorrichtung, ganz bevorzugt einer OLED oder OLEC und ganz besonders bevorzugt einer OLED enthaltend wenigstens eine Verbindung gemäß Formel (2) in der Kosmetik, bevorzugt zur Behandlung von Akne, alternder Haut (Skin Ageing), und von Zellulithe.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Emissionsschicht und zeigen verbesserte Leistungsdaten gegenüber Verbindungen aus dem Stand der Technik.
2. Die erfindungsgemäßen Verbindungen weisen eine relativ niedrige Sublimationstemperatur, eine hohe Temperaturstabilität und lassen sich daher unzersetzt und rückstandsfrei sublimieren. Weiterhin weisen sie eine hohe Oxidationsstabilität und eine hohe Glasübergangstemperatur auf, was sowohl für die Prozessierbarkeit, beispielweise aus Lösung oder aus der Gasphase, als auch für die Verbindung in elektronischen Vorrichtungen vorteilhaft ist.
3. Die Verwendung der erfindungsgemäßen Verbindungen in elektronischen Vorrichtungen, insbesondere eingesetzt als Matrixmaterial, aber auch als Elektronentransport- oder Elektroneninjektionsmaterial, führen zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Nur Beispiele die in den Ansprüchen fallen sind Teil der Erfindung.

### Beispiele

Die nachfolgenden Synthesen werden, sofern nicht anders angegeben, unter Schutzgasatmosphäre in getrockneten Lösungsmitteln durchgeführt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

### Beispiel 1

### Synthese von 3-Dibenzofuran-4-yl-6,9-diphenyl-9H-carbazol

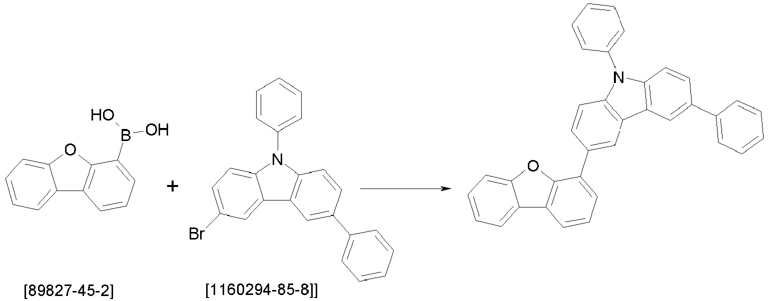

28,9 g (136 mmol) Dibenzofuran-4-boronsäure, 40 g (124,1 mmol) 3-Bromo-9-phenyl-9H-carbazol, 78,9 mL (158 mmol) Na₂CO₃ (2 M-Lösung) werden in 120 mL Toulol, 120 mL Ethanol und 100 mL Wasser suspendiert. Zu dieser Suspension werden 2,6 g (2.2 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 49,7 g (121 mmol), entsprechend 97% der Theorie.

Analog können folgende Verbindungen erhalten werden:

| Edukt 1 | Edukt 2 | Produkt | Ausbeute |
|---|---|---|---|
| 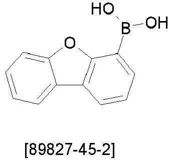 | 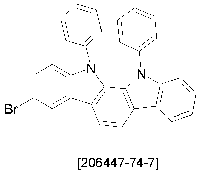 | 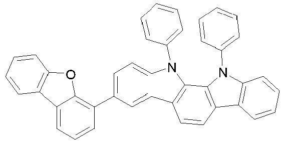 | 69% |
| 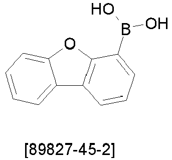 | 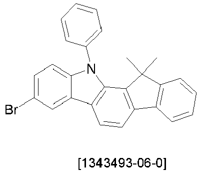 | 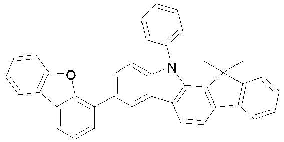 | 92% |
| 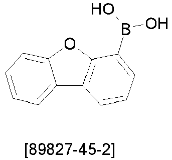 | 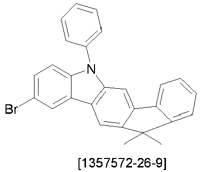 | 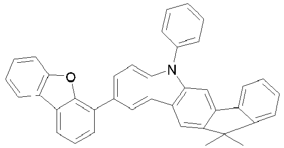 | 68% |
| 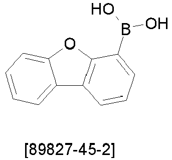 | 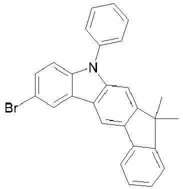 | 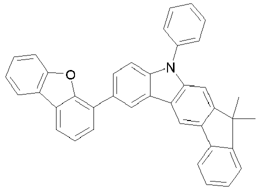 | 77% |
| 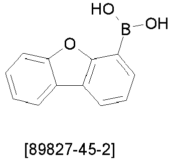 | 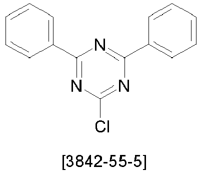 | 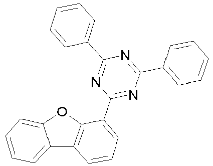 | 63% |
| 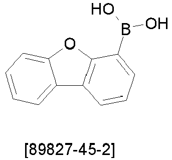 | 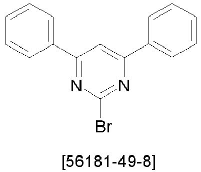 | 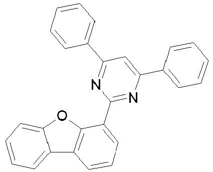 | 75% |
| 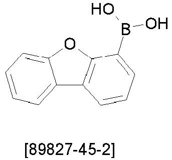 | 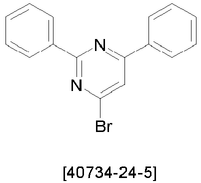 | 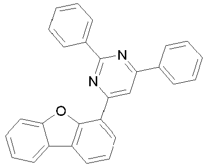 | 71% |
| 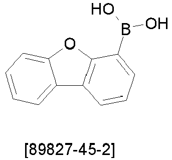 | 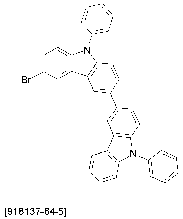 | 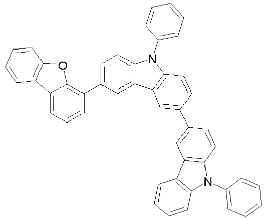 | 75% |
| 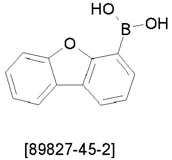 | 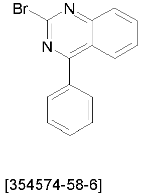 | 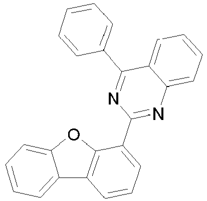 | 83% |

### Beispiel 2

### Synthese von 1-Dibenzofuran-4-yl-2-phenyl-1H-benzoimidazol

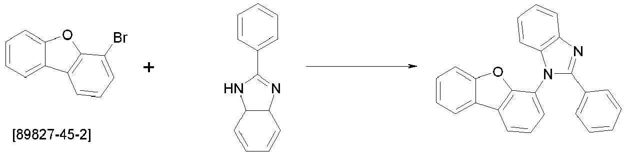

Eine gut gerührte Suspension von 42 g (234 mmol) 2-Phenyl-1H-Benzimidazol, 57,7 g (234 mmol) 4-Brom-Dibenzofuran und 416,4 g (1961 mmol) Kaliumphosphat in 1170 mL Dioxan wird mit 8,0 g (42,2 mmol) Kupfer(I)iodid und 11,7 mL (97,5 mmol) *trans*-Cyclohexandiamin versetzt und anschließend 16 h unter Rückfluss erhitzt. Nach dem Erkalten wird der ausgefallene Feststoff abgesaugt, dreimal mit 50 mL Toluol, dreimal mit 50 mL Ethanol:Wasser (1:1, v:v) und dreimal mit 100 mL Ethanol gewaschen. Ausbeute: 52 g (144 mmol), 85% der Theorie.

Analog können folgende Verbindungen erhalten werden:

| **Edukt 1** | **Edukt 2** | **Produkt** | **Ausbeute** |
|---|---|---|---|
| 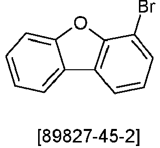 | 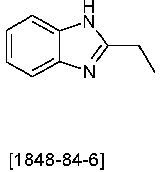 | 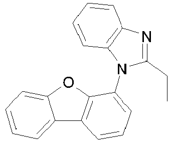 | 88% |
| 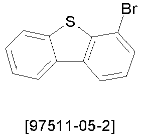 | 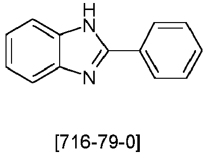 | 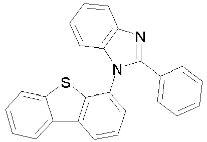 | 82% |

### Beispiel 3

### Synthese von 3,9-Diphenyl-6-(6-trimethylsilanyl-dibenzofuran-4-yl)-9H-carbazol

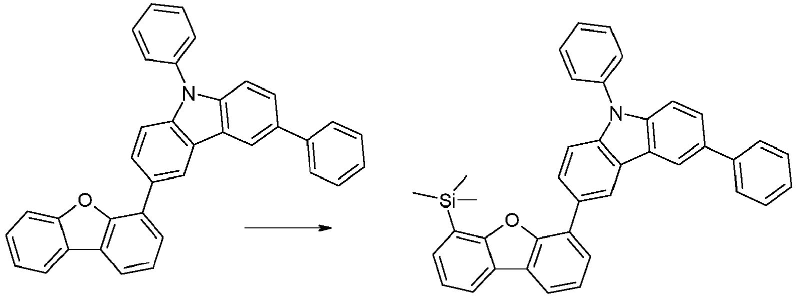

Eine auf 20°C gekühlte Lösung von 58,7 g (121 mmol) 3-Dibenzofuran-4-yl-6,9-diphenyl-9H-carbazol und 28 g (242 mmol)TMEDA in 1000 mL THF wird tropfenweise mit 127 ml (225,4 mmol) n-Buthyllithium (2.5 M in Hexan) versetzt. Die Reaktionsmischung wird 3 h bei Raumtemperatur gerührt, dann kühlt man auf 0°C ab und tropft innerhalb 30 min. 26 g (242 mmol) Chlortrimetylsilan hinzu und rührt 8 h bei Raumtemperatur. Anschließend wird das Lösungsmittel im Vakuum entfernt und der Rückstand chromatographisch über Kieselgel mit Chloroform als Laufmittel gereinigt. Ausbeute: 41 g (72 mmol), 61% der Theorie.

Analog können die folgenden Verbindungen erhalten werden:

| **Edukt 1** | **Produkt** | **Ausbeute** |
|---|---|---|
| 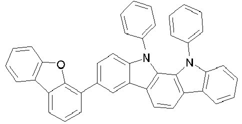 | 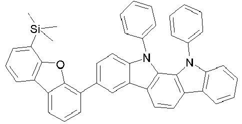 | 63% |
| 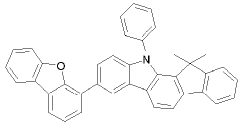 | 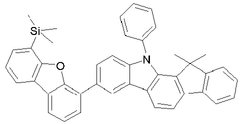 | 59% |
| 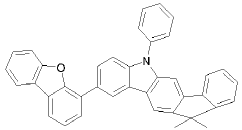 | 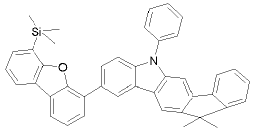 | 57% |
| 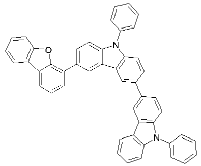 | 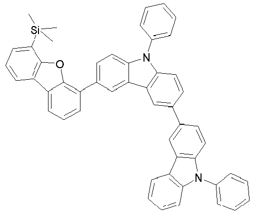 | 52% |
| 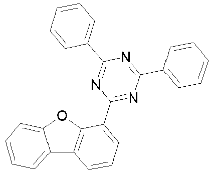 | 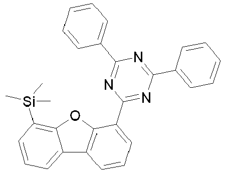 | 60% |
| 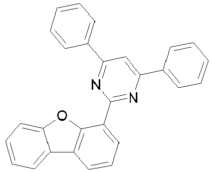 | 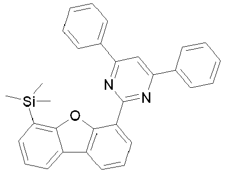 | 54% |
| 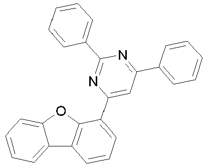 | 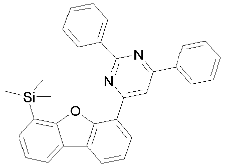 | 59% |
| 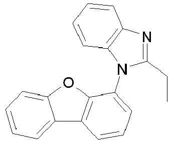 | 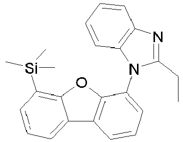 | 76% |
| 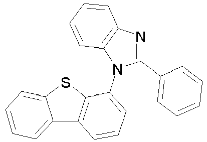 | 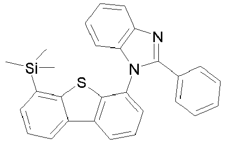 | 78% |
| 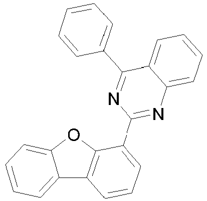 | 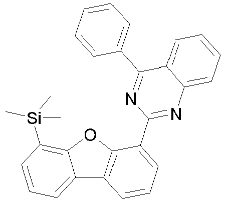 | 69% |

### Beispiel 4

### Synthese von 6-(3,9-Diphenyl-9H-carbazol-3-yl)-4-dibenzofuranyl]-boronsäure

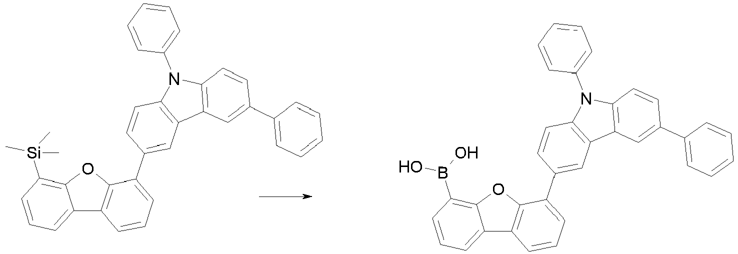

Unter Schutzgas wird eine Lösung von 40 g (72 mmol) 3,9-Diphenyl-6-(6-trimethylsilanyl-dibenzofuran-4-yl)-9H-carbazol in 500 mL Dichloromethan tropfenweise mit 21 g (86 mmol) Bromtribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 32 g (61 mmol), entsprechend 85% der Theorie.

Analog können die folgenden Verbindungen erhalten werden.

| **Edukt 1** | **Produkt** | **Ausbeute** |
|---|---|---|
| 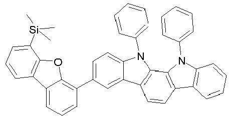 | 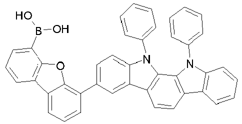 | 80% |
| 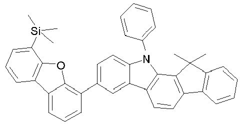 | 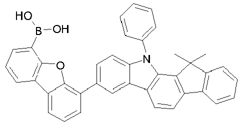 | 83% |
| 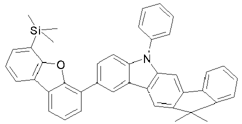 | 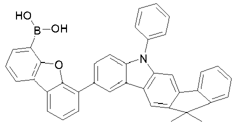 | 81% |
| 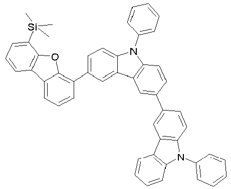 | 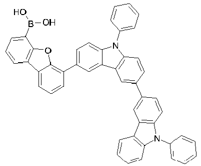 | 79% |
| 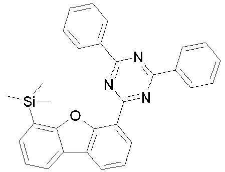 | 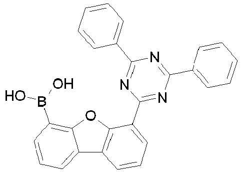 | 69% |
| 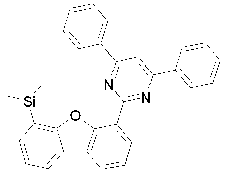 | 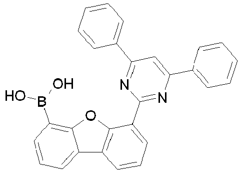 | 78% |
| 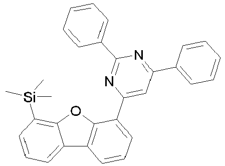 | 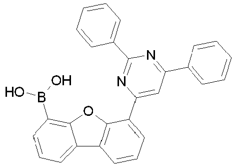 | 77% |
| 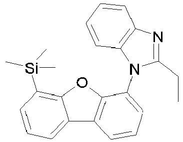 | 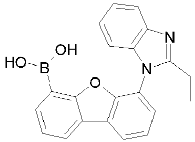 | 82% |
| 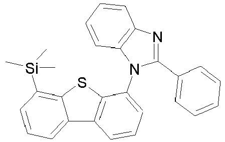 | 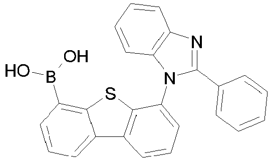 | 85% |
| 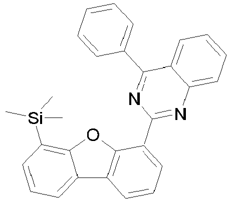 | 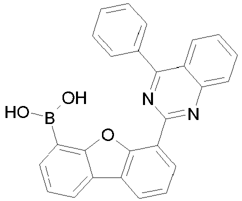 | 80% |

### Beispiel 5

### Synthese von 6-(3,9-Diphenyl-9H-carbazol-3-yl)-4-dibenzofuranyl]-boronsäure

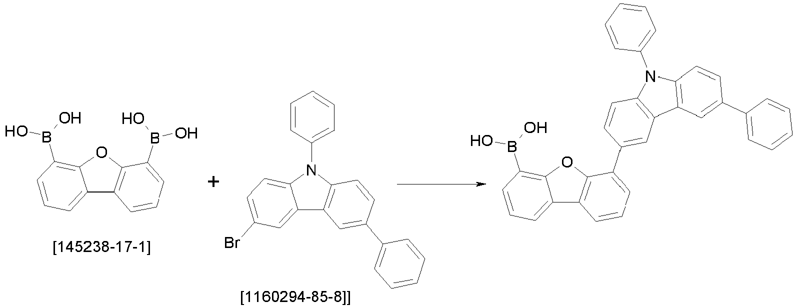

9 g (32 mmol) 4,6-dibenzofurandiylbisboronsäure, 12,5 g (31,6 mmol) 3-Bromo-9-phenyl-9H-carbazol, 31 mL (63 mmol) Na₂CO₃ (2 M-Lösung) werden in 120 mL Toulol, 120 mL Ethanol suspendiert. Zu dieser Suspension werden 0,73 g (0,63 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 11,2 g (21 mmol), entsprechend 67% der Theorie.

Analog können die folgenden Verbindungen erhalten werden:

| **Edukt 1** | **Edukt 2** | **Produkt** | **Ausbeute** |
|---|---|---|---|
| 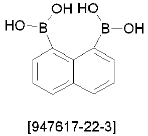 | 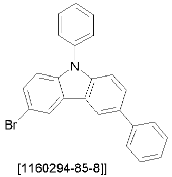 | 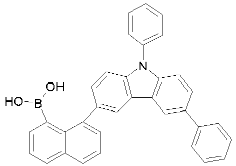 | 85% |
| 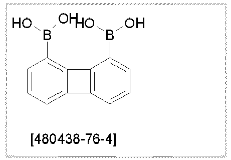 | 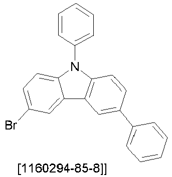 | 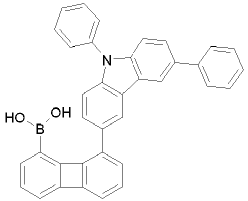 | 69% |
| 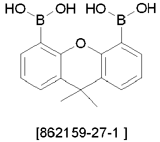 | 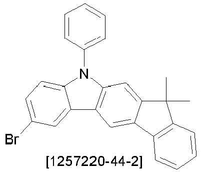 | 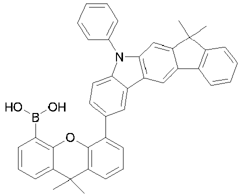 | 74% |
| 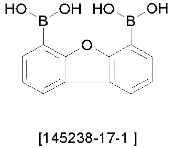 | 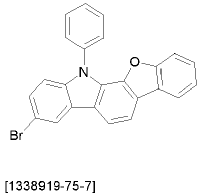 | 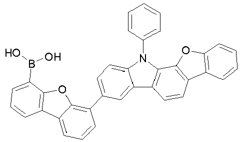 | 76% |

### Beispiel 6

### Synthese von 10-(6-Bromo-dibenzofuran-4-yl)-7-phenyl-7H-12-thia-7-aza-indeno[1,2-a]fluorin

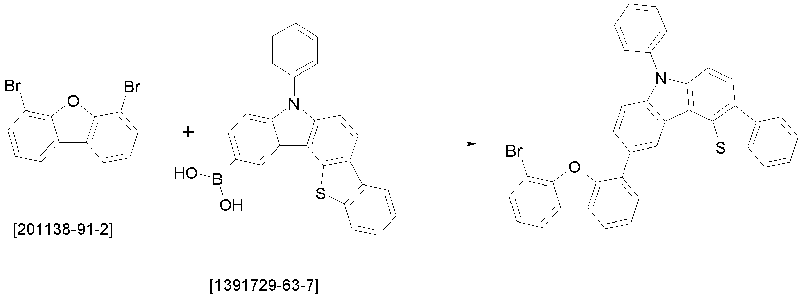

12,5 g (32 mmol) 5--Phenyl-5H[1]benzothieno[3,2-c]carbazol-3-yl) boronsäure, 8,9 g (31,6 mmol) 4,6-Dibromdibenzofuran, 31 mL (63 mmol) Na₂CO₃ (2 M-Lösung) werden in 120 mL Toulol und 120 mL Ethanol suspendiert. Zu dieser Suspension werden 0,73 g (0,63 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 13,3 g (22 mmol), entsprechend 73% der Theorie.

Analog können die folgenden Verbindungen erhalten werden.

| **Edukt 1** | **Edukt 2** | **Produkt** | **Ausbeute** |
|---|---|---|---|
| 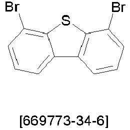 | 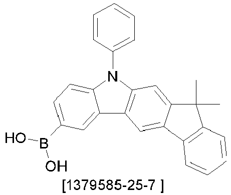 | 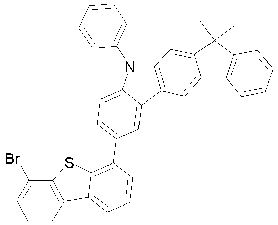 | 51% |
| 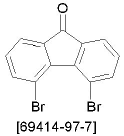 | 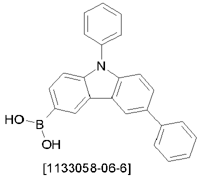 | 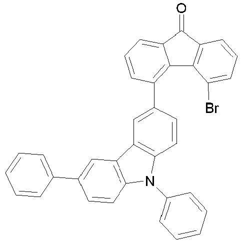 | 65% |
| 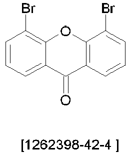 | 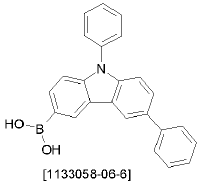 | 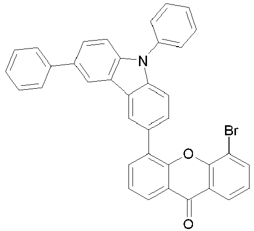 | 69% |
| 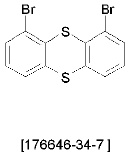 | 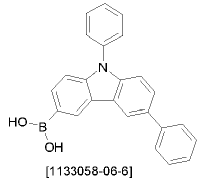 | 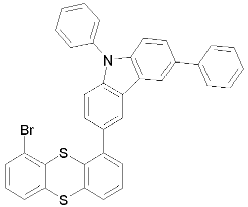 | 67% |
| 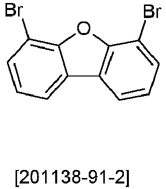 | 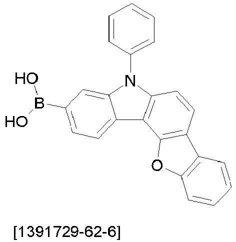 | 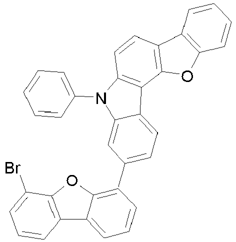 | 62% |
| 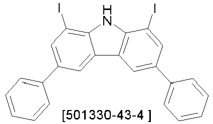 | 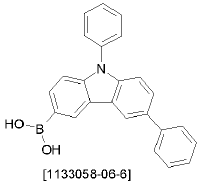 | 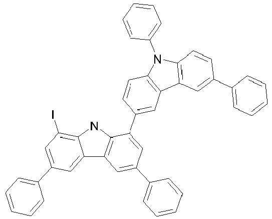 | 62% |
| 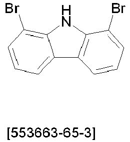 | 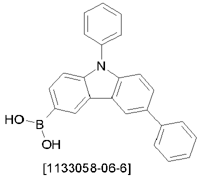 | 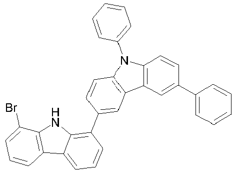 | 61% |
| 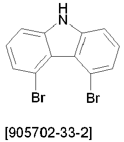 | 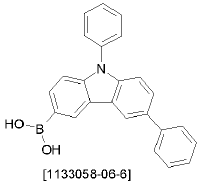 | 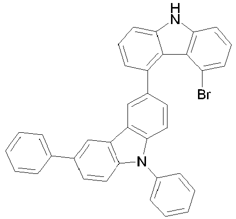 | 64% |
| 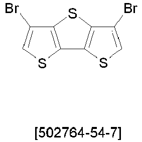 | 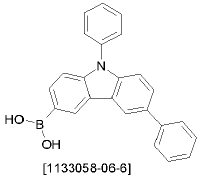 | 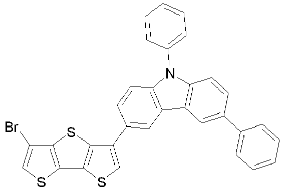 | 66% |
| 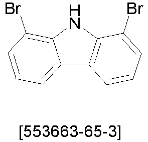 | 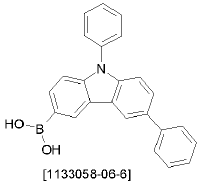 | 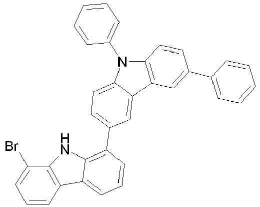 | 56% |
| 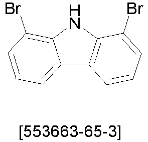 | 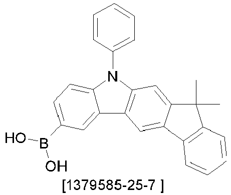 | 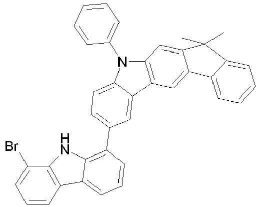 | 72% |

Analog können auch die folgenden Verbindungen durch eine zweite Addition mit den entsprechenden Borosäuren erhalten werden: Der Rückstand wird aus Toluol umkristallisiert und abschließend im Hochvakuum (p = 5 x 10⁻⁵ mbar) sublimiert

| **Edukt 1** | **Edukt 2** | **Produkt** | **Ausbeute** |
|---|---|---|---|
| 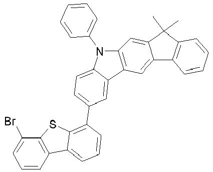 | 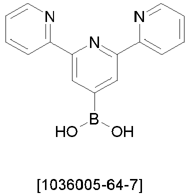 | 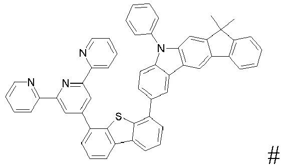 | 81% |
| 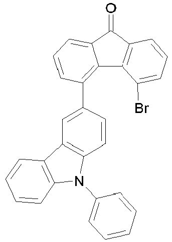 | 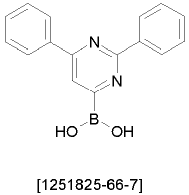 | 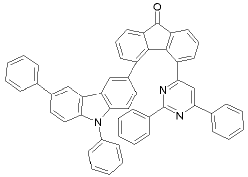 | 79% |
| 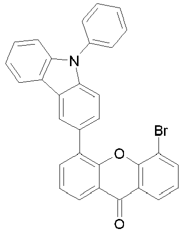 | 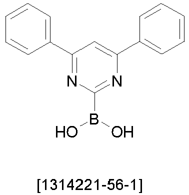 | 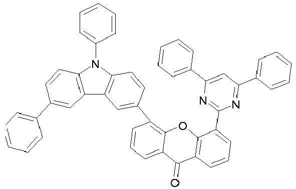 | 66% |
| 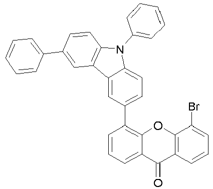 | 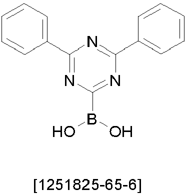 | 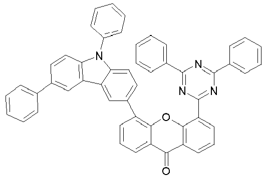 | 78% |
| 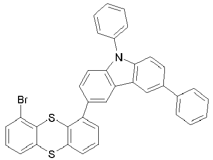 | 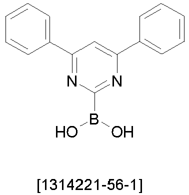 | 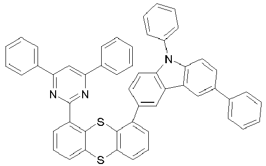 | 77% |
| 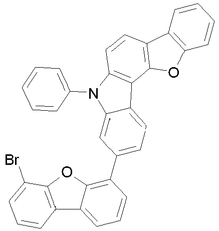 | 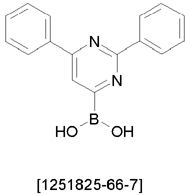 | 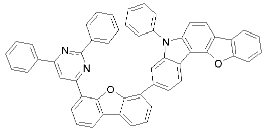 | 79% |
| 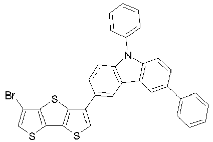 | 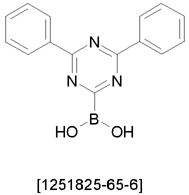 | 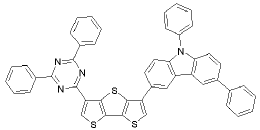 | 83% |
| 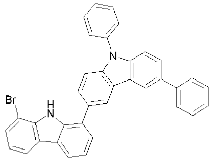 | 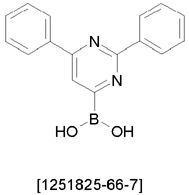 | 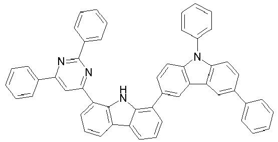 | 83% |
| 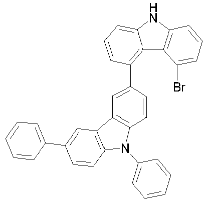 | 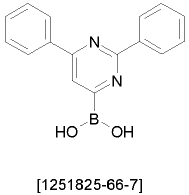 | 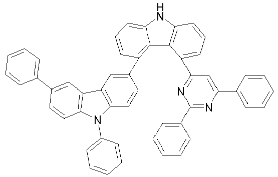 | 74% |
| 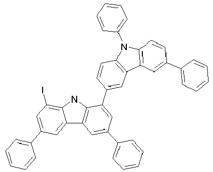 | 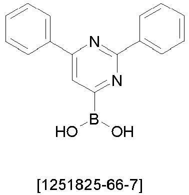 | 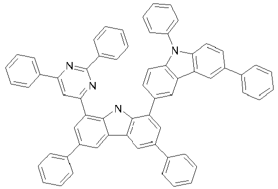 | 78% |
| 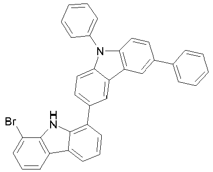 | 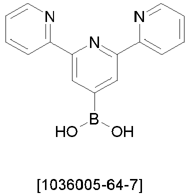 | 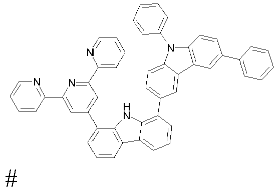 | 79% |
| 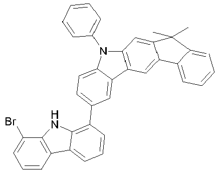 | 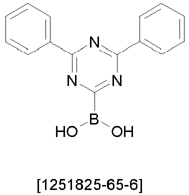 | 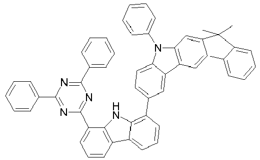 | 77% |
| 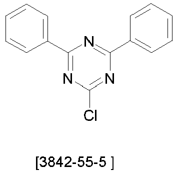 | 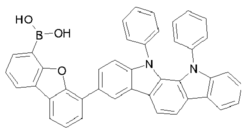 | 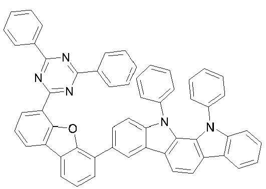 | 65% |
| 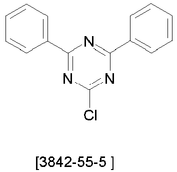 | 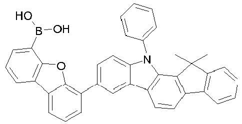 | 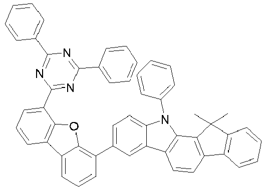 | 66% |
| 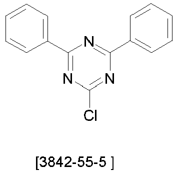 | 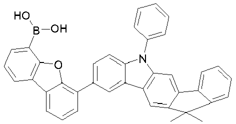 | 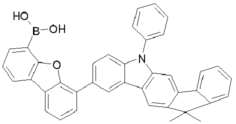 | 69% |
| 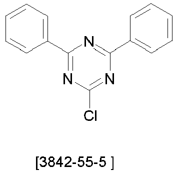 | 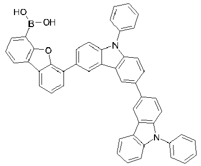 | 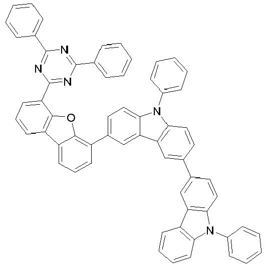 | 71% |
| 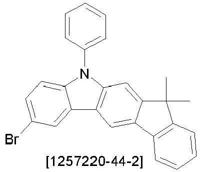 | 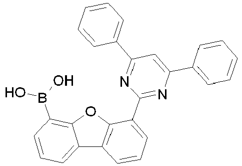 | 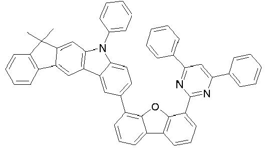 | 79% |
| 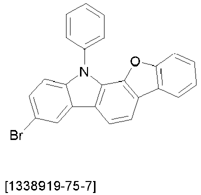 | 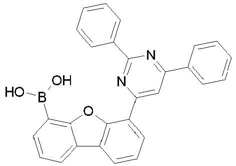 | 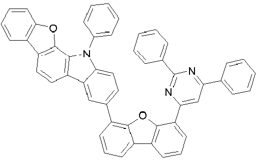 | 76% |
| 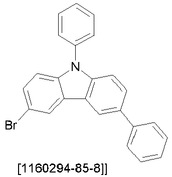 | 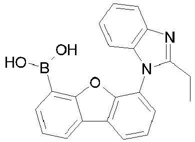 | 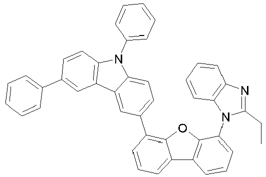 | 83% |
| 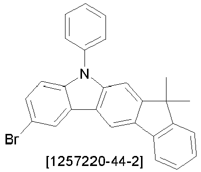 | 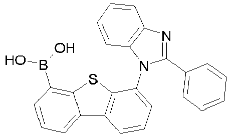 | 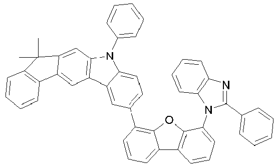 | 80% |
| 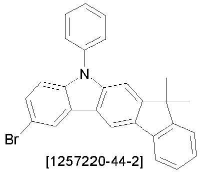 | 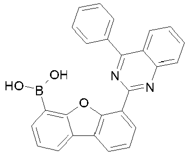 | 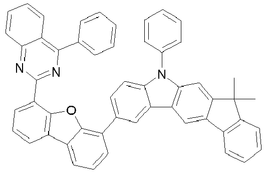 | 81% |

# nicht erfindungsgemäß

### Beispiel 7

### Synthese von 3-[6-(4,6-Diphenyl-[1,3,5]triazin-2-yl)-dibenzofuran-4-yl]-6,9-diphenyl-9H-carbazol

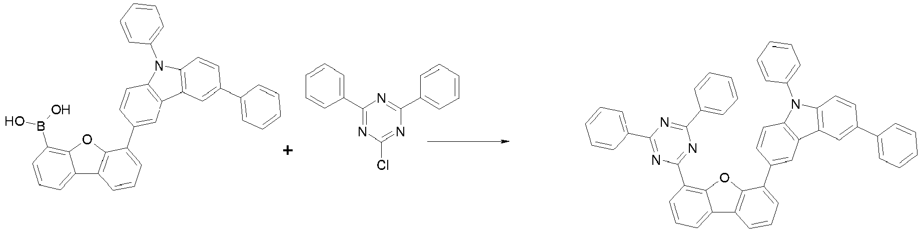

37 g (70 mmol) 6-(3,9-Diphenyl-9H-carbazol-3-yl)-4-dibenzofuranyl]-boronsäure, 18,8 g (70 mmol) 2-Chlor-4,6-diphenyl-[1,3,5]triazin, 78,9 mL (158 mmol) Na₂CO₃ (2 M-Lösung) werden in 120 mL Ethanol und 100 mL Wasser suspendiert. Zu dieser Suspension werden 1,3 g (1,1 mmol) Pd(PPh₃)₄ gegeben, und die Reaktionsmischung wird 16 h unter Rückfluss erhitzt. Nach Erkalten wird die Mischung mit Dichloromethan versetzt, die organische Phase abgetrennt, über Kieselgel filtriert. Die Ausbeute beträgt 41 g (58 mmol), entsprechend 83% der Theorie. Der Rückstand wird aus Toluol umkristallisiert und abschließend im Hochvakuum (p = 5 x 10⁻⁵ mbar) sublimiert. Die Reinheit beträgt 99.9%.

Analog können die folgenden Verbindungen erhalten werden.

| **Edukt 1** | **Edukt 2** | **Produkt** | **Ausbeute** |
|---|---|---|---|
| 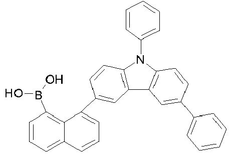 | 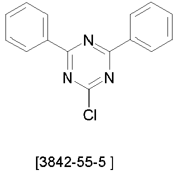 | 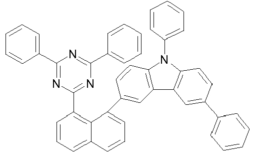 | 59% |
| 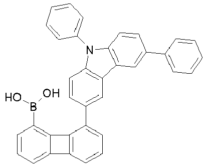 | 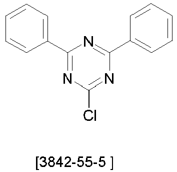 | 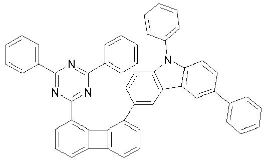 | 67% |
| 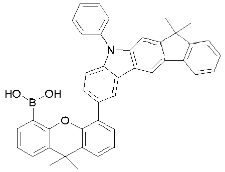 | 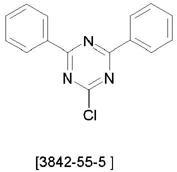 | 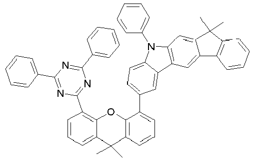 | 68% |

### Beispiel 8

### Synthese von 8-(4,6-Diphenyl-[1,3,5]triazin-2-yl)-9,6',9'-triphenyl-9H,9'H-[1,2']bicarbazolyl

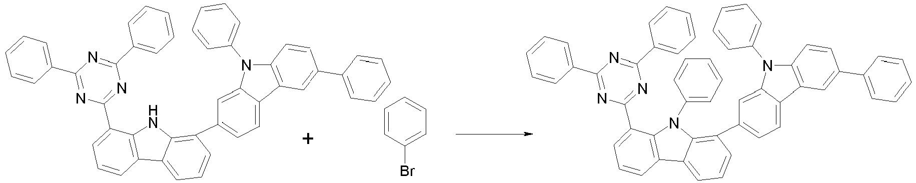

50,4 g (70,58 mmol) 8-(4,6-Diphenyl-[1,3,5]triazin-2-yl)-6',9'-diphenyl-9H,9'H-[1,2']bicarbazolyl und 16,4 g (105,87 mmol) Brom-benzol werden in Toluol gelöst und mittels Schutzgaseinleitung entgast. Anschließend wird mit 7 mL (7 mmol, 1 M Lösung in Toluol) Tri-tert-butylphosphin, 633,8 mg (2,82 mmol) Pd(OAc)₂ und 10,2 g (105,87 mmol) NaOtBu versetzt. Die Feststoffe werden zuvor entgast, die Reaktionsmischung wird nachentgast und anschließend unter Rückfluss für 3 h gerührt. Die warme Reaktionslösung wird über Alox B (Aktivitätsstufe 1) filtriert, mit Wasser gewaschen, getrocknet und eingeengt. Die Ausbeute beträgt 44 g (55 mmol), entsprechend 79% der Theorie. Der Rückstand wird aus Toluol umkristallisiert und abschließend im Hochvakuum (p = 5 x 10⁻⁵ mbar) sublimiert. Die Reinheit beträgt 99.9%.

Analog können die folgenden Verbindungen erhalten werden.

| **Edukt 1** | **Edukt 2** | **Produkt** | **Ausbeute** |
|---|---|---|---|
| 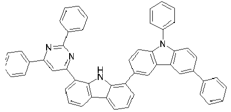 | 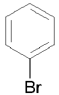 | 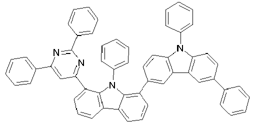 | 79% |
| 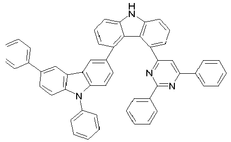 | 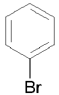 | 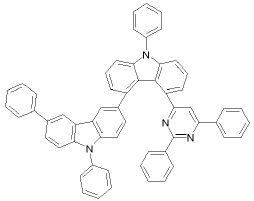 | 80% |
| 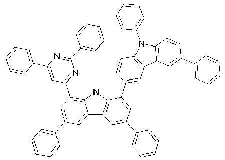 | 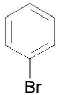 | 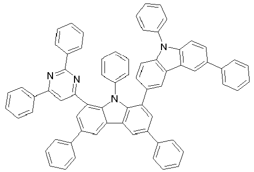 | 80% |
| 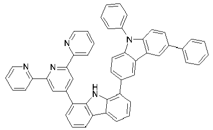 | 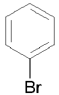 | 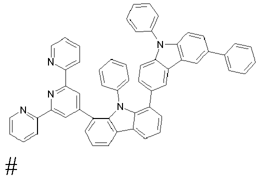 | 70% |
| 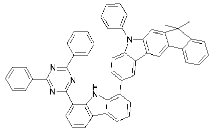 | 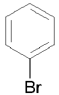 | 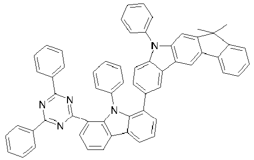 | 69% |

### Beispiel 10 (Vergleich)

### Synthese von 3-{4-[6-(4,6-Diphenyl-[1,3,5]triazin-2-yl)-dibenzofuran-4-yl]-phenyl}-9-phenyl-9H-carbazol

### a) Synthese von 2-(6-Bromo-dibenzofuran-4-yl)-4,6-diphenyl-[1,3,5]triazin

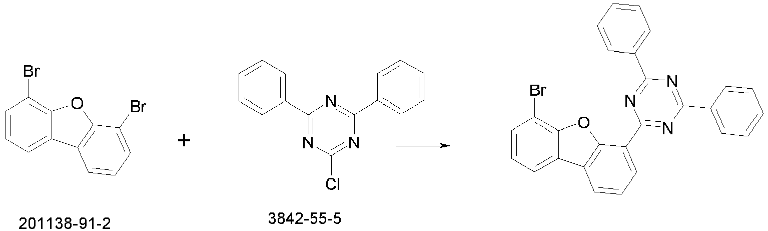

80 g (245 mmol) 4,6-Dibromdibenzofuran werden in einem ausgeheizten Kolben in 500 mL getrocknetem THF gelöst. Die Reaktionsmischung wird auf -78°C gekühlt. Bei dieser Temperatur werden 57 mL einer 1,9 M-Lösung n-Phenyllithium in Dibutylether (115 mmol) langsam zugetropft. Der Ansatz wird 1 Stunde bei -73°C nachgerührt. Anschließend werden 65 g 2-Chlor-4,6-diphenyl-1,3,5-triazin (245 mmol) in 150 ml THF gelöst und bei -70°C zugetropft. Nach beendeter Zugabe wird die Reaktionsmischung langsam auf Raumtemperatur erwärmt, über Nacht bei Raumtemperatur gerührt, mit Wasser gequencht und anschließend am Rotationsverdampfer eingeengt. Dabei fällt ein weißer Feststoff aus. Der Ansatz wird nun auf Raumtemperatur abgekühlt und der ausgefallene Feststoff wird abgesaugt und mit Methanol nachgewaschen. Die Ausbeute beträgt 40 g (84 mmol), entsprechend 34% der Theorie.

### b) Synthese von 3-{4-[6-(4,6-Diphenyl-[1,3,5]triazin-2-yl)-dibenzofuran-4-yl]-phenyl}-9-phenyl-9H-carbazol

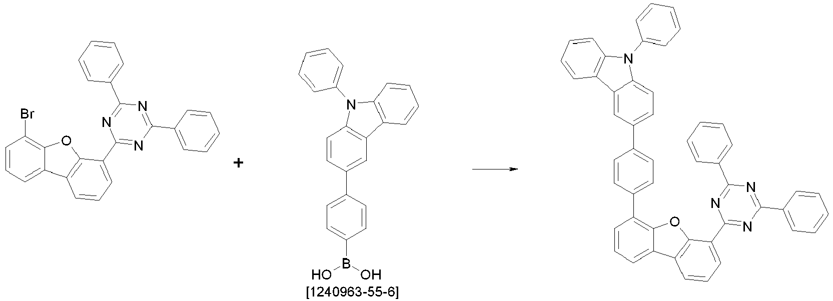

33,4 g (70 mmol) 2-(6-Bromo-dibenzofuran-4-yl)-4,6-diphenyl-[1,3,5]triazin, 25,4 g (70 mmol) 4-(9-phenyl-9H-carbazol-3-yl)phenyl-boromsäure , 78,9 mL (158 mmol) Na₂CO₃(2 M-Lösung) werden in 120 mL Ethanol und 100 mL Wasser suspendiert. Zu dieser Suspension werden 1,3 g (1,1 mmol) Pd(PPh₃)₄ gegeben, und die Reaktionsmischung wird 16 h unter Rückfluss erhitzt. Nach dem Erkalten wird die Mischung mit Dichloromethan versetzt, die organische Phase abgetrennt, über Kieselgel filtriert und aus Toluol umkristallisiert. Die Ausbeute beträgt 40 g (56 mmol), entsprechend 80% der Theorie.

Analog kann die folgende Verbindung hergestellt werden.

| **Edukt 1** | **Edukt 2** | **Produkt** | **Ausbeute** |
|---|---|---|---|
| 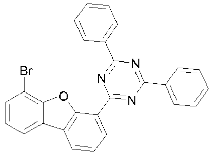 | 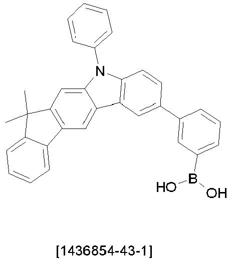 | 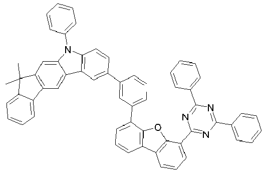 | 86% |

### Beispiel 11(Vergleich)

### Synthese von 3-{7-[4-(4,6-Diphenyl-[1,3,5]triazin-2-yl)-phenyl]-dibenzofuran-4-yl}-9-phenyl-9H-carbazol

### a) Herstellung von von 2-(4-Dibenzofuran-3-yl-phenyl)-4,6-diphenyl-[1,3,5]triazin

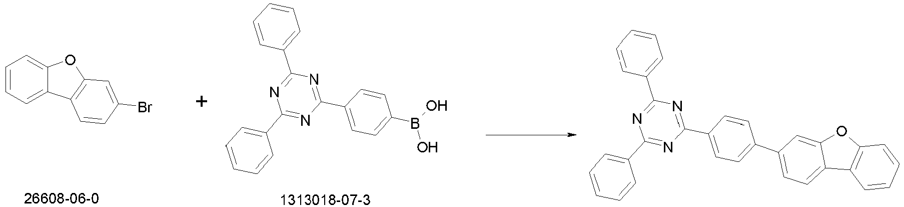

24 g (70 mmol) 4-(4-6-diphenyl-1,3,5-triazin-2yl)phenyl)- boronsäure , 17,3 g (70 mmol) 3-Bromo-Dibenzofuran, 78,9 ml (158 mmol) Na₂CO₃ (2 M-Lösung) werden in 120 mL Ethanol und 100 mL Wasser suspendiert. Zu dieser Suspension werden 1,3 g (1,1 mmol) Pd(PPh₃)₄ gegeben, und die Reaktionsmischung wird 16 h unter Rückfluss erhitzt. Nach dem Erkalten wird die Mischung mit Dichloromethan versetzt, die organische Phase abgetrennt, über Kieselgel filtriert und aus Toluol umkristallisiert. Die Ausbeute beträgt 28 g (58 mmol), entsprechend 86% der Theorie.

Analog kann die folgende Verbindung hergestellt werden.

| **Edukt 1** | **Edukt 2** | **Produkt** | **Ausbeute** |
|---|---|---|---|
| 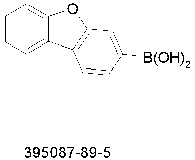 | 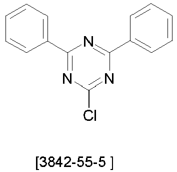 | 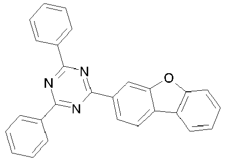 | 87% |

### b) Synthese von 2,4-Diphenyl-6-[4-(6-trimethylsilanyl-dibenzofuran-3-yl)-phenyl]-[1,3,5] triazin

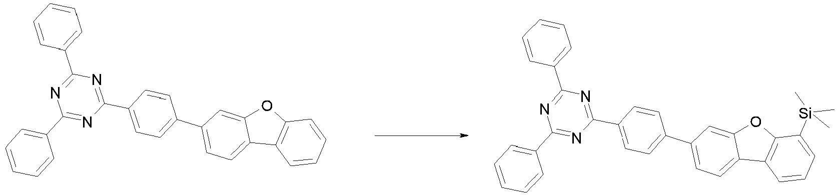

Eine auf 20°C gekühlte Lösung von 57,4 g (121 mmol) 2-(4-Dibenzofuran-3-yl-phenyl)-4,6-diphenyl-[1,3,5]triazin und 28 g (242 mmol) TMEDA in 1000 mL THF wird tropfenweise mit 127 mL (225,4 mmol) n-Buthyllithium (2.5 M in Hexan) versetzt. Die Reaktionsmischung wird 3 h bei Raumtemperatur gerührt, dann auf 0°C abgekühlt und innerhalb von 30 min. 26 g (242 mmol) Chlortrimetylsilan hinzugetropft. Es wird 8 h bei Raumtemperatur gerührt. Anschließend wird das Lösungsmittel im Vakuum entfernt und der Rückstand Chromateographisch über Kieselgel mit Chloroform als Laumittel gereinigt. Ausbeute: 41 g (74 mmol), 63% der Theorie.

Analog kann die folgende Verbindung hergestellt werden.

| **Edukt 1** | **Produkt** | **Ausbeute** |
|---|---|---|
| 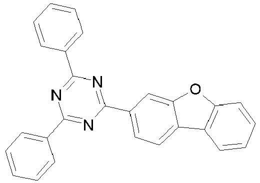 | 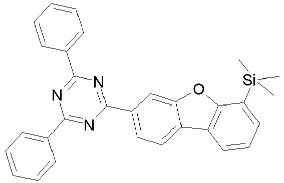 | 87% |

### c) Synthese von 3-[4-(4,6-Diphenyl-[1,3,5]triazin-2-yl)-phenyl]-Dibenzofuran- 6- boronsäure

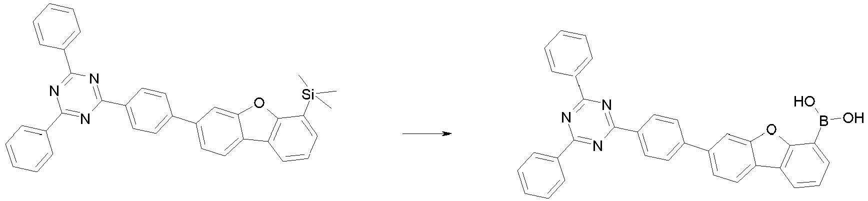

Unter Schutzgas wird eine Lösung von 39 g von 2,4-Diphenyl-6-[4-(6-trimethylsilanyl-dibenzofuran-3-yl)-phenyl]-[1,3,5] triazin in 500 mL Dichloromethan tropfenweise mit 21 g (86 mmol) Bromtribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 32 g (62 mmol), entsprechend 87% der Theorie.

Analog kann die folgende Verbindung hergestellt werden.

| **Edukt 1** | **Produkt** | **Ausbeute** |
|---|---|---|
| 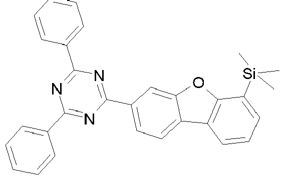 | 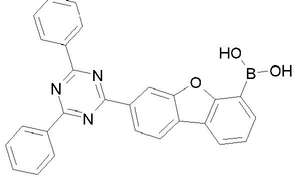 | 90% |

### d) Synthese von 3-{7-[4-(4,6-Diphenyl-[1,3,5]triazin-2-yl)-phenyl]-dibenzofuran-4-yl}-6,9-diphenyl-9H-carbazole

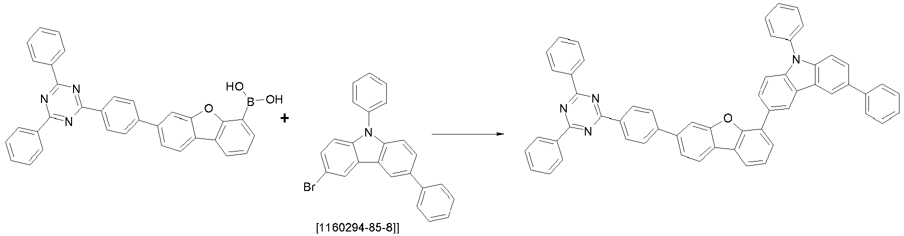

36 g (70 mmol) 3-[4-(4,6-Diphenyl-[1,3,5]triazin-2-yl)-phenyl -dibenzofuran-6- boronsäure 27 g (70 mmol) 3-Bromo-6,9-diphenyl-9H-carbazol und 78,9 mL (158 mmol) Na₂CO₃ (2 M-Lösung) werden in 120 mL Ethanol und 100 mL Wasser suspendiert. Zu dieser Suspension werden 1,3 g (1,1 mmol) Pd(PPh₃)₄ gegeben, und die Reaktionsmischung wird 16 h unter Rückfluss erhitzt. Nach Erkalten wird Die Mischung mit Dichloromethan versetzt, die organische Phase abgetrennt, über Kieselgel filtriert und aus Toluol umkristallisier. Der Rückstand wird aus Toluol umkristallisiert und abschließend im Hochvakuum (p = 5 x 10⁻⁵mbar) sublimiert. Die Ausbeute beträgt 36 g (53 mmol), entsprechend 80% der Theorie.

Analog kann die folgende Verbindung hergestellt werden.

| Edukt 1 | Edukt 2 | Produkt | Ausbeute |
|---|---|---|---|
| 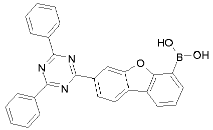 | 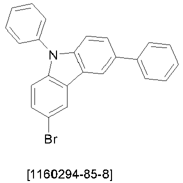 | 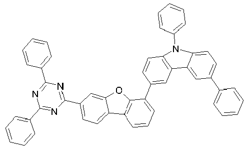 | 86% |

### Beispiel 12

### Synthese von 3-Dibenzofuran-4-yl-9-phenyl-9H-carbazol

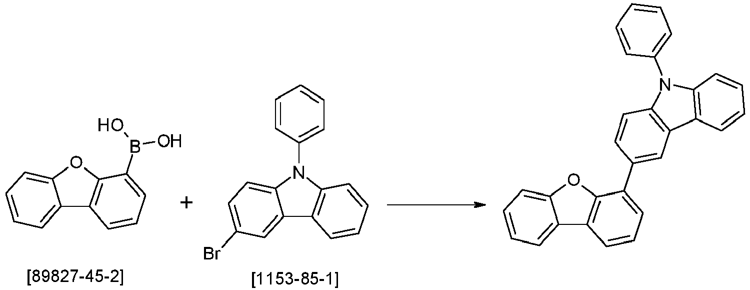

28,9 g (136 mmol) Dibenzofuran-4-boronsäure, 40 g (124,1 mmol) 3-Bromo-9-phenyl-9H-carbazole, 78,9 ml (158 mmol) Na₂CO₃ (2M-Lösung) werden in 120 mL Toulol, 120 mL Ethanol und 100 mL Wasser suspendiert. Zu dieser Suspension werden 2,6 g (2.2 mmol) Pd(PPh₃)₄ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 49,7 g (121 mmol), entsprechend 97 % der Theorie.

Analog können folgende Verbindungen erhalten werden:

| Edukt 1 | Edukt 2 | Produkt | Ausbeute |
|---|---|---|---|
| 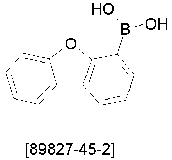 | 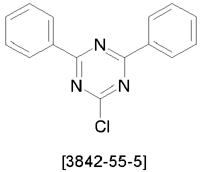 | 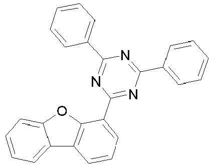 | 69% |

### Beispiel 13

### Synthese von 9-Phenyl-3-(6-trimethylsilanyl-dibenzofuran-4-yl)-9H-carbazol

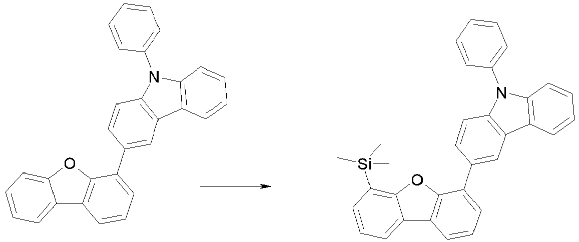

Eine auf 20°C gekühlte Lösung von 49 g (121 mmol) 3-Dibenzofuran-4-yl-9-phenyl-9H-carbazol und 28 g (242 mmol)TMEDA in 1000 mL THF wird tropfenweise mit 127 mL (225,4 mmol) n-Buthyllithium (2.5 M in Hexan) versetzt. Die Reaktionsmischung wird 3 h bei Raumtemperatur gerührt, dann kühlt man auf 0°C und tropft innerhalb 30 min. 26 g (242 mmol) Chlortrimetylsilan hinzu und rührt 8 h bei Raumtemperatur. Anschließend wird das Lösungsmittel im Vakuum entfernt und der Rückstand Chromateographisch über Kieselgel mit Chloroform als Laumittel gereinigt. Ausbeute: 34 g (72 mmol), 60% der Theorie.

Analog können folgende Verbindungen erhalten werden.

| Edukt 1 | Produkt | Ausbeute |
|---|---|---|
| 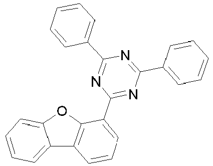 | 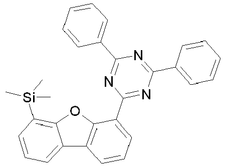 | 65% |
| 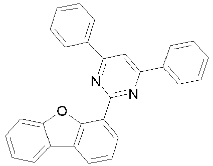 | 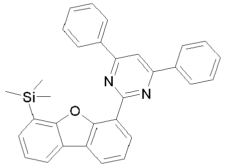 | 64% |

### Beispiel 14

### Synthese von B-[6-(Phenyl-9H-carbazol-3-yl)-4-dibenzofuranyl]-boronsäure

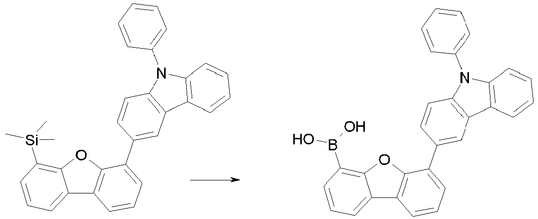

Unter Schutzgas wird eine Lösung von 34 g (72 mmol) B-[6-(Phenyl-9H-carbazol-3-yl)-4-dibenzofuranyl]-boronsäure in 500 mL Dichloromethan tropfenweise mit 21 g (86 mmol) Bromtribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 28 g (62 mmol), entsprechend 86% der Theorie.

Analog können folgende Verbindungen erhalten werden.

| Edukt 1 | Produkt | Ausbeute |
|---|---|---|
| 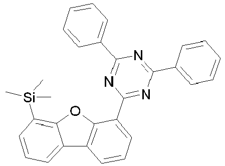 | 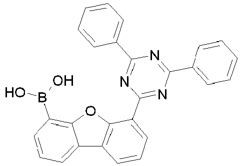 | 69% |
| 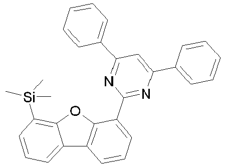 | 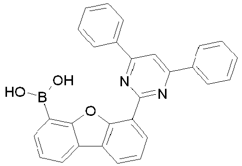 | 78% |

### Beispiel 15

### Synthese von 10-(6-Bromo-dibenzofuran-4-yl)-7-phenyl-7H-12-thia-7-aza-indeno[1,2-a]fluoren

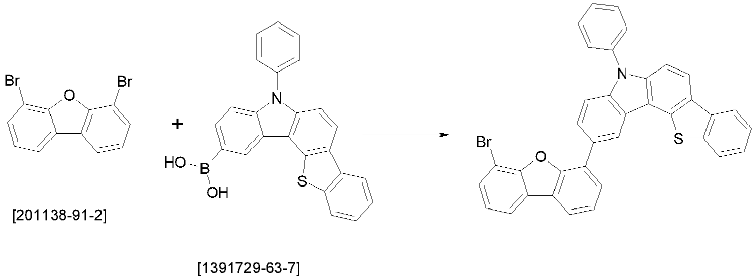

12,5 g (32 mmol) 5--Phenyl-5H[1]benzothieno[3,2-c]carbazol-3-yl) boronsäure, 8,9 g (31,6 mmol) 4,6-Dibromdibenzofuran, 31 mL (63 mmol) Na₂CO₃ (2 M-Lösung) werden in 120 mL Toulol, 120 mL Ethanol suspendiert. Zu dieser Suspension werden 0,73 g (0,63 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 13,3 g (22 mmol), entsprechend 73% der Theorie.

Analog können die folgenden Verbindungen erhalten werden.

| Edukt 1 | Edukt 2 | Produkt | Ausbeute |
|---|---|---|---|
| 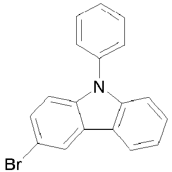 | 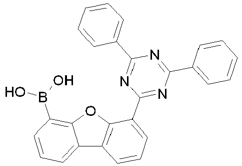 | 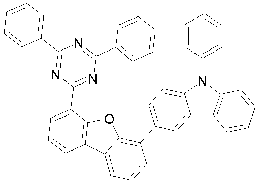 | 87% |
| 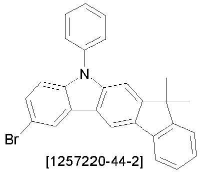 | 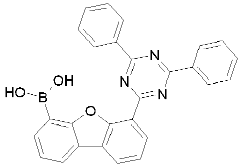 | 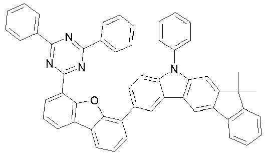 | 89% |

### Beispiel 16

### Herstellung und Charakterisierung der OLEDs

In den folgenden Beispielen V1-V7 und E1-E23 (siehe Tabellen 1 und 2) werden die Daten verschiedener OLEDs vorgestellt.

**Vorbehandlung für die Beispiele V1-E23:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly-(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 70% in Tabelle 2 bedeutet, dass die in Spalte LD angegebene Lebensdauer der Zeit entspricht, nach der die Anfangsleuchtdichte von 4000 cd/m² auf 2800 cd/m² absinkt. Analog bedeutet L0;j0 = 20mA/cm², L1 = 80%, dass die Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiele V1- V7 sind Vergleichsbeispiele gemäß dem Stand der Technik, die Beispiele E1-E23 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben bei Einsatz als Matrix-materialien in phosphoreszierenden OLEDs wesentliche Verbesserungen gegenüber dem Stand der Technik in allen Parametern, vor allem bezüglich Lebensdauer und externer Quanteneffizienz.
Durch Einsatz der erfindungsgemäßen Verbindungen FF1 und FF2 in Kombination mit dem grün emittierenden Dotanden TEG1 lässt sich eine Steigung der Lebendsdauer um ca. 30-40% gegenüber dem Stand der Technik StdT1 und StdT2 beobachten (Beispiele V1, E1 und V2, E2). Desweiteren ermöglicht die erfindungsgemäße Verbindung FF3 eine um ca. 25% erhöhte externe Quanteneffizienz gegenüber dem Stand der Technik StdT3 (Beispiele V3, E3).

**Tabelle 1: Aufbau der OLEDs**

| Bsp | HTL Dicke | IL Dicke | EBL Dicke | EML Dicke | HBL Dicke | ETL Dicke | EIL Dick e |
|---|---|---|---|---|---|---|---|
| V1 | SpA1 70nm | HATCN 5nm | SpMA1 90nm | StdT1:TEG1 (90%:10%) 30nm | --- | ST1:LiQ (50%:50%) 40nm | --- |
| V2 | SpA1 70nm | HATCN 5nm | SpMA1 90nm | StdT2:TEG1 (90%:10%) 30nm | --- | ST1:LiQ (50%:50%) 40nm | --- |
| V3 | SpA1 70nm | HATCN 5nm | SpMA1 90nm | StdT3:TEG1 (90%:10%) 30nm | --- | ST1:LiQ (50%:50%) 40nm | --- |
| V4 | SpA1 70nm | HATCN 5nm | SpMA1 90nm | StdT4:TEG1 (90%:10%) 30nm | --- | ST1:LiQ (50%:50%) 40nm | --- |
| V5 | SpA1 70nm | HATCN 5nm | SpMA1 90nm | StdT5:TEG1 (90%:10%) 30nm | --- | ST1:LiQ (50%:50%) 40nm | --- |
| V6 | SpA1 70nm | HATCN 5nm | SpMA1 90nm | StdT6:TEG1 (90%:10%) 30nm | --- | ST1:LiQ (50%:50%) 40nm | --- |
| V7 | SpA1 70nm | HATCN 5nm | SpMA1 90nm | StdT7:TEG1 (90%:10%) 30nm | --- | ST1:LiQ (50%:50%) 40nm | --- |
| E1 | SpA1 70nm | HATCN 5nm | SpMA1 90nm | FF1:TEG1 (90%:10%) 30nm | --- | ST1:LiQ (50%:50%) 40nm | --- |
| E2 | SpA1 70nm | HATCN 5nm | SpMA1 90nm | FF2:TEG1 (90%:10%) 30nm | --- | ST1:LiQ (50%:50%) 40nm | --- |
| E3 | SpA1 70nm | HATCN 5nm | SpMA1 90nm | FF3:TEG1 (90%:10%) 30nm | --- | ST1:LiQ (50%:50%) 40nm | --- |
| E4 | SpA1 70nm | HATCN 5nm | SpMA1 90nm | FF4:TEG1 (90%:10%) 40nm | --- | ST1:LiQ (50%:50%) 30nm | --- |
| E5 | HATCN 5nm | SpMA1 70nm | SpMA2 15nm | FF2:L1:TEY1 (45%:45%:10%) 25nm | --- | ST1 45nm | LiQ 3nm |
| E6 | SpA1 70nm | HATCN 5nm | SpMA1 90nm | FF5:TEG1 (90%:10%) 30nm | --- | ST1:LiQ (50%:50%) 40nm | --- |
| E7 | SpA1 70nm | HATCN 5nm | SpMA1 90nm | FF2:TEG1 (90%:10%) 30nm | --- | FF6:ST1 (50%:50%) 40nm | LiQ 3nm |
| E8 | SpA1 70nm | HATCN 5nm | SpMA1 90nm | FF7:TEG1 (90%:10%) 30nm | --- | ST1:LiQ (50%:50%) 40nm | --- |
| E9 | SpA1 70nm | HATCN 5nm | SpMA1 90nm | FF8:TEG1 (90%:10%) 30nm | --- | ST1:LiQ (50%:50%) 40nm | --- |
| E10 | SpA1 90nm | HATCN 5nm | SpMA1 130nm | FF9:TER3 (92%:8%) 30nm | --- | ST1:LiQ (50%:50%) 40nm | --- |
| E11 | SpA1 70nm | HATCN 5nm | SpMA1 90nm | FF10:TEG1 (90%:10%) 30nm | --- | ST1:LiQ (50%:50%) 40nm | --- |
| E12 | SpA1 70nm | HATCN 5nm | SpMA1 90nm | FF11:TEG1 (90%:10%) 30nm | --- | ST1:LiQ (50%:50%) 40nm | --- |
| E13 | SpA1 70nm | HATCN 5nm | SpMA1 90nm | FF12:TEG1 (90%:10%) 30nm | --- | ST1:LiQ (50%:50%) 40nm | --- |
| E14 | SpA1 70nm | HATCN 5nm | SpMA1 90nm | FF13:TEG1 (90%:10%) 30nm | IC1 10nm | ST1:LiQ (50%:50%) 30nm | --- |
| E15 | SpA1 70nm | HATCN 5nm | SpMA1 90nm | FF14:IC3:TEG1 (45%:45%:10%) 30nm | IC1 10nm | ST1:LiQ (50%:50%) 30nm | --- |
| E16 | SpA1 70nm | HATCN 5nm | SpMA1 90nm | FF15:IC3:TEG1 (45%:45%:10%) 30nm | IC1 10nm | ST1:LiQ (50%:50%) 30nm | --- |
| E17 | SpA1 70nm | HATCN 5nm | SpMA1 90nm | IC1:TEG1 (90%:10%) 30nm | --- | FF16:ST1 (50%:50%) 40nm | LiF 1nm |
| E18 | SpA1 70nm | HATCN 5nm | SpMA1 90nm | IC1:TEG1 (90%:10%) 30nm | FF17 10nm | ST1:LiQ (50%:50%) 30nm | --- |
| E19 | SpA1 70nm | HATCN 5nm | SpMA1 90nm | IC1 :TEG1 (90%:10%) 30nm | --- | FF18:ST1 (50%:50%) 40nm | LiQ 3nm |
| E20 | SpA1 70nm | HATCN 5nm | SpMA1 90nm | FF19:TEG1 (90%:10%) 30nm | --- | ST1:LiQ (50%:50%) 40nm | |
| E21 | SpA1 70nm | HATCN 5nm | SpMA1 90nm | FF20:TEG1 (90%:10%) 30nm | --- | ST1:LiQ (50%:50%) 40nm | --- |
| E22 | SpA1 70nm | HATCN 5nm | SpMA1 90nm | FF21:TEG1 (90%:10%) 30nm | --- | ST1:LiQ (50%:50%) 40nm | --- |
| E23 | SpA1 70nm | HATCN 5nm | SpMA1 90nm | FF22:TEG1 (90%:10%) 30nm | --- | ST1:LiQ (50%:50%) 40nm | --- |

**Tabelle 2: Daten der OLEDs**

| Bsp. | U1000 (V) | SE1000 (cd/A) | LE1000 (lm/W) | EQE 1000 | CIE x/y bei 1000 cd/m² | L₀; j₀ | L1 % | LD (h) |
|---|---|---|---|---|---|---|---|---|
| V1 | 3.6 | 50 | 44 | 14.4% | 0.33/0.62 | 20mA/cm² | 80 | 120 |
| V2 | 3.3 | 60 | 57 | 17.0% | 0.34/0.62 | 20mA/cm² | 80 | 110 |
| V3 | 4.1 | 41 | 31 | 11.9% | 0.32/0.63 | 20mA/cm² | 80 | 110 |
| V4 | 3.3 | 57 | 54 | 15.5% | 0.32/0.63 | 20mA/cm² | 80 | 100 |
| V5 | 3.7 | 59 | 50 | 15.6% | 0.33/0.62 | 20mA/cm² | 80 | 110 |
| V6 | 3.8 | 61 | 50 | 16.3% | 0.33/0.62 | 20mA/cm² | 80 | 120 |
| V7 | 3.7 | 65 | 55 | 17.3% | 0.33/0.63 | 20mA/cm² | 80 | 160 |
| E1 | 3.5 | 52 | 46 | 14.6% | 0.33/0.62 | 20mA/cm² | 80 | 160 |
| E2 | 3.3 | 63 | 59 | 17.2% | 0.33/0.63 | 20mA/cm² | 80 | 155 |
| E3 | 3.5 | 51 | 45 | 14.8% | 0.34/0.62 | 20mA/cm² | 80 | 100 |
| E4 | 3.1 | 65 | 66 | 17.4% | 0.33/0.63 | 20mA/cm² | 80 | 150 |
| E5 | 2.7 | 86 | 100 | 24.5% | 0.42/0.57 | 50mA/cm² | 90 | 95 |
| E6 | 3.4 | 65 | 60 | 17.1% | 0.33/0.62 | 20mA/cm² | 80 | 150 |
| E7 | 3.3 | 56 | 53 | 16.2% | 0.33/0.62 | 20mA/cm² | 80 | 145 |
| E8 | 3.5 | 62 | 56 | 16.9% | 0.38/0.59 | 20mA/cm² | 80 | 130 |
| E9 | 3.4 | 60 | 55 | 16.8% | 0.38/0.59 | 20mA/cm² | 80 | 145 |
| E10 | 4.5 | 11 | 8 | 12.0% | 0.67/0.33 | 4000 cd/m² | 80 | 310 |
| E11 | 3.6 | 50 | 44 | 14.4% | 0.33/0.62 | 20mA/cm² | 80 | 130 |
| E12 | 3.4 | 63 | 58 | 17.0% | 0.32/0.63 | 20mA/cm² | 80 | 160 |
| E13 | 3.5 | 62 | 56 | 17.3% | 0.32/0.63 | 20mA/cm² | 80 | 150 |
| E14 | 3.6 | 57 | 50 | 15.3% | 0.33/0.62 | 20mA/cm² | 80 | 105 |
| E15 | 3.4 | 57 | 53 | 16.0% | 0.34/0.63 | 20 mA/cm² | 80 | 410 |
| E16 | 3.5 | 58 | 52 | 16.1% | 0.34/0.63 | 20 mA/cm² | 80 | 290 |
| E17 | 3.2 | 67 | 66 | 18.1% | 0.33/0.62 | 20 mA/cm² | 80 | 160 |
| E18 | 3.3 | 66 | 62 | 17.8% | 0.33/0.62 | 20 mA/cm² | 80 | 170 |
| E19 | 3.1 | 69 | 70 | 18.4% | 0.33/0.62 | 20 mA/cm² | 80 | 165 |
| E20 | 3.5 | 62 | 56 | 17.1% | 0.34/0.62 | 20 mA/cm² | 80 | 135 |
| E21 | 3.5 | 57 | 51 | 15.6% | 0.33/0.63 | 20mA/cm² | 80 | 85 |
| E22 | 3.4 | 51 | 47 | 14.8% | 0.34/0.62 | 20mA/cm² | 80 | 80 |
| E23 | 3.6 | 53 | 46 | 15.0% | 0.33/0.62 | 20mA/cm² | 80 | 95 |

**Tabelle 3: Strukturformeln der Materialien für die OLEDs**

| | |
|---|---|
| 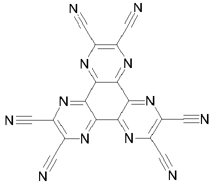 | 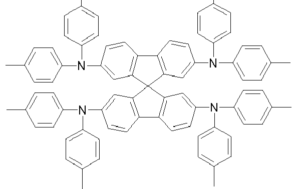 |
| HATCN | SpA1 |
| 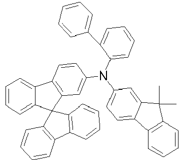 | 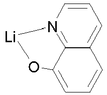 |
| SpMA1 | LiQ |
| 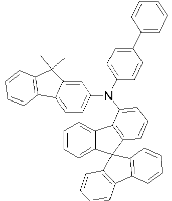 | 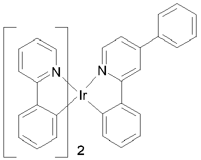 |
| SpMA2 | TEY1 |
| 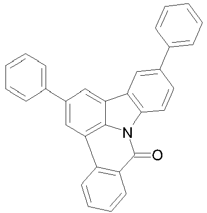 | 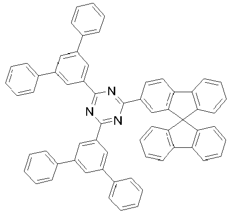 |
| L1 | ST1 |
| 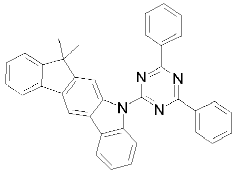 | 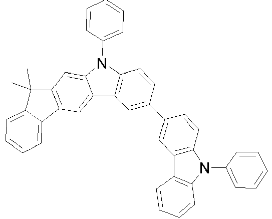 |
| IC1 | IC3 |
| 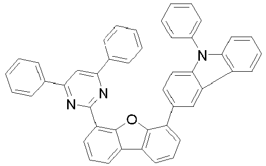 | 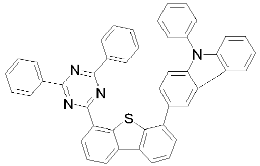 |
| StdT1 | StdT2 |
| 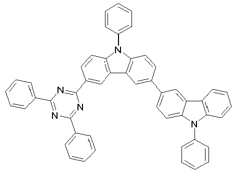 | 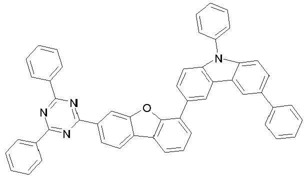 |
| StdT3 | StdT4 |
| 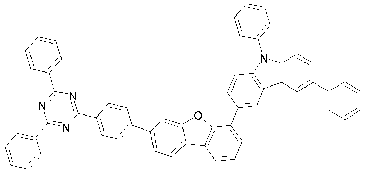 | 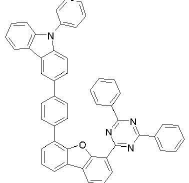 |
| StdT5 | StdT6 |
| 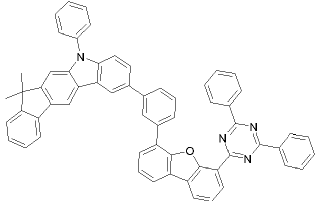 | 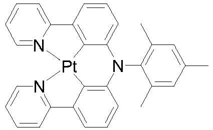 |
| StdT7 | TER3 |
| 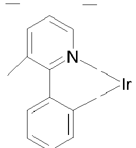 | |
| TEG1 | |
| 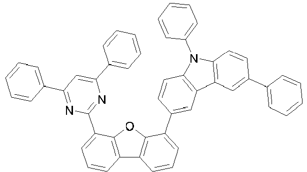 | 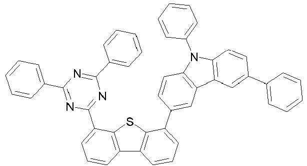 |
| FF1 | FF2 |
| 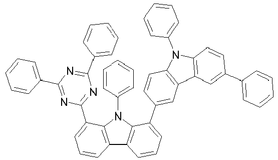 | 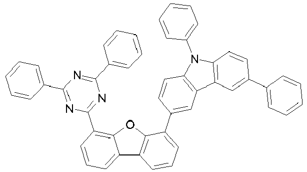 |
| FF3 | FF4 |
| 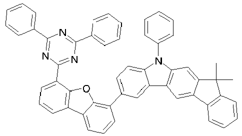 | 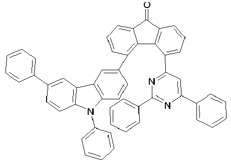 |
| FF5 | FF6 |
| 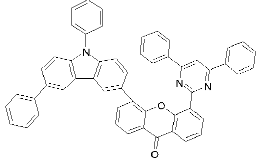 | 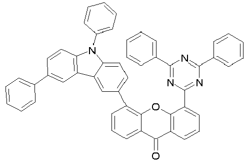 |
| FF7 | FF8 |
| 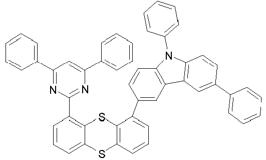 | 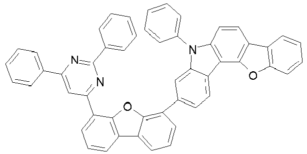 |
| FF9 | FF10 |
| 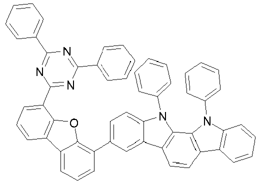 | 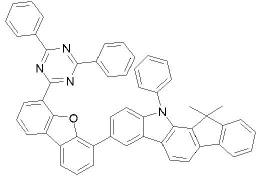 |
| FF11 | FF12 |
| 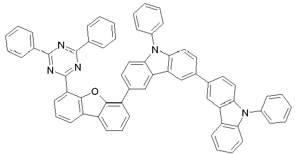 | 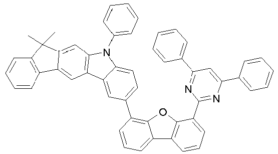 |
| FF13 | FF14 |
| 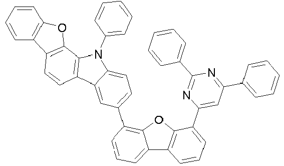 | 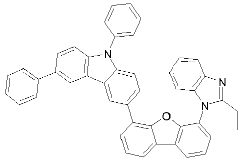 |
| FF15 | FF16 |
| 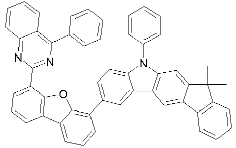 | 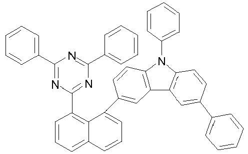 |
| FF17 | FF18 |
| 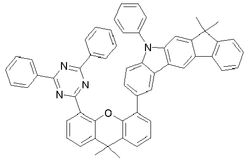 | 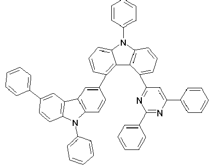 |
| FF19 | FF20 |
| 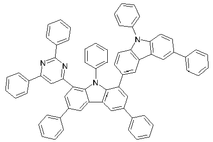 | 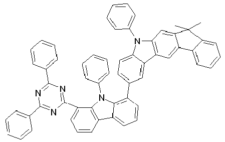 |
| FF21 | FF22 |

## Patentansprüche

1. Verbindung der allgemeinen Formel (2) 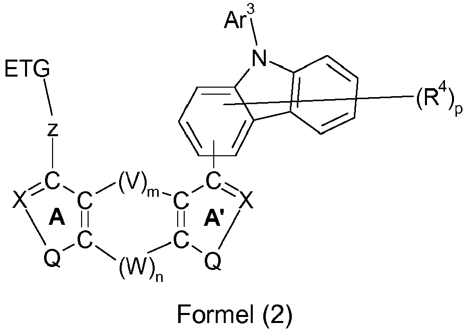 wobei für die verwendeten Symbole und Indizes gilt:
X ist gleich oder verscheiden bei jeden Auftreten N oder CR¹;
Q ist gleich oder verschieden bei jedem Auftreten X=X, S, O oder NR¹, bevorzugt X=X, S und O, ganz bevorzugt X=X und S und ganz besonders bevorzugt X=X
ETG ist eine organische elektronentransportierende Gruppe (ETG) aus der Gruppe der elektronenarmen heteroaromatischen Gruppen, wobei die ETG ausgewählt ist aus der Gruppe der Pyrazine, Pyrimidine, Pyridazine, 1,2,4-Triazine, 1,3,5-Triazine, Chinoxaline, Pyrazole, Imidazole und Benzimidazole, wobei die Gruppe ETG mit einem oder mehreren voneinander unabhängigen Resten R¹ substituiert sein kann;
Z ist eine Einfachbindung oder eine bivalente Gruppe; wenn Z eine Einfachbindung ist, dann bindet die Gruppe ETG direkt an das Kohlenstoffatom des Rings A;
V ist eine Einfachbindung, C=O, C(R¹)₂, NAr³, O, S, Si(R¹)₂, BR¹, PR¹, P(=O)R¹, SO oder SO₂, wobei im Fall einer Einfachbindung die Kohlenstoffatome der Ringe A und A' direkt durch eine Einfachbindung miteinander verbunden sind, wobei eine Einfachbindung, C(R¹)₂, NAr³, O und S bevorzugt sind, wobei eine Einfachbindung, C(R¹)₂, O und S ganz bevorzugt sind, wobei O und S ganz besonders bevorzugt sind, wobei O insbesondere bevorzugt ist;
W ist eine Einfachbindung, C=O, C(R¹)₂, NR¹, O, S, Si(R¹)₂, BR¹, PR¹, P(=O)R¹, SO oder SO₂, wobei im Fall einer Einfachbindung die Kohlenstoffatome der Ringe A und A' direkt durch eine Einfachbindung miteinander verbunden sind, wobei eine Einfachbindung, C(R¹)₂, NR¹, O und S bevorzugt sind, wobei eine Einfachbindung, C(R¹)₂, O und S ganz bevorzugt sind, wobei O und S ganz besonders bevorzugt sind, wobei O insbesondere bevorzugt ist;
m ist entweder 0 oder 1;
n ist entweder 0 oder 1,
wobei m = n gilt;
Ar³ ist ein aromatischer oder heteroaromatischer Ring oder Ringsystem mit 5 bis 30 Ring-atomen, wobei der Ring oder das Ringsystem jeweils durch einen oder mehrere Reste R² substi-tuiert sein kann, die durch einen oder mehrere Reste R³ substituiert sein können, wobei zwei oder mehr Reste R² miteinander einen Ringschluss bilden können;
R¹ ist gleich oder verschieden bei jedem Auftreten H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Arylalkoxy- oder Heteroaryloxygruppe mit 5 bis 60 aromatischen Ringatomen, die durch einen oder mehrere Reste R² substituiert sein kann, oder eine Diarylaminogruppe, Dihetero-arylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen oder eine vernetzbare Gruppe Q; dabei können zwei oder mehrere benachbarte Reste R¹ miteinander ein mono- oder polycyclisches, aliphatisches oder aromatisches Ringsystem bilden, wobei bevorzugt ist, wenn zwei oder mehr benachbarte Reste R¹ miteinander kein mono- oder polycyclisches, aliphatisches oder aromatisches Ringsystem bilden;
R² ist gleich oder verschieden bei jedem Auftreten H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy-, Arylalkoxy- oder Heteroaryloxygruppe mit 5 bis 60 aromatischen Ringatomen, die durch einen oder mehrere Reste R³ substituiert sein kann, oder eine Diarylaminogruppe, Dihetero-arylaminogruppe oder Arylheteroarylaminogruppe mit 10 bis 40 aromatischen Ringatomen, welche durch einen oder mehrere Reste R³ substituiert sein kann, oder eine Kombination aus zwei oder mehr dieser Gruppen; dabei können zwei oder mehrere benachbarte Reste R² miteinander ein mono- oder polycyclisches, aliphatisches oder aromatisches Ringsystem bilden;
R³ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches oder aromatisches Ringsystem bilden;
p ist 1 oder 2, ganz besonders bevorzgt genau 2 und insbesonders bevorzugt genau 1;
R⁴ ist gleich oder verschieden bei jedem Auftreten ein aromatisches oder heteroaromatisches Ringsystem mit 5 bis 60 aromatischen Ringatomen, das jeweils durch einen oder mehrere Reste R² substituiert sein kann; dabei können zwei oder mehrere benachbarte Reste R⁴ miteinander ein mono- oder polycyclisches, aliphatisches oder aromatisches Ringsystem bilden;
und wobei die folgenden Verbindungen ausgenommen sind:

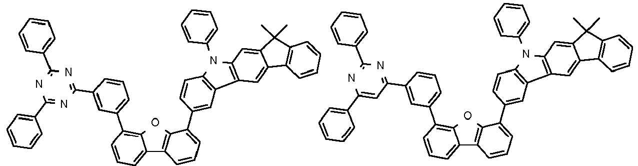

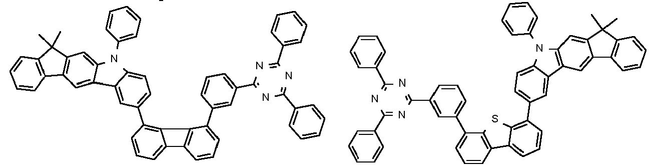

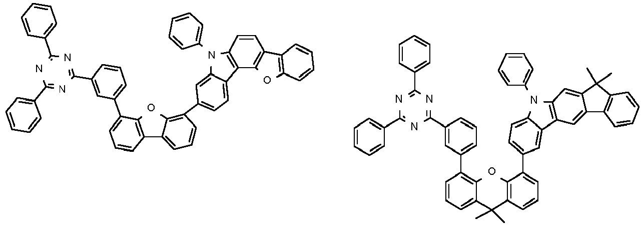

2. Verbindung gemäß Anspruch 1, **dadurch gekennzeichnet, dass** sie die Formel (3) aufweist

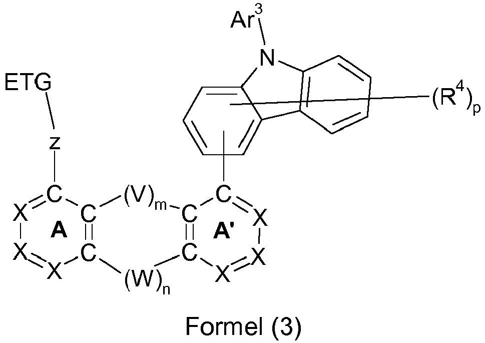

3. Verbindung gemäß Anspruch 1 oder 2, **dadurch gekennzeichnet, dass** sie die Formel (3a) aufweist 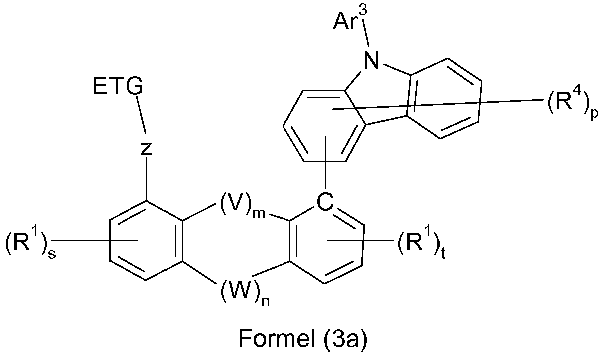 wobei s und t ganze Zahlen von 0 bis 3 sein können und wobei s+t gleich eine ganze Zahl von 0 bis 6 ist, bevorzugt ist s+t gleich 4, ganz bevorzugt ist s+t gleich 2, ganz besonders bevorzugt ist s+t gleich 1 und insbesondere bevorzugt ist s+t gleich 0.

4. Verbindung gemäß einem oder mehreren der Ansprüche 1 bis 3, **dadurch gekennzeichnet, dass** sie die Formel (3b) aufweist 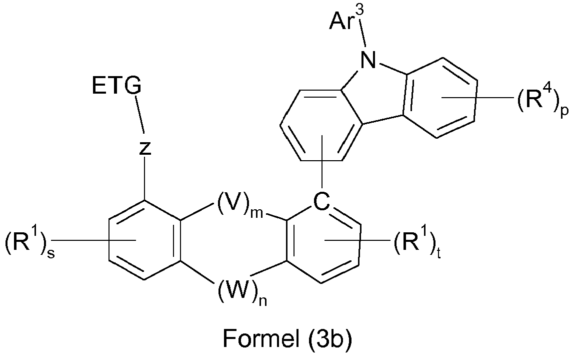 wobei p genau 2 und ganz bevorzugt genau 1 ist.

5. Verbindung gemäß einem oder mehreren der Ansprüche 1 bis 4, **dadurch gekennzeichnet, dass** sie die Formel (3c) aufweist

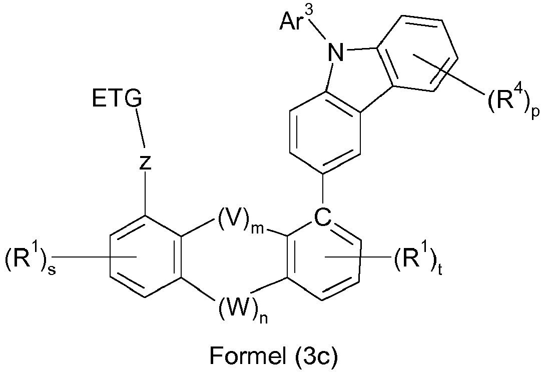

6. Verbindung gemäß einem oder mehreren der Ansprüche 1 bis 5, **dadurch gekennzeichnet, dass** die ETG eine elektronenarme heteroaromatische Gruppe ist und ausgewählt wird aus den folgenden Gruppen

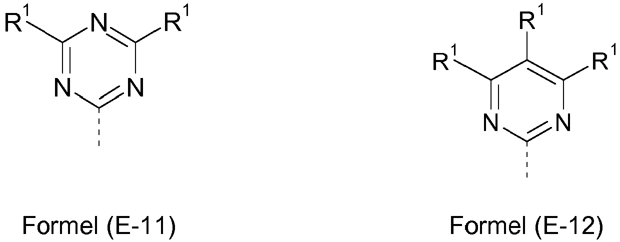

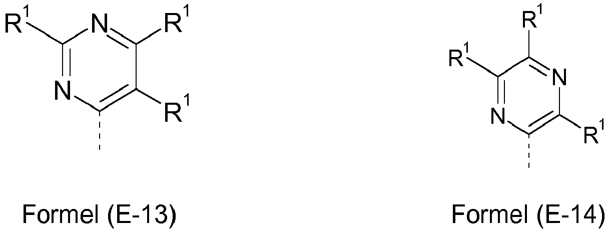

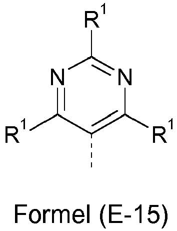

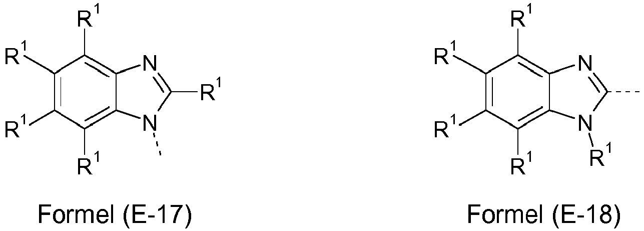

7. Verbindung gemäß einem oder mehreren der Ansprüche 1 bis 5, **dadurch gekennzeichnet, dass** Z eine Einfachbindung oder ein bivalenter aromatischer oder heteroaromatischer Ring oder Ringsystem mit 5 bis 60 Ringatomen ist und bevorzugt ein aromatischer Ring oder Ringsystem mit 6 bis 60 Ringatomen ist.

8. Verfahren zur Herstellung der Verbindung gemäß einem oder mehreren der Ansprüche 1 bis 7 mit Hilfe der Suzuki-Kupplung.

9. Verfahren zur Herstellung der Verbindung gemäß einem oder mehreren der Ansprüche 1 bis 7 mit Hilfe der Buchwald- oder Ullmann-Kupplung

10. Zusammensetzung enthaltend wenigstens eine Verbindung gemäß einem oder mehreren der Ansprüche 1 bis 7 sowie wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien, n-Dotanden und p-Dotanden.

11. Zusammensetzung gemäß Anspruch 10, dadurch charakterisiert, dass die zusätzliche Verbindung ein phosphoreszierender Emitter ist.

12. Zusammensetzung gemäß Anspruch 10 oder 11, dadurch charakterisiert, dass die zusätzliche Verbindung ein Host- oder Matrixmaterial ist.

13. Zusammensetzung gemäß einem oder meherern der Ansprüche 10 bis 13, dadurch charakterisiert, dass die zusätzliche Verbindung eine Bandlücke (band gap) von 2.5 eV oder mehr, bevorzugt 3.0 eV oder mehr, ganz bevorzugt von 3.5 eV oder mehr aufweist.

14. Formulierung enthaltend wenigstens einer Verbindung gemäß einem oder mehreren der Ansprüche 1 bis 7 oder wenigstens einer Zusammensetzung gemäß einem oder mehreren der Ansprüche 10 bis 15 sowie wenigstens ein Lösungsmittel.

15. Verwendung wenigstens einer Verbindung gemäß einem oder mehreren der Ansprüche 1 bis 7 oder wenigstens einer Zusammensetzung gemäß einem oder mehreren der Ansprüche 10 bis 13 in einer elektronischen Vorrichtung, bevorzugt in einer organischen Elektrolumineszenzvorrichtung, ganz bevorzugt in einer organischen lichtemittierenden Diode (OLED) oder organischen lichtemittierenden elektrochemischen Zelle (OLEC, LEEC, LEC), ganz besonders bevorzugt in einer OLED, bevorzugt in einer Emissionsschicht (EML), Elektronentransportschicht (ETL) und in einer Lochblockierschicht (HBL), ganz bevorzugt in einer EML und ETL und ganz besonders bevorzugt in einer EML.

16. Elektronische Vorrichtung enthaltend wenigstens eine Verbindung gemäß einem oder mehreren der Ansprüche 1 bis 7 oder wenigstens eine Zusammensetzung gemäß einem oder mehreren der Ansprüche 10 bis 13, bevorzugt in einer Emissionsschicht (EML), Elektronentransportschicht (ETL) und in einer Lochblockierschicht (HBL), ganz bevorzugt in einer EML und ETL und ganz besonders bevorzugt in einer EML.

17. Elektronische Vorrichtung gemäß Anspruch 16, **dadurch gekennzeichnet, dass** sie gewählt ist aus organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren, organischen Photorezeptoren, bevorzugt eine organische Elektroluminesszenzvorrichtung.

18. Elektronische Vorrichtung gemäß Anspruch 16 oder 17, **dadurch gekennzeichnet, dass** sie eine organische Elektrolumineszenzvorrichtung ist, die ausgewählt ist auch der Gruppe bestehend aus organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

19. Verfahren zur Herstellung einer elektronischen Vorrichtung gemäß einem oder mehreren der Ansprüche 16 bis 18, **dadurch gekennzeichnet, dass** mindestens eine organische Schicht durch Gasphasenabscheidung oder aus Lösung aufgebracht wird.

20. Elektronische Vorrichtung gemäß Anspruch 18, zur Verwendung in der Medizin zur Phototherapie, bevorzugt zur Verwendung zur Phototherapie der Haut, ganz bevorzugt zur Verwendung zur Behandlung oder Vorbeugung von Psoriasis, atopischer Dermatitis, Ikterus, Neugeborenenikterus, Vitiligo, Inflammation, Schwerzen und zur Verwendung zur Wundheilung.

21. Verwendung der Vorrichtung gemäß Anspruch 18 in der Kosmetik, bevorzugt zur Behandlung oder Vorbeugung von Hautalterung (Skin Ageing), Hautfalten, Krähenfüsse, Akne, Komedonen und Zellulithe.

22. Verwendung der Vorrichtung gemäß Anspruch 18 in Displays oder zur Beleuchtung.

## Claims

1. Compound of the general formula (2) 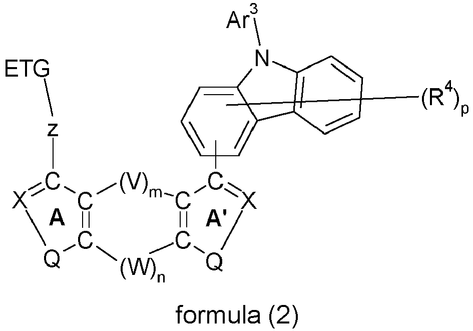 where following applies to the symbols and indices used:
X is, identically or differently on each occurrence, N or CR¹;
Q is, identically or differently on each occurrence, X=X, S, O or NR¹, preferably X=X, S and O, very preferably X=X and S and very particularly preferably X=X.
ETG is an organic electron-transporting group (ETG) from the group of electron-deficient heteroaromatic groups, where the ETG is selected from the group of the pyrazines, pyrimidines, pyridazines, 1,2,4-triazines, 1,3,5-triazines, quinoxalines, pyrazoles, imidazoles and benzimidazoles, where the group ETG may be substituted by one or more radicals R¹, which are independent of one another;
Z is a single bond or a divalent group; if Z is a single bond, the group ETG is then bonded directly to the carbon atom of ring A;
V is a single bond, C=O, C(R¹)₂, NAr³, O, S, Si(R¹)₂, BR¹, PR¹, P(=O)R¹, SO or SO₂, where, in the case of a single bond, the carbon atoms of rings A and A' are connected directly to one another by a single bond, where a single bond, C(R¹)₂, NAr³, O and S are preferred, where a single bond, C(R¹)₂, O and S are very preferred, where O and S are very particularly preferred, where O is especially preferred;
W is a single bond, C=O, C(R¹)₂, NR¹, O, S, Si(R¹)₂, BR¹, PR¹, P(=O)R¹, SO or SO₂, where, in the case of a single bond, the carbon atoms of rings A and A' are connected directly to one another by a single bond, where a single bond, C(R¹)₂, NR¹, O and S are preferred, where a single bond, C(R¹)₂, O and S are very preferred, where O and S are very particularly preferred, where O is especially preferred;
m is either 0 or 1;
n is either 0 or 1,
where m = n;
Ar³ is an aromatic or heteroaromatic ring or ring system having 5 to 30 ring atoms, where the ring or ring system may in each case be substituted by one or more radicals R², which may be substituted by one or more radicals R³, where two or more radicals R² may form a ring closure with one another;
R¹ is, identically or differently on each occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy, arylalkoxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups or a cross-linkable group Q; two or more adjacent radicals R¹ may form a mono- or polycyclic, aliphatic or aromatic ring system with one another, where it is preferred for two or more adjacent radicals R¹ not to form a mono- or polycyclic, aliphatic or aromatic ring system with one another;
R² is, identically or differently on each occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals R³, where one or more non-adjacent CH₂ groups may be replaced by R³C=CR³, C≡C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy, arylalkoxy or heteroaryloxy group having 5 to 60 aromatic ring atoms, which may be substituted by one or more radicals R³, or a diarylamino group, diheteroarylamino group or arylheteroarylamino group having 10 to 40 aromatic ring atoms, which may be substituted by one or more radicals R³, or a combination of two or more of these groups; two or more adjacent radicals R² may form a mono- or polycyclic, aliphatic or aromatic ring system with one another;
R³ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents R³ may also form a mono- or polycyclic, aliphatic or aromatic ring system with one another;
p is 1 or 2, very particularly preferably precisely 2 and especially preferably precisely 1;
R⁴ is, identically or differently on each occurrence, an aromatic or heteroaromatic ring system having 5 to 60 aromatic ring atoms, which may in each case be substituted by one or more radicals R²; two or more adjacent radicals R⁴ may form a mono- or polycyclic, aliphatic or aromatic ring system with one another,
and where the following compounds are excluded:

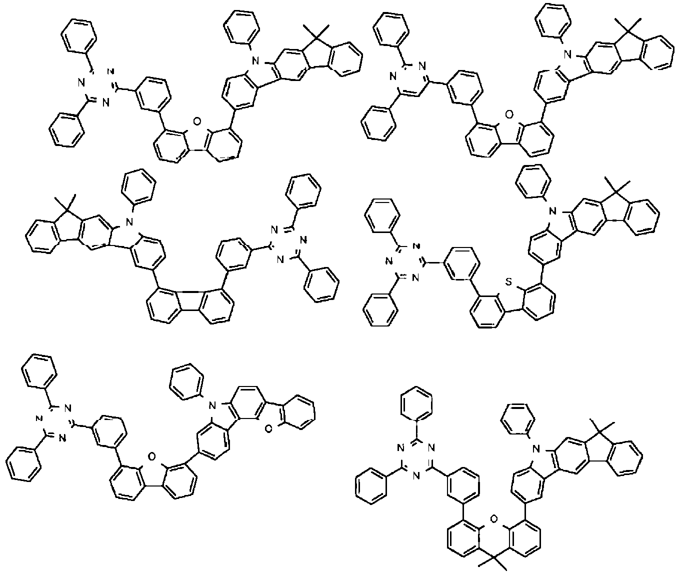

2. Compound according to Claim 1, **characterised in that** it has the formula (3)

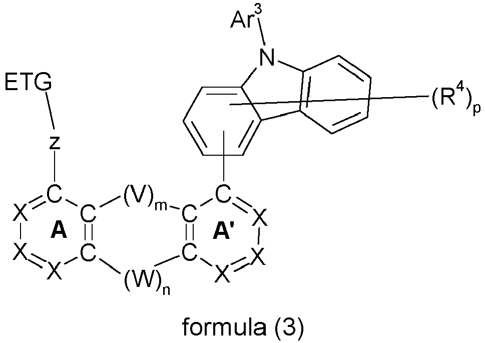

3. Compound according to Claim 1 to 2, **characterised in that** it has the formula (3a) 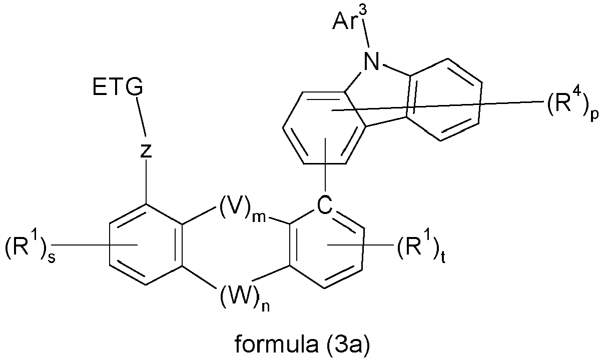 where s and t can be integers from 0 to 3 and where s+t is equal to an integer from 0 to 6, s+t is preferably equal to 4, s+t is very preferably equal to 2, s+t is very particularly preferably equal to 1 and s+t is especially preferably equal to 0.

4. Compound according to one or more of Claims 1 to 3, **characterised in that** it has the formula (3b) 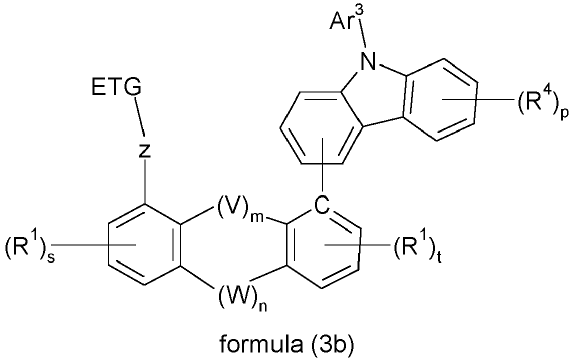 where p is precisely 2 and very preferably precisely 1.

5. Compound according to one or more of Claims 1 to 4, **characterised in that** it has the formula (3c)

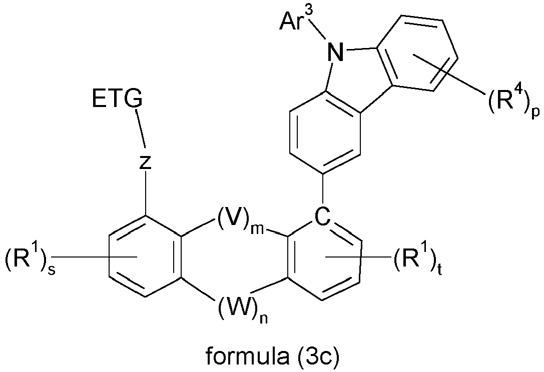

6. Compound according to one or more of Claims 1 to 5, **characterised in that** the ETG is an electron-deficient heteroaromatic group and is selected from the following groups

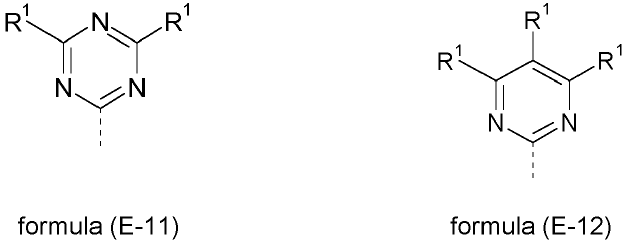

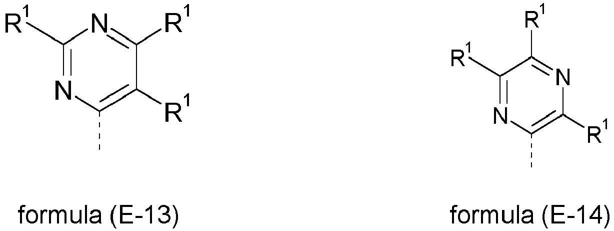

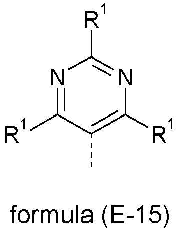

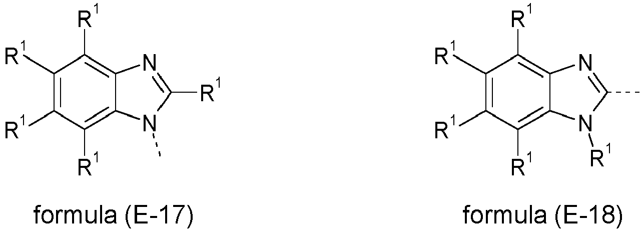

7. Compound according to one or more of Claims 1 to 5, **characterised in that** Z is a single bond or a divalent aromatic or heteroaromatic ring or ring system having 5 to 60 ring atoms and is preferably an aromatic ring or ring system having 6 to 60 ring atoms.

8. Process for the preparation of the compound according to one or more of Claims 1 to 7 with the aid of Suzuki coupling.

9. Process for the preparation of the compound according to one or more of Claims 1 to 7 with the aid of Buchwald or Ullmann coupling.

10. Composition comprising at least one compound according to one or more of Claims 1 to 7 and at least one further compound selected from the group consisting of fluorescent emitters, phosphorescent emitters, host materials, matrix materials, electron-transport materials, electron-injection materials, hole-conductor materials, hole-injection materials, electron-blocking materials, hole-blocking materials, n-dopants and p-dopants.

11. Composition according to Claim 10, **characterised in that** the additional compound is a phosphorescent emitter.

12. Composition according to Claim 10 or 11, **characterised in that** the additional compound is a host material or matrix material.

13. Composition according to one or more of Claims 10 to 12, **characterised in that** the additional compound has a band gap of 2.5 eV or more, preferably 3.0 eV or more, very preferably 3.5 eV or more.

14. Formulation comprising at least one compound according to one or more of Claims 1 to 7 or at least one composition according to one or more of Claims 10 to 13 and at least one solvent.

15. Use of at least one compound according to one or more of Claims 1 to 7 or at least one composition according to one or more of Claims 10 to 13 in an electronic device, preferably in an organic electroluminescent device, very preferably in an organic light-emitting diode (OLED) or organic light-emitting electrochemical cell (OLEC, LEEC, LEC), very particularly preferably in an OLED, preferably in an emission layer (EML), electron-transport layer (ETL) and in a hole-blocking layer (HBL), very preferably in an EML and ETL and very particularly preferably in an EML.

16. Electronic device comprising at least one compound according to one or more of Claims 1 to 7 or at least one composition according to one or more of Claims 10 to 13, preferably in an emission layer (EML), electron-transport layer (ETL) and in a hole-blocking layer (HBL), very preferably in an EML and ETL and very particularly preferably in an EML.

17. Electronic device according to Claim 16, **characterised in that** it is selected from organic integrated circuits (OICs), organic field-effect transistors (OFETs), organic thin-film transistors (OTFTs), organic electroluminescent devices, organic solar cells (OSCs), organic optical detectors, organic photoreceptors, preferably an organic electroluminescent device.

18. Electronic device according to Claim 16 or 17, **characterised in that** it is an organic electroluminescent device selected from the group consisting of organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

19. Process for the production of an electronic device according to one or more of Claims 16 to 18, **characterised in that** at least one organic layer is applied by gas-phase deposition or from solution.

20. Electronic device according to Claim 18, for use in medicine for phototherapy, preferably for use for phototherapy of the skin, very preferably for use for the treatment or prevention of psoriasis, atopic dermatitis, jaundice, jaundice of the newborn, vitiligo, inflammation, pain and for use for wound healing.

21. Use of the device according to Claim 18 in cosmetics, preferably for the treatment or prevention of skin ageing, skin wrinkles, crow's feet, acne, blackheads and cellulite.

22. Use of the device according to Claim 18 in displays or for lighting.

## Revendications

1. Composé de la formule générale (2) 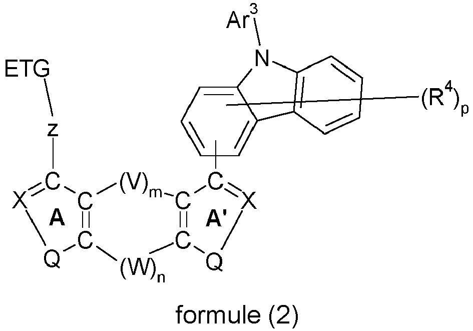 dans laquelle ce qui suit s'applique aux symboles et aux indices qui sont utilisés :
X est, de manière identique ou différente pour chaque occurrence, N ou CR¹ ;
Q est, de manière identique ou différente pour chaque occurrence, X=X, S, O ou NR¹, de préférence X=X, S et O, de façon très préférable X=X et S, et de façon très particulièrement préférable X=X,
ETG est un groupe de transport d'électrons organique (ETG) pris parmi l'ensemble de groupes hétéroaromatiques déficients en électrons, où le groupe ETG est sélectionné parmi le groupe des pyrazines, des pyrimidines, des pyridazines, des 1,2,4-triazines, des 1,3,5-triazines, des quinoxalines, des pyrazoles, des imidazoles et des benzimidazoles, où le groupe ETG peut être substitué par un radical ou par plusieurs radicaux R¹, lesquels radicaux sont indépendants les uns des autres ;
Z est une liaison simple ou un groupe divalent ; si Z est une liaison simple, le groupe ETG est alors lié directement à l'atome de carbone de cycle A ;
V est une liaison simple, C=O, C(R¹)₂, NAr³, O, S, Si(R¹)₂, BR¹, PR¹, P(=O)R¹, SO ou SO₂, où, dans le cas d'une liaison simple, les atomes de carbone de cycles A et A' sont connectés directement l'un à l'autre ou les uns aux autres par une liaison simple, où une liaison simple, C(R¹)₂, NAr³, O et S ont la préférence, où une liaison simple, C(R¹)₂, O et S sont très préférés, où O et S sont très particulièrement préférés, où O est tout spécialement préféré ;
W est une liaison simple, C=O, C(R¹)₂, NR¹, O, S, Si(R¹)₂, BR¹, PR¹, P(=O)R¹, SO ou SO₂, où, dans le cas d'une liaison simple, les atomes de carbone de cycles A et A' sont connectés directement l'un à l'autre ou les uns aux autres par une liaison simple, où une liaison simple, C(R¹)₂, NR¹, O et S ont la préférence, où une liaison simple, C(R¹)₂, O et S sont très préférés, où O et S sont très particulièrement préférés, où O est tout spécialement préféré ;
m est soit 0, soit 1 ;
n est soit 0, soit 1 ;
où m = n ;
Ar³ est un cycle ou système de cycle aromatique ou hétéroaromatique qui comporte de 5 à 30 atomes de cycle, où le cycle ou système de cycle peut dans chaque cas être substitué par un radical ou par plusieurs radicaux R², lequel peut être substitué par un radical ou par plusieurs radicaux R³, où deux radicaux R² ou plus peuvent former une fermeture de cycle l'un avec l'autre ou les uns avec les autres ;
R¹ est, de manière identique ou différente pour chaque occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy, arylalcoxy ou hétéroaryloxy qui comporte de 5 à 60 atomes de cycle aromatique, lequel peut être substitué par un radical ou par plusieurs radicaux R², ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R², ou une combinaison de deux de ces groupes ou plus ou un groupe réticulable Q ; deux radicaux R¹ adjacents ou plus peuvent former un système de cycle aliphatique ou aromatique mono- ou polycyclique l'un avec l'autre ou les uns avec les autres, où il est préférable que deux radicaux R¹ adjacents ou plus ne forment pas un système de cycle aliphatique ou aromatique mono- ou polycyclique l'un avec l'autre ou les uns avec les autres ;
R² est, de manière identique ou différente pour chaque occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux R³, où un ou plusieurs groupe(s) CH₂ non adjacents peut/ peuvent être remplacé(s) par R³C=CR³, C=C, Si(R³)₂, Ge(R³)₂, Sn(R³)₂, C=O, C=S, C=Se, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R³, ou un groupe aryloxy, arylalcoxy ou hétéroaryloxy qui comporte de 5 à 60 atomes de cycle aromatique, lequel peut être substitué par un radical ou par plusieurs radicaux R³, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R³, ou une combinaison de deux ou plus de ces groupes ; deux radicaux R² adjacents ou plus peuvent former un système de cycle aliphatique ou aromatique mono- ou polycyclique l'un avec l'autre ou les uns avec les autres ;
R³ est, de manière identique ou différente pour chaque occurrence, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants R³ ou plus peuvent également former un système de cycle aliphatique ou aromatique mono- ou polycyclique l'un avec l'autre ou les uns avec les autres ;
p est 1 ou 2, de façon très particulièrement préférable, précisément 2 et de façon tout spécialement préférable, précisément 1 ;
R⁴ est, de manière identique ou différente pour chaque occurrence, un système de cycle aromatique ou hétéroaromatique qui comporte de 5 à 60 atomes de cycle aromatique, lequel peut dans chaque cas être substitué par un radical ou par plusieurs radicaux R² ; deux radicaux R⁴ adjacents ou plus peuvent former un système de cycle aliphatique ou aromatique mono- ou polycyclique l'un avec l'autre ou les uns avec les autres,
et où les composés qui suivent sont exclus :

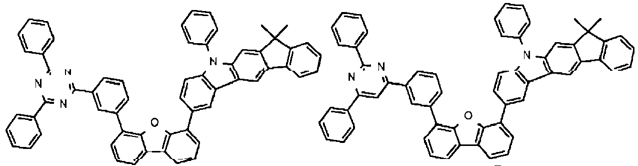

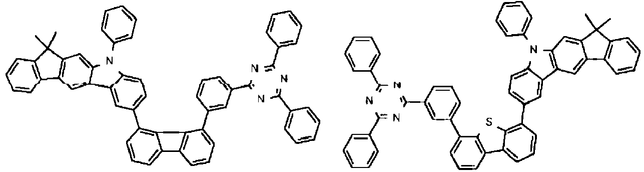

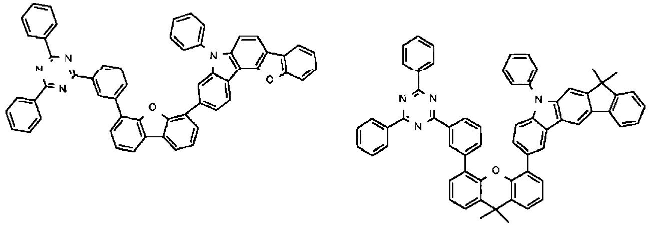

2. Composé selon la revendication 1, **caractérisé en ce qu'**il présente la formule (3)

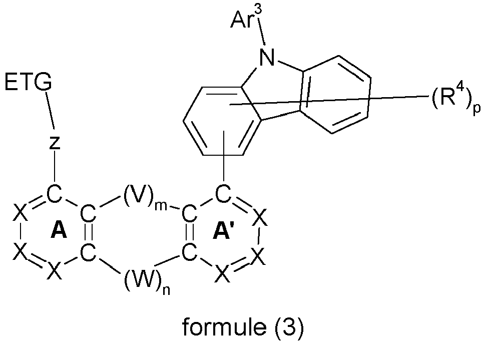

3. Composé selon les revendications 1 et 2, **caractérisé en ce qu'**il présente la formule (3a) 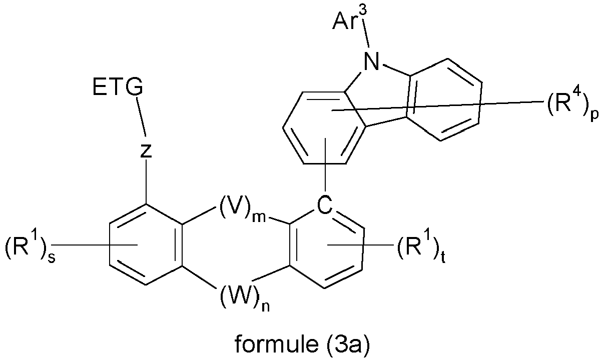 dans laquelle s et t peuvent être des entiers de 0 à 3 et où s+t est égal à un entier de 0 à 6, s+t est de préférence égal à 4, s+t est de façon très préférable égal à 2, s+t est de façon très particulièrement préférable égal à 1 et s+t est de façon tout spécialement préférable égal à 0.

4. Composé selon une ou plusieurs des revendications 1 à 3, **caractérisé en ce qu'**il présente la formule (3b) 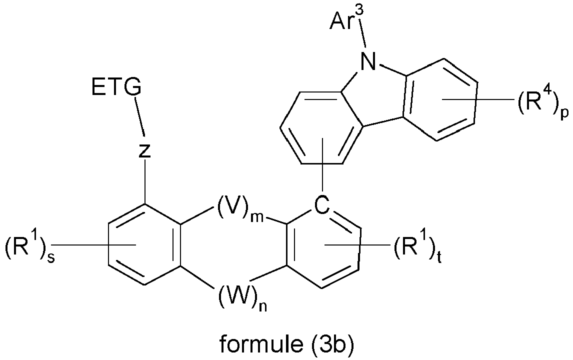 dans laquelle p est précisément 2 et de façon très préférable, est précisément 1.

5. Composé selon une ou plusieurs des revendications 1 à 4, **caractérisé en ce qu'**il présente la formule (3c)

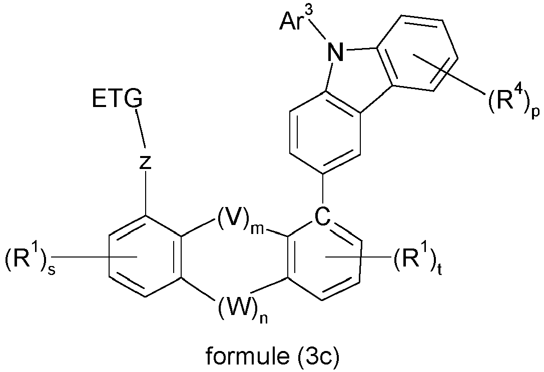

6. Composé selon une ou plusieurs des revendications 1 à 5, **caractérisé en ce que** le groupe ETG est un groupe hétéroaromatique déficient en électrons et est sélectionné parmi les groupes qui suivent

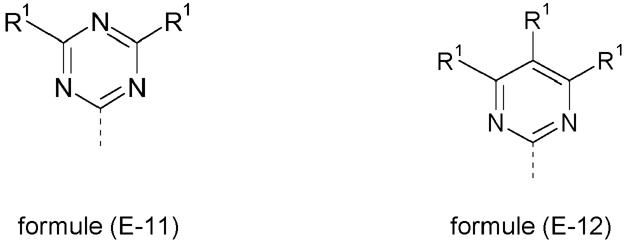

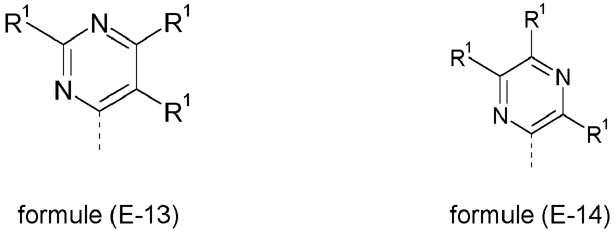

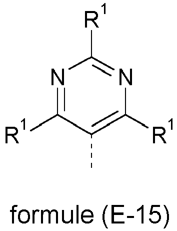

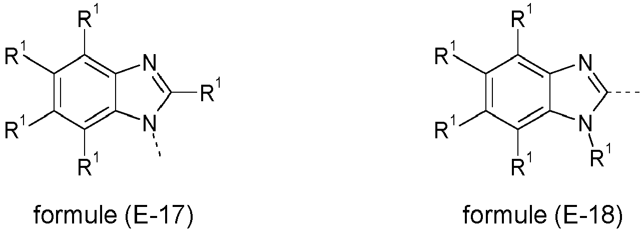

7. Composé selon une ou plusieurs des revendications 1 à 5, **caractérisé en ce que** Z est une liaison simple ou un cycle ou système de cycle aromatique ou hétéroaromatique divalent qui comporte de 5 à 60 atomes de cycle et est de préférence un cycle ou système de cycle aromatique qui comporte de 6 à 60 atomes de cycle.

8. Procédé pour la préparation du composé selon une ou plusieurs des revendications 1 à 7 à l'aide d'un couplage de Suzuki.

9. Procédé pour la préparation du composé selon une ou plusieurs des revendications 1 à 7 à l'aide d'un couplage de Buchwald ou de Ullmann.

10. Composition comprenant au moins un composé selon une ou plusieurs des revendications 1 à 7 et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons, les matériaux de blocage de trous, les dopants n et les dopants p.

11. Composition selon la revendication 10, **caractérisée en ce que** le composé additionnel est un émetteur phosphorescent.

12. Composition selon la revendication 10 ou 11, **caractérisée en ce que** le composé additionnel est un matériau hôte ou un matériau de matrice.

13. Composition selon une ou plusieurs des revendications 10 à 12, **caractérisée en ce que** le composé additionnel présente une bande interdite de 2,5 eV ou plus, de préférence de 3,0 eV ou plus, de façon très préférable de 3,5 eV ou plus.

14. Formulation comprenant au moins un composé selon une ou plusieurs des revendications 1 à 7 ou au moins une composition selon une ou plusieurs des revendications 10 à 13 et au moins un solvant.

15. Utilisation d'au moins un composé selon une ou plusieurs des revendications 1 à 7 ou d'au moins une composition selon une ou plusieurs des revendications 10 à 13 dans un dispositif électronique, de préférence dans un dispositif électroluminescent organique, de façon très préférable, dans une diode à émission de lumière ou électroluminescente organique (OLED) ou dans une cellule électrochimique à émission de lumière ou électroluminescente organique (OLEC, LEEC, LEC), de façon très particulièrement préférable, dans une OLED, de préférence dans une couche d'émission (EML), dans une couche de transport d'électrons (ETL) et dans une couche de blocage de trous (HBL), de façon très préférable dans une EML et dans une ETL et de façon très particulièrement préférable, dans une EML.

16. Dispositif électronique comprenant au moins un composé selon une ou plusieurs des revendications 1 à 7 ou au moins une composition selon une ou plusieurs des revendications 10 à 13, de préférence dans une couche d'émission (EML), dans une couche de transport d'électrons (ETL) et dans une couche de blocage de trous (HBL), de façon très préférable dans une EML et dans une ETL et de façon très particulièrement préférable, dans une EML.

17. Dispositif électronique selon la revendication 16, **caractérisé en ce qu'**il est sélectionné parmi les circuits intégrés organiques (OIC), les transistors à effet de champ organiques (OFET), les transistors à film mince organiques (OTFT), les dispositifs électroluminescents organiques, les cellules solaires organiques (OSC), les détecteurs optiques organiques, les photorécepteurs organiques, de préférence un dispositif électroluminescent organique.

18. Dispositif électronique selon la revendication 16 ou 17, **caractérisé en ce qu'**il s'agit d'un dispositif électroluminescent organique qui est sélectionné parmi le groupe qui est constitué par les transistors à émission de lumière ou électroluminescents organiques (OLET), les dispositifs à extinction de champ organiques (OFQD), les cellules électrochimiques à émission de lumière ou électroluminescentes organiques (OLEC, LEC, LEEC), les diodes laser organiques (O-laser) et les diodes à émission de lumière ou électroluminescentes organiques (OLED), de préférence les OLEC et les OLED, de façon très préférable, les OLED.

19. Procédé pour la fabrication d'un dispositif électronique selon une ou plusieurs des revendications 16 à 18, **caractérisé en ce qu'**au moins une couche organique est appliquée au moyen d'un dépôt en phase gazeuse ou à partir d'une solution.

20. Dispositif électronique selon la revendication 18, pour une utilisation en médecine pour la photothérapie, de préférence pour une utilisation pour la photothérapie de la peau, de façon très préférable, pour une utilisation pour le traitement ou pour la prévention du psoriasis, de la dermatite atopique, de la jaunisse/de l'ictère, de la jaunisse/de l'ictère du nouveau-né, du vitiligo, de l'inflammation, de la douleur et pour une utilisation pour la cicatrisation des plaies.

21. Utilisation du dispositif selon la revendication 18 dans les cosmétiques, de préférence pour le traitement ou pour la prévention du vieillissement de la peau, des rides de la peau, des pattes d'oie, de l'acné, des points noirs ou comédons et de la cellulite.

22. Utilisation du dispositif selon la revendication 18 dans les affichages ou pour l'éclairage.
